(19) Europäisches Patentamt
European Patent Office
Office européen des brevets

(11) EP 1 416 047 A1

(12) EUROPEAN PATENT APPLICATION
published in accordance with Art. 158(3) EPC

(43) Date of publication:
**06.05.2004 Bulletin 2004/19**

(21) Application number: **02745876.9**

(22) Date of filing: **09.07.2002**

(51) Int Cl.[7]: **C12N 15/09**, C12M 1/00, C12M 1/12

(86) International application number:
**PCT/JP2002/006939**

(87) International publication number:
**WO 2003/006650 (23.01.2003 Gazette 2003/04)**

(84) Designated Contracting States:
**AT BE BG CH CY CZ DE DK EE ES FI FR GB GR IE IT LI LU MC NL PT SE SK TR**
Designated Extension States:
**AL LT LV MK RO SI**

(30) Priority: **09.07.2001 JP 2001208514**
**29.11.2001 JP 2001364878**

(71) Applicant: **Asahi Kasei Kabushiki Kaisha**
**Osaka-shi, Osaka 530-8205 (JP)**

(72) Inventors:
- **KANNO, Kimiyoshi**
**Fuji-shi, Shizuoka 416-0949 (JP)**
- **ODA, Naozumi**
**Fuji-shi, Shizuoka 416-0933 (JP)**
- **ARITOMI, Masaharu**
**Fuji-shi, Shizuoka 417-0001 (JP)**
- **SATO, Akiko**
**Fuji-shi, Shizuoka 416-0909 (JP)**

(74) Representative:
**Forstmeyer, Dietmar, Dr. rer. nat., Dipl.-Chem. et al**
**Boeters & Lieck,**
**Bereiteranger 15**
**81541 München (DE)**

(54) **METHOD OF PURIFYING NUCLEIC ACID USING NONWOVEN FABRIC AND DETECTION METHOD**

(57) A method of separating and purifying nucleic acids from samples containing cells, such as blood and culture solutions. According to the method of the invention, a cell extract obtained by cell disruption is adsorbed by a filter made of a nonwoven fabric and the nucleic acid is eluted after washing the filter. Alkaline conditions of pH 12 or higher may be employed for elution of the nucleic acid, or the filter-adsorbed nucleic acid may be eluted by treatment with active oxygen or by using a surfactant. Nucleic acids separated and purified by the method of the invention can be used in nucleic acid amplification and nucleic acid sequence analysis techniques.

EP 1 416 047 A1

Printed by Jouve, 75001 PARIS (FR)

## Description

### Technical Field

The present invention relates to a simple method of preparing nucleic acids at a high purity from cells using a nonwoven fabric, and to a preparation kit therefor. The invention further relates to a method of amplifying nucleic acid from a nonwoven fabric which has adsorbed the nucleic acid, to a method of detecting a nucleic acid sequence from a nonwoven fabric which has adsorbed the nucleic acid, and to a method and kit for preparation of the nonwoven fabric which has adsorbed the nucleic acid, to be used in the aforementioned method.

### Background Art

Nucleic acids, including DNA, are generally prepared from cells by treating a sample containing the cells with SDS or Proteinase K and then denaturing and removing the protein with phenol to purify the nucleic acid (Molecular Cloning 2nd Edition, 9.16-9.23, Cold Spring Harbor Laboratory Press, 1989). Because of the trouble and time required for this procedure, however, simpler methods are desired.

An example of a simpler method employing silica is disclosed in EP0389063. In this method, the cells are first treated with a chaotropic reagent to lyse the cells and release the nucleic acid. Next, the nucleic acid is adsorbed onto a nucleic acid-binding support composed of silica or a derivative thereof, and the support is centrifuged and rinsed with a chaotropic reagent or organic solvent. Finally, the nucleic acid is eluted with a low salt buffer. Although this method is simpler than the phenol method, it has a disadvantage of entailing numerous steps and requiring a centrifugation procedure. Furthermore, because it uses a chaotropic agent or ethanol which strongly inhibit PCR and other enzyme reactions, it has a disadvantage of a necessity of thoroughly removing such substances through complicated and time-consuming procedures.

Japanese Examined Patent Publication HEI No. 8-24583 and Japanese Unexamined Patent Publication HEI No. 8-280384 disclose methods of purifying nucleic acids from peripheral blood leukocytes using cell adsorbing fiber aggregates such as leukocyte separation filters.

According to the method described in Japanese Examined Patent Publication HEI No. 8-24583, blood is first passed through a leukocyte separation filter to adsorb the blood leukocytes onto the filter and separate them from the other blood components. The filter is then rinsed with TE Buffer (10 mM Tris; 1 mM EDTA; pH 7.6) to remove the hemoglobin and other protein. The separated and rinsed leukocytes are frozen on each filter at -80°C, for example, and then allowed to stand at room temperature for thawing of the leukocytes. Next, TE Buffer-mix (TE Buffer, 10 mM NaCl, 1.5 mM $MgCl_2$, pH 7.5) is added to the fibrous material, and the leukocytes adsorbed onto the fibrous material of the filter are recovered from the fibrous material. However, extraction of the genomic DNA from the recovered leukocytes in the method of Japanese Examined Patent Publication HEI No. 8-24583 is accomplished by a prior art procedure. Specifically, a surfactant such as 10% sodium dodecyl sulfate (SDS) and a protease (Proteinase K) are added to the leukocytes, incubation is performed at 65°C for 15 hours, and then an RNase (RNaseA) is added prior to further incubation at 37°C for 1 hour. This is then treated with a phenol reagent and the DNA is precipitated with ethanol and purified.

Japanese Unexamined Patent Publication HEI No. 8-280384 discloses a method in which adsorption of nucleated cells is followed by extraction of the nucleic acid or protein in the nucleated cells. The method of extracting the nucleic acid or protein is carried out by passing a cytolytic solution through cell-binding ultrafine fiber aggregates, for lysis or disruption of the cells. The advantage of this method is that the adsorbed cells can be directly disrupted or lysed. Moreover, since the adsorbed cells are disrupted or lysed without dissociating the adsorbed cells, the method can be carried out more easily than the method of dissociating the adsorbed cells. However the method described for purification of the nucleic acid after cell lysis is a prior art method, and a new method is not disclosed therein. That is, the cell-adsorbing ultrafine fiber aggregates are treated with purified water or a surfactant, and the nucleic acid is purified by the ordinary phenol-chloroform method from the cytolytic solution which has passed through the ultrafine fiber aggregates.

Such a method of preparing nucleic acid from peripheral blood leukocytes using cell-adsorbing fiber aggregates as leukocyte separation filters has been known, but these methods had a disadvantage that after the disclosed filtering method employed up to the steps of leukocyte separation or nucleic acid extraction, the nucleic acid must be purified by existing nucleic acid purification methods, thereby complicating the procedure and requiring more time and trouble.

Recently, a method of directly purifying nucleic acid from cells using a filter has been disclosed in WO00/21973. This method comprises the following steps: (1) The cell-containing sample is passed through a filter to adsorb the cells onto the filter; (2) The cells adsorbed on the filter are lysed; (3) Filtration is carried out using the filter; (4) The nucleic acid adsorbed onto the filter is rinsed; (5) The nucleic acid is eluted from the filter. The adsorbed nucleic acid elutes upon heating from 40°C to 125°C, and the pH of the elution buffer is in the range of 5 to 11, with either a high or low salt concentration. The absorbance ratio $A_{260}/A_{280}$ of the purified nucleic acid is 1.8 and it may be used as a template for PCR. WO00/21973 mentions Whatman GF/D variant filters as filters that can be used for purification of nucleic acid, and Whatman GF/C filters as examples of unusable filters. It is stated that characteristic features of the filters suitable for this method are that it is impossible to capture a purified DNA when it passes through the filters, and that when lysed cells are passed through filters, the DNA yield is reduced by 80% and thus it is not practical. Furthermore, when nucleic acid is prepared from blood by this method, the experimenter must hemolyze the erythrocytes before lysing the leukocytes. Such methods wherein purification is carried out after adsorbing the cells onto the filter also have a drawback that the filter must be selected according to the type of cell.

U.S. Patent No. 5,187,083 and U.S. Patent No. 5,234,824 disclose methods of purifying DNA by lysing blood cells with a surfactant and then passing them through a filter with a pore size of 0.2 to 0.8 μm. The method claimed in U.S. Patent No. 5,234,824 is a method of rapidly preparing purified DNA from blood cells, and comprises the following steps. (1) The cells are gently lysed with a solution containing a surfactant and a viscosity reinforcer for a period of 2 to 40 minutes without a high shear force, to prepare a lysate containing the cellular DNA. The DNA must have a sufficiently large molecular weight in order to be trapped by the filter. (2) The extract is then filtered with a filter having a pore size of from 0.2 to 0.8 μm to trap the DNA in the filter. (3) The filter is heated in purified water at about 100°C for 5 to 15 minutes to extract the DNA. The extract may also contain up to 100 mM magnesium or calcium. The viscosity reinforcer used in step (1) may be polyvinyl alcohol (molecular weight: 70,000-100,000), a water-soluble polymer, sugar, a polypeptide, gelatin or the like. Also, blood must be diluted at least 10-fold in order to purify the DNA from the blood by this method. Otherwise, the viscosity is too high to allow filtration with the filter.

Japanese Patent Public Inspection No. 2001-520894 discloses a method of isolating nucleic acids through the steps of (1) loading the nucleic acids onto a surface from a prescribed direction, (2) immobilizing the nucleic acids on the surface, (3) releasing the immobilized nucleic acids from the surface and (4) removing the nucleic acids released from the surface, primarily in the loading direction. A membrane may be used as the surface, and the membrane may be either hydrophobic or hydrophilic. As membrane materials there may be used nylon, polysulfone, polyethersulfone, polycarbonate or polyacrylate, as well as acrylic acid copolymers, polyurethane, polyamide, polyvinyl chloride, polyfluorocarbonate, polytetrafluoroethylene, polyvinylidene fluoride, polyvinylidene difluoride, polyethylene-tetrafluoroethylene copolymer, polyethylene-chlorotrifluoroethylene copolymer, or polyphenylene sulfide. The pores of the membrane have a diameter of 0.001-50 μm, preferably 0.01-20 μm and most preferably 0.05-10 μm. For immobilization of the nucleic acids there may be used salts of mineral acids and alkali or alkaline earth metals, salts of alkali or alkaline earth metals and monobasic, polybasic or polyfunctional organic acids, hydroxyl derivatives of aliphatic or acyclic saturated or unsaturated hydrocarbons, phenols or polyphenols, or chaotropic agents.

This method has a drawback that an alcohol or a chaotropic agent must be added during adsorption in order to increase the yield of the nucleic acid. For example, when adsorbing RNA onto Hydrolon (Pall) which is a hydrophobic nylon membrane with a pore size of 1.2 μm, using 1 M NaCl as the binding solution gives a yield of 0.15 μg, whereas using 1 M NaCl containing 36% ethanol gives a 10-fold yield of 1.55 μg. Also, using 500 mM guanidium isothiocyanate containing 36% ethanol or 1 M guanidinium hydrochloride containing 36% ethanol as the binding solution gives yields of 2.3 μg or 6.7 μg, respectively. Using a chaotropic agent requires subsequent thorough washing with an alcohol-containing buffer, followed by drying.

Japanese Patent Public Inspection No. 11-501504 discloses a method of isolating nucleic acid from a sample, which method comprises contacting the sample with a surfactant and an immobilizing support to bind the soluble nucleic acid in the sample onto the support, and then separating the nucleic acid-binding support from the sample. Magnetic particles marketed under the name of DYNABEADS are mentioned as particularly suitable as the immobilizing support for this invention, and are used exclusively in the examples. The surfactant may be any type of surfactant, i.e., an anionic or cationic surfactant, or a non-ionic or amphoteric surfactant.

When magnetic particles are used, the sample volume will usually be from several microliters to several tens of microliters. A larger sample volume therefore requires prior concentration of the cells by a procedure such as centrifugation, or concentration of the target cells after removing the unwanted cells and substances. For example, when purifying leukocytic DNA in 0.5 ml of blood, the procedure required includes hemolysis followed by centrifugation to prepare a leukocyte pellet. The DNA is collected as a gel-like DNA/DYNABEADS complex, which easily disintegrates by such manipulation as pipetting. Care must therefore be taken to carry out the washing in a gentle manner so as not to break up the complex and thereby notably to reduce the DNA yield.

Elution of solid surface-immobilized nucleic acid with either active oxygen or an alkali has not been hitherto reported.

With the rapid development of human genome analysis in recent years, attempts have been made to actively link the resulting genomic information with qualitative improvements in medical care. Processing of data on single nucleotide polymorphisms (SNPs) is expected to allow discrimination of drug effects between different individuals to determine optimum drugs and doses for each patient, thereby opening the door to so-called "order-made medical treatment". In order to realize order-made medical treatment utilizing SNPs, it is essential to develop rapid, accurate and inexpensive SNP typing techniques. Here, SNP typing techniques encompass all of the techniques used from specimen processing to output of the final examination results, including genomic extraction and purification, nucleic acid amplification and nucleic acid sequence analysis.

For amplification of specific nucleic acid sequences by nucleic acid amplifying reactions such as the Polymerase Chain Reaction (PCR), or for analysis of nucleic acid sequences by SNP typing techniques, the nucleic acid target of amplification or sequence analysis is usually supplied to the reaction system in a liquid state. In all of the references cited above (WO00/21973, U.S. Patent No. 5,187,083, U.S. Patent No. 5,234,824, Japanese Patent Public Inspection No. 2001-520894), the nucleic acid is collected as a solution. Nucleic acid preparation methods are commonly known, but elution is time consuming and requires setting of suitable elution conditions. The time necessary for examination can be notably shortened by reducing the time required for nucleic acid elution or by shortening the elution process itself and proceeding to the subsequent examination steps.

U.S. Patent No. 5,756,126 describes a method of directly analyzing nucleic acid adsorbed onto a filter. In this method, (1) a sample containing a nucleic acid substance is added to a dry solid medium onto which is adsorbed a weak base, a chelating agent, an anionic surfactant and a component necessary for analysis, (2) the sample is stored, (3) the protein or hemoglobin in the sample is removed and (4) the sample is analyzed. U.S. Patent No. 5,972,386 describes a method of directly analyzing nucleic acid adsorbed on a filter, using a dry solid medium for nucleic acid storage comprising the following 3 elements: (a) a solid matrix having adsorbed thereto a protein denaturing agent, (b) a component necessary for analysis, and (c) a retaining agent for retaining the component on the matrix. Specifically, (1) a sample containing a nucleic acid substance is added to a dry solid medium, (2) the sample is stored, (3) the protein or hemoglobin in the sample is removed and (4) the sample is analyzed.

In these two patents, cellulose paper having uric acid, Tris, EDTA and SDS adsorbed thereon is mentioned as an example of the dry solid medium. The analysis is conducted after placing 3 μl of blood on a 9 $mm^2$ piece of the cellulose paper, or 100 μl of blood on a 100 $mm^2$ piece, and drying and storing it. The analysis referred to in this case is PCR or electrophoresis based on Restriction Fragment Length Polymorphism (RFLP). These methods have drawbacks because they require labor for preparation of the dry solid medium and are limited in the volumes of samples that can be added to the dry solid medium, thereby reducing the density of nucleic acid that can be immobilized on the medium, while organic solvents such as phenol or alcohol must be used to remove the protein including hemoglobin, and the procedure is thus complicated and time consuming.

EP 389,063 describes a method of separating nucleic acid by mixing a sample, a chaotropic agent and a nucleic acid-binding solid phase, binding the nucleic acid to the solid phase, and then separating and washing the solid phase. As examples of nucleic acid-binding solid phases there are mentioned silica beads, as well as filters of PVDF (MILLIPORE), Nitrocellulose (Schleicher and Schuell), Hybond-N (Amersham) or the like. A method of PCR using the nucleic acid-adsorbed filter directly as a template is also described. The nucleic acid solution is mixed with a guanidine thiocyanate solution and the filter to adsorb the nucleic acid onto the filter, and it is then washed with the chaotropic agent and 70% ethanol and dried at 56°C. The filter is directly added to a PCR reaction system to allow amplification of the target nucleic acid. The drawbacks of this method, however, include a necessity of using a chaotropic agent and organic solvent, the need to completely remove the chaotropic agent and ethanol, which strongly inhibit the PCR reaction, and the resulting complex and time consuming operations. In addition, because the filter is normally used for blotting, it is not suited for the purpose of purifying nucleic acid from biological substances such as blood.

WO98/51693 describes a general method for detecting cells in a sample using nucleic acid, wherein (1) cells in a sample are bound to a solid, (2) the cells are lysed, (3) the nucleic acid freed from the cells is bound to the same solid as in (1) and (4) the nucleic acid of the target cells is detected. The disadvantages of this method are that because the cells must be bound first to the solid, the solid must therefore be selected to match the type of cell, and that there is a restriction on the flow rate for filtration, because the cells must also be bound without disrupting them.

## Disclosure of the Invention

It is an object of the present invention to provide a method of purifying cellular nucleic acids from a sample containing blood cells or the like in a simpler and higher yield manner than prior art techniques. It is another object of the invention to provide a rapid and simple method of preparing nucleic acids which can be used for conventional nucleic acid amplification techniques or nucleic acid sequence analysis techniques.

The present invention therefore provides the following:

(1) A method of preparing cellular nucleic acid from a cell-containing sample, which method comprises:

(a) a step of disrupting cells to prepare a cell extract;
(b) a step of contacting the cell extract with a nonwoven fabric to adsorb the nucleic acid in the cell extract onto the nonwoven fabric; and
(c) a step of eluting the nucleic acid from the nonwoven fabric.

(2) The method of (1), wherein the nucleic acid adsorbed onto the nonwoven fabric is heated in a temperature range of 40°C to 100°C and preferably a temperature range of 80°C to 95°C to elute the nucleic acid.
(3) The method of (1) or (2), wherein the nucleic acid adsorbed onto the nonwoven fabric is treated under alkaline conditions of pH 12 or higher to elute the nucleic acid.
(4) The method of (1) or (2), wherein the nucleic acid adsorbed onto the nonwoven fabric is fragmented for elution.
(5) The method of (4), wherein active oxygen is used to fragment the nucleic acid.
(6) The method of (5), wherein the active oxygen used is generated by adding a divalent metal ion to a reducing sugar.
(7) The method of (5), wherein the active oxygen is hydrogen peroxide.
(8) The method of (5), wherein the active oxygen used is generated by adding a divalent metal ion to hydrogen peroxide.
(9) The method of (4), wherein an enzyme is used to fragment the nucleic acid.
(10) The method of (1) or (2), wherein the nucleic acid adsorbed onto the nonwoven fabric is treated with a surfactant for elution.
(11) The method of (10), wherein the surfactant is a non-ionic surfactant or amphoteric surfactant.
(12) A method of preparing and amplifying cellular nucleic acid from a cell-containing sample, which method comprises:

(a) a step of disrupting cells to prepare a cell extract;
(b) a step of contacting the cell extract with a nonwoven fabric to adsorb the nucleic acid in the cell extract onto the nonwoven fabric;
(c) a step of adding a nucleic acid amplification solution to the nonwoven fabric and using the nucleic acid adsorbed onto the nonwoven fabric as the template for amplification of the nucleic acid; and
(d) a step of recovering the reaction solution.

(13) The method of (12), wherein the nucleic acid is amplified by a PCR or LAMP method.
(14) A method of preparing cellular nucleic acid from a cell-containing sample and detecting a specific nucleic acid sequence, which method comprises:

(a) a step of disrupting cells to prepare a cell extract;
(b) a step of contacting the cell extract with a nonwoven fabric to adsorb the nucleic acid in the cell extract onto the nonwoven fabric;
(c) a step of adding a solution for the detection of a nucleic acid sequence to the nonwoven fabric for reaction; and
(d) a step of measuring the reaction solution.

(15) A method of preparing cellular nucleic acid from a cell-containing sample and detecting a specific nucleic acid sequence, which method comprises:

(a) a step of disrupting cells to prepare a cell extract;
(b) a step of contacting the cell extract with a nonwoven fabric to adsorb the nucleic acid in the cell extract onto the nonwoven fabric;
(c) a step of adding a solution for the detection of a nucleic acid sequence to the nonwoven fabric for reaction; and
(d) a step of measuring the surface of the nonwoven fabric.

(16) The method of (14) or (15), wherein the nucleic acid sequence is detected by a PCR or LAMP method.
(17) The method of (15), wherein the specific nucleic acid sequence is detected by hybridization using a probe.
(18) The method of (14) or (15), wherein the specific. nucleic acid sequence is detected by nucleic acid extension reaction using primers and a polymerase.
(19) The method of (18), wherein the detection is accomplished by incorporating a labeled nucleotide during the nucleic acid extension reaction.
(20) The method of (18), wherein pyrophosphoric acid produced by the nucleic acid extension reaction is detected.
(21) A method of preparing a cellular nucleic acid-adsorbed nonwoven fabric, which method comprises:

(a) a step of disrupting cells to prepare a cell extract; and
(b) a step of contacting the cell extract with a nonwoven fabric to adsorb the nucleic acid in the cell extract onto the nonwoven fabric.

(22) A method of preparing cellular nucleic acid from a cell-containing sample and labeling the nucleic acid, which method comprises:

(a) a step of disrupting cells to prepare a cell extract;
(b) a step of contacting the cell extract with a nonwoven fabric to adsorb the nucleic acid in the cell extract onto the nonwoven fabric; and
(c) a step of adding a nucleic acid-labeling solution to the nonwoven fabric and reacting it therewith.

(23) The method of (22), wherein the nucleic acid is labeled with biotin, fluorescence or a hapten.
(24) The method of (22) or (23), wherein the nucleic acid adsorbed onto the nonwoven fabric is itself labeled.
(25) The method of any one of (1) to (24), wherein the nonwoven fabric material is polyester, polypropylene or nylon.
(26) The method of any one of (1) to (24), wherein the nonwoven fabric material is polyethylene terephthalate.
(27) The method of any one of (1) to (26), wherein the nonwoven fabric pore size is 2-150 μm.
(28) The method of any one of (1) to (27), wherein the nonwoven fabric fiber size is 0.3-20 μm.
(29) The method of any one of (1) to (28), wherein a cell extract not containing a viscosity enhancer, chaotropic agent or alcohol is used.
(30) The method of any one of (1) to (29), wherein a salt-containing cell extract is used.
(31) The method of (30), wherein the salt in the cell extract is a sodium salt, potassium salt, magnesium salt, calcium salt, ammonium salt, phosphate, sulfate or hydrochloride.
(32) The method of (30), wherein the salt in the cell extract is sodium chloride, and its concentration is from 10 mM to 1000 mM.
(33) The method of (30), wherein the salt in the cell extract is magnesium chloride, and its concentration is from 1 mM to 100 mM.
(34) The method of (30), wherein the salt in the cell extract is sodium phosphate, and its concentration is from 2 mM to 100 mM.
(35) The method of (30), wherein the salt in the cell extract is ammonium sulfate, and its concentration is from 20 mM to 1000 mM.
(36) The method of any one of (1) to (35), wherein a cytolytic solution containing an anionic surfactant, an amphoteric surfactant or a non-ionic surfactant is used.
(37) The method of any one of (1) to (36), wherein the step of disrupting cells to prepare a cell extract is carried out in a temperature range of 80°C to 110°C.
(38) The method of any one of (1) to (37), wherein the step of disrupting cells to prepare a cell extract is carried out in the presence of a reducing agent.
(39) The method of (38), wherein the reducing agent is a thiol group-containing compound.
(40) The method of any one of (1) to (39), wherein the method of contacting the cell extract with a nonwoven fabric is filtration.
(41) The method of any one of (1) to (40), wherein the step of adsorbing the nucleic acid in the cell extract onto the nonwoven fabric is followed by a step of washing the nonwoven fabric.
(42) The method of (41), wherein the nonwoven fabric is washed with a solution not containing a chaotropic agent or alcohol.
(43) The method of (41) or (42), wherein the nonwoven fabric is washed with a cytolytic solution.
(44) The method of any one of (41) to (43), wherein the nonwoven fabric is washed with a salt solution having a concentration range of 0.5 M to 2 M.
(45) A kit for preparation of cellular nucleic acid from a cell-containing sample, which kit comprises:

(a) a device incorporating a nonwoven fabric; and (b) a solution containing either a cytolytic solution or a nucleic acid eluent.

(46) A kit for extraction of nucleic acid from cells and preparation of a nonwoven fabric having the nucleic acid adsorbed thereon, which kit comprises:

(a) a device incorporating a nonwoven fabric; and (b) a solution containing either a cytolytic solution or a washing solution.

(47) A method of eluting nucleic acid adsorbed on a solid surface by treating it under alkaline conditions of pH 12 or higher.

(48) A method of eluting solid surface-adsorbed nucleic acid by treating it with active oxygen.

(49) A method of eluting nucleic acid adsorbed on solid surface by treating it with a surfactant.

(50) A nonwoven fabric which has adsorbed nucleic acid.

The present invention will now be explained in more detail.

According to the invention, "cells" are eukaryotic cells, prokaryotic cells or viruses, and include particularly human somatic cells, human peripheral blood leukocytes and infectious fungi, bacteria and viruses.

According to the invention, the cell-containing sample may be any sample which contains cells, including blood, urine, spinal fluid, sputum, body fluids, cell suspensions, cell cultures and the like. It may also be a treated solution obtained by some sort of treatment of such specimens. Treatment methods include, for example, dissolution of a highly viscous specimen such as sputum with a sputum-treating agent.

According to the invention, the cell extract is a mixture containing cellular constituent components obtained by cell disruption, and it may be prepared by allowing a cytolytic solution to act on a cell-containing sample. A cytolytic solution is a solution used to disrupt cells and extract the nucleic acid, and includes surfactants, enzymes, EDTA and the like, although not all of these are essential. The enzyme used may be a protease such as Proteinase K, but depending on the cell type it is also possible to use lysozyme, lysostaphin *(Staphylococcus),* β1,3-glucanase, mannase, chitinase (fungi) or the like. If it is preferred to remove the RNA, a ribonuclease such as RNaseA may be added. An enzyme will not be necessary in all cases, as it is possible to disrupt animal cells by simple contact with a hypotonic solution. EDTA is used to inhibit DNA degrading enzymes, and is normally used at a concentration of 25 mM. The cell extract may also be prepared by applying physical force such as subjecting the cell-containing sample to ultrasonic waves or disruption with a homogenizer.

A salt-containing cell extract is preferably used to adsorb the nucleic acid in the cell extract onto the nonwoven fabric. The salt may be added to the cytolytic solution, or it may be added when the cell extract is contacted with the nonwoven fabric. Although various different salts may be used, sodium salt, potassium salt, magnesium salt, calcium salt, ammonium salt, phosphate, sulfate and hydrochloride are preferred. The concentration range promoting nucleic acid adsorption will differ depending on the salt used. For example, suitable concentrations for nucleic acid adsorption are from 10 mM to 1000 mM and preferably 50 mM to 200 mM for sodium chloride, from 1 mM to 100 mM and preferably 2 mM to 20 mM for magnesium chloride, from 2 mM to 100 mM and preferably 20 mM to 100 mM for sodium phosphate, and from 20 mM to 1000 mM and preferably 20 mM to 200 mM for ammonium sulfate. The nucleic acid is preferably adsorbed in a pH range of 6-11 and preferably in a pH range of 6-8.

According to the invention, a nonwoven fabric is any sheet-like or web structure formed by bonding or entangling staple fibers or filaments using mechanical, thermal or chemical means (Fiber Handbook, 2nd Edition, Japan Fiber Association, Maruzen Publ.). Nonwoven fabrics are produced by various methods, but the basic steps are formation of a web (a fiber mass sheet with a certain degree of directional alignment of the fibers), bonding of the web and finishing of the web. Numerous types of fibers, from natural fibers to chemical fibers, are used in nonwoven fabrics, the most common ones being cotton, rayon, polyester, polypropylene and nylon, as well as acryl, vinylon, glass fiber, pulp, carbon fiber and the like. Systems for forming webs are largely classified into wet, dry and direct processes. Direct processes are also known as melt spinning processes, wherein fibers spun from a melted polymer solution are aggregated directly into a web. Such processes include spunless, spunbond, melt blow, needlepunch and stitchbond processes. Ultrafine fiber nonwoven fabrics produced by melt blow processes are most suitable for the present invention.

According to the invention, contact of the cell extract with the nonwoven fabric may be accomplished, for example, by floating the nonwoven fabric in the cell extract, by floating and soaking the nonwoven fabric in the cell extract, or by pouring the cell extract over the surface of the nonwoven fabric.

According to the invention, washing is performed to wash off substances such as proteins and lipids which have adhered to the nonwoven fabric. A surfactant such as SDS or Triton X-100 or a salt solution such as NaCl at 0.5 M or greater concentration may be used as the washing solution, but there is no limitation to these and the washing solution may be selected according to the type of adhering substances. In some cases, the washing step may even be omitted.

According to the invention, "alkali treatment" means soaking the filter in an aqueous solution of pH 12 or higher. Normally used alkali solutions include, but are not limited to, NaOH, KOH and the like. The alkali treatment is preferably followed by neutralization to return the pH to neutral, using a compound or acid having buffering capacity near neutral, such as a phosphate.

Active oxygen used for the invention includes, but is not limited to, active oxygen in the strict sense, i.e., the superoxide ($O_2 \cdot^-$) radical, which is the one-electron reduced form of $^3O_2$, the two-electron reduced species hydrogen peroxide ($H_2O_2$), singlet oxygen ($^1O_2$) which is an oxygen molecular species in an electron-excited state, or the hydroxyl radical (HO·), and active oxygen in the wide sense, i.e., primarily metal-oxygen complexes (including metal oxo acids), as well as compounds derived from reaction between such active species and biomolecules (for example, unsaturated fatty acids L), including peroxy radicals (LOO·), alkoxy radicals (LO·), hydroxyperoxide (LOOH) and nitric oxide (NO). Active oxygen is described in detail in "Kassei Sanso" [Active Oxygen] (Proteins, Nucleic Acids and Enzymes, Extra Edition, Vol.33, No.16) and "Kosanka Busshitsu no Subete" [All About Antioxidants] (Sentan Igakusha).

Several methods for generating active oxygen have been reported, and for example, active oxygen can be easily generated by the following method. Although hydrogen peroxide is itself an active oxygen form, addition of divalent metal ions such as $Ca^{2+}$ or $Fe^{2+}$ to hydrogen peroxide causes the hydrogen peroxide to act as an oxidizer to generate the hydroxy radical (HO·). It has also been reported that addition of the metal ion $Cu^{2+}$ to reducing sugars such as D-ribose generates active oxygen, producing cytotoxicity and nucleic acid injury. It is known that the antitumor antibiotic bleomycin in the presence of iron (II) and oxygen or iron (III) and hydrogen peroxide generates an active oxygen complex species which cleaves DNA.

Particularly preferred active oxygen species according to the invention include hydroxy radical obtained by adding divalent metal ions such as $Cu^{2+}$ or $Fe^{2+}$ to reducing sugars such as D-ribose-5-phosphate or D-fructose-6-phosphate, and hydroxy radical obtained by adding divalent metal ions such as $Cu^{2+}$ or $Fe^{2+}$ to hydrogen peroxide. When a divalent metal ion is added to generate active oxygen, a metal chelating agent such as EDTA is preferably added to the active oxygen reaction mixture as this will halt generation of the active oxygen and prevent excessive injury to the nucleic acid. The time period for the nucleic acid treatment using the active oxygen may be from 1 second to 10 minutes, preferably from 1 second to 5 minutes and more preferably from 10 seconds to 1 minute.

The preferred conditions for elution of the nucleic acid adsorbed onto the nonwoven fabric will depend on the amount and type of the adsorbed nucleic acid, and for example, when generating hydroxy radical by addition of $Fe^{2+}$ as the divalent metal ion to D-ribose-5-phosphate, the concentration of the D-ribose-5-phosphate may be from 1 mM to 1 M, preferably from 10 mM to 500 mM and more preferably from 50 mM to 200 mM. The $Fe^{2+}$ concentration may be from 0.001 mM to 10 mM, preferably from 0.01 mM to 10 mM and more preferably from 0.05 mM to 5 mM. The reaction temperature may be 30-65°C, preferably 40-60°C and more preferably 45-55°C.

According to the invention, the nucleic acid adsorbed onto the nonwoven fabric may be fragmented by a method other than one using active oxygen, such as a method using a restriction enzyme which recognizes and cleaves a specific sequence of the nucleic acid, or a nucleic acid degrading enzyme such as DNase which cleaves optional sequences. The nonwoven fabric may also be exposed to ultraviolet rays to fragment the nucleic acid. Preferred restriction enzymes to be used for the invention include, but are not limited to, HaeIII, Sau3AI, EcoRI and BamHI. The length of nucleic acid recognized will differ depending on the restriction enzyme, and for example, 4-base recognition sequence restriction enzymes are preferred over 6-base recognition sequence restriction enzymes because they produce smaller nucleic acid fragments and thus increase the efficiency of elution from the nonwoven fabric. When an enzyme is used, the amount of the enzyme (concentration, proportion, etc.) will depend on the amount of nucleic acid and the type of enzyme.

According to the invention, "adding a nucleic acid amplification solution" or "adding a nucleic acid sequence detection solution" to the nonwoven fabric means that all or a portion of the nucleic acid-adsorbed nonwoven fabric is provided directly to the reaction system for nucleic acid amplification or nucleic acid sequence detection without elution of the nucleic acid.

According to the invention, the nucleic acid amplification method is a method of amplifying a specific target nucleic acid sequence and is typically PCR, but methods other than PCR may also be used, including Ligase Chain Reaction (LCR), Transcription-Mediated Amplification (TMA), Branched DNA (bDNA) Assay, Nucleic Acid Sequence Based Amplification (NASBA), Strand Displacement Amplification (SDA), Cycling Probe Technology (CPT), Q-Beta Replicase Amplification Technology, Rolling Circle Amplification Technology (RCAT), Loop-Mediated Isothermal Amplification (LAMP) and Isothermal and Chimeric primer-initiated Amplification of Nucleic acids (ICAN), with no particular limitation to these. Q-Beta Replicase, RCAT, NASBA, SDA, TMA, LAMP, ICAN and the like accomplish amplification reaction isothermally, while PCR and LCR accomplish amplification reaction by thermal cycling.

According to the invention, the nucleic acid sequence detection method is a technique which allows nucleic acid sequence analysis on a filter without using electrophoresis or mass spectrometry. Such techniques include, for example, common probe hybridization methods using fluorescence, luminescence, color development or radioisotopes, methods using nucleic acid intercalators, and SNPs typing techniques. Specific examples of SNPs typing techniques include Invader™ Assay, TaqMan™, Rolling Circle Amplification (RCA), Pyrosequencing, Primer Extension, LAMP and UCAN.

According to the invention, the nucleic acid extension reaction using primers and a nucleic acid synthetase is a primer specific 5'→3' DNA or RNA synthesis reaction using DNA-dependent DNA polymerase, DNA-dependent RNA polymerase, RNA-dependent RNA polymerase, reverse transcriptase, strand-displacement DNA polymerase or the like. As DNA polymerases there may be used Klenow Fragment, T4 DNA polymerase, Taq DNA polymerase or the like, with no particular limitation to these. Examples of methods for detecting specific nucleic acid sequences by nucleic acid extension reaction with primers and DNA polymerases include the aforementioned SNPs typing techniques of Pyrosequencing, Primer Extension, LAMP, etc.

According to the invention, a chaotropic agent is the general term for a substance which disrupts the structure of water molecules in an aqueous solution.

According to the invention, a "surfactant" is a compound having both a hydrophilic portion and a lipophilic portion, and surfactants are largely classified into ionic surfactants and non-ionic surfactants. Ionic surfactants may be generally categorized as anionic surfactants, cationic surfactants and amphoteric surfactants. Surfactants to be used in the cytolytic solution of the invention may be anionic surfactants, amphoteric surfactants or non-ionic surfactants, but cationic surfactants cannot be used. As examples of anionic surfactants there may be mentioned sodium dodecyl sulfate, sodium dodecyl sulfonate, sodium dodecyl-N-sarcosinate, sodium cholate, sodium deoxycholate and sodium taurodeoxycholate. As examples of amphoteric surfactants there may be mentioned 3-[(3-cholamidopropyl) dimethylammonio]-1-propanesulfonic acid, 3-[(3-cholamidopropyl)dimethylammonio]-2-hydroxy-1-propanesulfonic acid, palmitoyl-lysolecithin, dodecyl-N-betaine and dodecyl-β-alanine. As examples of non-ionic surfactants there may be mentioned octyl glucoside, heptyl thioglucoside, decanoyl-N-methylglucamide, polyoxyethylene dodecylether, polyoxyethylene heptamethylhexyl ether, polyoxyethylene isooctylphenyl ether, polyoxyethylene nonylphenylether, polyoxyethylene nonylphenylether, polyoxyethylene fatty acid esters, sucrose fatty acid esters and polyoxyethylene sorbitol esters.

According to the invention, "wherein the nucleic acid adsorbed onto the nonwoven fabric is itself labeled" means that labeling is directly introduced into the adsorbed nucleic acid without newly synthesizing the nucleic acid. Examples of such labeling methods include non-enzymatic or chemical introduction of biotin or digoxigenin, or fluorescent substances such as rhodamine, fluorescein, Cy3, Cy5 or the like, and reagents therefor are commercially available. On the other hand, "labeling the nucleic acid adsorbed on a nonwoven fabric" includes the aforementioned direct labeling as well as labeling by synthesizing a complementary chain of the nucleic acid using a nucleic acid synthetase and labeled nucleotides. A "hapten" according to the invention is a compound which has low immunogenicity by itself but when chemically bound to a carrier can induce specific antibody production. For example, in the case of the above described digoxigenin labeling, the digoxigenin serves as the hapten.

The present invention will now be explained in more detail. The present inventors examined methods of convenient and rapid purification of a nucleic acid without using chaotropic agents or organic solvents such as ethanol. As a result, it was discovered that nonwoven fabrics are highly suitable for nucleic acid purification. It was found that some other types of porous filters, for example, the polycarbonate track-etched membrane with a pore size of 8 μm as described in Comparative Example 2, are not capable of adsorbing nucleic acids, and therefore not all porous filters can be used for nucleic acid purification. The track-etched membrane is coated with a thin-film bilayer, with SiCN as the top layer. On the other hand, nonwoven fabrics with pore sizes of about 10-130 μm and fibers sizes of about 1.2-20 μm were found to be suitable for purification of nucleic acid from blood regardless of the material used. That is, a nonwoven fabric may be used so long as it has a mean pore size of 2-150 μm, within which 7-13 μm is particularly preferred. Also, a mean fiber size of 0.3-20 μm can be used, with 0.7-1.7 μm being particularly preferred. Although different materials for the nonwoven fabric have superiority or inferiority with respect to nucleic acid yields and purification degrees from blood, it was found that each of polyester, cellulose, acryl, polypropylene and nylon can be used either alone or in combinations. Upon soaking a polyethylene terephthalate (PET) sheet membrane and a nonwoven fabric in a purified genomic DNA solution containing an appropriate salt it was found, surprisingly, that the genomic DNA became bound to the nonwoven fabric but not to the sheet membrane. Here, "PET sheet membrane" means a completely poreless PET sheet. Since the DNA became bound to the nonwoven fabric simply by contacting the DNA solution with the nonwoven fabric, it was thought that the DNA had bound to the nonwoven fabric by physical adsorption as opposed to being filtered by the filter. These results indicated that the form of a nonwoven fabric is highly suited for nucleic acid purification.

Upon still further examination, it was discovered that PET nonwoven fabrics, and particularly PET nonwoven fabrics having pore sizes of about 10 μm and fiber sizes of about 1.2 μm, are superior among nonwoven fabrics. Simple contact of purified genomic DNA with a PET nonwoven fabric results in adsorption of 80-90% thereof onto the nonwoven fabric. Although a salt such as NaCl, $MgCl_2$, $HPO_4^{2-}$ or the like was necessary for the nucleic acid to bind to the nonwoven fabric, there was no need to add a chaotropic agent or ethanol to increase the nucleic acid yield as is described in Japanese Patent Public Inspection No. 2001-520894, and in fact, rather addition of ethanol notably reduced the amount of adsorption. Heating or nucleic acid fragmentation was necessary to elute the nucleic acid bound to the nonwoven fabric. Also, although it was necessary to conduct heating at 95°C for 20 minutes in TE Buffer for the nucleic acid elution, it was found that the time could be shortened to 5 minutes in an alkali solution, and that using active oxygen allowed elution to be accomplished in 10 seconds at room temperature.

The present inventors have further discovered surprising features of nucleic acid-adsorbed nonwoven fabrics. Specifically, it was found that it is not only possible to amplify the adsorbed nucleic acid by PCR or the like without elution of the nucleic acid and to hybridize a probe onto the nonwoven fabric, but that primer-dependent nucleic acid extension also occurs on the nonwoven fabric. Using this method thus allows purification and detection of nucleic acid to be carried out on the same nonwoven fabric, thereby notably simplifying the procedure time and steps. Detection was possible on the nonwoven fabric because the nonwoven fabric had a smooth, thin sheet-like surface. The present invention has been completed based on the research described above.

The invention will now be explained in more detail. It is a feature of the invention that cellular nucleic acid can be purified in a simple and rapid manner without using a chaotropic agent or an organic solvent such as ethanol. Because chaotropic agents and organic solvents such as ethanol strongly inhibit enzyme reactions including PCR, methods which employ these must be followed by washing well and, in the case of ethanol, thorough drying. Since the present invention does not use these reagents and therefore does not require such thorough washing or drying, purification of cellular nucleic acid can be accomplished in a simple and rapid manner.

It is another feature of the invention that the method is highly flexible for application to any type of cell because the cellular nucleic acid is released from cells and applied to the nonwoven fabric in a solution state. The cells may be eukaryotic cells such as human leukocytes, prokaryotic cells such as *E. coli*, or even viruses. In methods wherein cells are first captured directly on a filter and then lysed, it is necessary to optimize the filter properties and adsorption conditions for the particular type of cell. Furthermore, since the cells must be bound to the filter without being disrupted, the filtering speed is restricted, usually requiring slow passage through the filter. According to the present invention, it is a cell extract that is passed through the filter, and therefore the flow speed can be increased.

It is yet another feature of the invention that the nucleic acid is adsorbed onto the nonwoven fabric by simply passing the cell extract through the nonwoven fabric. Furthermore, there are no restrictions on the number of cells in the sample or the sample volume so long as they are within the nucleic acid adsorbing capacity range of the filter. In contrast, in methods of lysing cells after impregnating a substrate with a cell solution such as FTA™, the maximum sample volume that can be absorbed by the substrate limits the treatment volume, so that such methods are not very suitable for extraction of nucleic acids from large-volume samples. For example, when obtaining nucleic acid from the total cell content of a large-volume sample with low cell density, it is necessary to first concentrate the cells by centrifugation or other means. The filter of the invention, however, has no such restriction on sample volumes.

The method of preparing the cell extract is crucial. The cell extract is preferably an impurity-free solution. The viscosity of the cell extract is preferably not too high as this will tend to impair passage through the nonwoven fabric. Although addition of Proteinase K (final concentration: 0.1 mg/ml) is preferred, it is not essential. For example, in the case of lysis of blood at room temperature, the nucleic acid yield will tend to be improved by addition of Proteinase K, but purification of the nucleic acid can still be accomplished even without Proteinase K. On the other hand, addition of Proteinase K is essential for lysis of blood at 50°C, as blood extracts prepared at this temperature without addition of Proteinase K, when applied to nonwoven fabrics, will tend to cause clogging and prevent purification of the nucleic acid. A treatment time of 5 minutes is sufficient at room temperature, but a shorter time within 5 minutes is also possible. There is also no problem with extending the treatment time up to 60 minutes. When preparing a cell extract according to the invention, there is no need for 10-fold or greater dilution of the blood or addition of a viscosity reinforcer such as polyvinyl alcohol, as is described in U.S. Patent No. 5,234,824. The present inventors have also discovered that highly viscous specimens such as sputum can be heat treated in a temperature range of 80-110°C, or treated in the presence of a reducing agent, to reduce their viscosity and facilitate passage through the nonwoven fabric. For example, unless sputum is heat treated or treated with a reducing agent, it will tend to cause clogging and fail to pass through the nonwoven fabric. Treatment with heat or a reducing agent denatures the protein and other substances responsible for the viscosity of sputum, thereby lowering the viscosity and allowing it to pass through without clogging the nonwoven fabric. The heating temperature is preferably 80°C or higher, but an excessively high temperature is not preferred because it may degrade the nucleic acid. An upper limit of 110°C is preferred. The heating time may be a time sufficient to heat the interior of the sample, which will usually be at least 1 minute in the temperature range specified above, and 1-15 minutes is preferred. A reducing agent to be used is any substance which denatures the protein but does not affect the nucleic acid. Thiol group-containing substances are preferred as reducing agents, and examples thereof include dithiothreitol, cysteine, acetylcysteine and β-mercaptoethanol. The optimum concentration and treatment time for the reducing agent will vary depending on the reducing capacity of the reducing agent used. In the case of dithiothreitol, for example, a concentration of about 1-10 mM and a treatment time of about 1-10 minutes are preferred. Heat treatment and reducing agent treatment may also be carried out together.

Nucleic acid may be prepared according to the invention using a sample stored by any method, so long as the cellular nucleic acid is not degraded. For example, the cell-containing sample may be one which has been cryopreserved immediately after sampling or preparation. In the case of blood, the nucleic acid may be purified from either fresh blood or cryopreserved blood.

It is a feature of the invention that a nonwoven fabric is used as the porous filter. An advantage of a nonwoven fabric is that since the mean pore size of a nonwoven fabric used for the invention is larger than that of the filter with a pore size of 0.2 to 0.8 μm (disclosed in U.S. Patent No. 5,234,824), a greater flow speed can be achieved to prevent clogging. This is particularly advantageous when purifying nucleic acids from samples with high viscosity, such as blood lysis solutions. A040C01, for example, has a mean pore size of 10 µm. Here, the mean pore size of the nonwoven fabric is the (median) value measured with a mercury porosimeter. The mean pore size is an index related to the degree of entangling of the fibers and the sizes of the gaps constituting a filter element. It was considered that since the fibers in a nonwoven fabric are aligned in a planar direction perpendicular to the flow of liquid and are uniformly packed, producing little side flow, it would therefore exhibit excellent nucleic acid adsorbing capacity while maintaining a high treatment speed. When clogging is a problem, nonwoven fabrics with different fiber sizes or pore sizes may be used in combination. Moreover, nonwoven fabrics are advantageous over magnetic beads in that contact with DNA solutions can be accomplished simply by filtering with nonwoven fabrics. In order to efficiently contact DNA with magnetic beads, methods employing such beads must use minimal sample volumes, which are usually limited from several microliters to several tens of microliters. This necessitates special measures, for example, concentration of the target cells by a procedure such as centrifugation.

The nonwoven fabric used for nucleic acid purification may be, for example, a hydrophilic coated product such as HM-3-coated A040C01 or a non-coated product such as non-coated A040C01. A040C01 was found to have higher purified genomic DNA adsorbing capacity than HM-3-coated A040C01. A040C01 is a PET nonwoven fabric manufactured by Asahi Kasei Corp. HM-3-coated A040C01 is obtained by coating A040C01 with a solution of a copolymer composed of HM-3 (2-hydroxyethyl methacrylate (hereinafter abbreviated as HEMA) and N,N-dimethylethyl methacrylate (hereinafter abbreviated as DM); HEMA:DM = 97:3) in ethanol, and it is used as the main filter in Sepacell® by Asahi Medical Co., Ltd. The nonwoven fabric is preferably one whose components, such as the surface coating agent, do not elute at temperatures of 80-100°C, and which is resistant to clogging. There is no particular correlation between the leukocyte adsorbing capacity of a nonwoven fabric and its nucleic acid adsorbing capacity. Nonwoven fabrics exhibiting nucleic acid adsorbing capacity include HM-3-coated A040C01 and A040C01, which also exhibit excellent leukocyte adsorbing capacity, but also include filters such as E05070 which exhibit virtually no leukocyte adsorbing capacity. This is one of the notable differences between the characteristics of filters to be used for the invention and the characteristics of filters required in, for example, WO00/21973.

The present invention is a simpler method, with fewer steps, than the method of lysing cells after directly capturing them in a filter, as described in WO00/21973. When purifying nucleic acid from blood, for example, the method of WO00/21973 requires the following three steps prior to adsorption of the nucleic acid on the filter: 1) pouring the blood, 2) pouring a hemolytic solution to disrupt the erythrocytes and 3) pouring a cytolytic solution to disrupt the leukocytes. According to the invention, however, the blood and cytolytic solution are simply mixed and passed through the filter.

A nucleic acid adsorbed onto a nonwoven fabric does not elute merely by immersing the filter in water and allowing it to stand for 1-2 minutes at 10-30°C, as described in Japanese Patent Public Inspection No. 2001-520894. Nucleic acid adsorbed onto a nonwoven fabric elutes upon heating in a temperature range of 40-100°C and preferably in a temperature range of 80-95°C in water or a low-salt buffer. Specifically, it was discovered that nucleic acid elutes by heating in TE Buffer for 60 minutes at 80°C or for 20 minutes at 95°C, according to the present invention. A method of elution with alkali treatment was also discovered, and the use of this method was found to be highly useful because it allows elution of adsorbed DNA in a shorter time than by elution in TE Buffer. Heating further increases the elution rate, resulting in elution in 5 minutes at 95°C. Since alkali treatment is well known to denature double-stranded DNA into single-stranded DNA, it is thought that such denaturation facilitated elution of the nucleic acid adsorbed onto the filter. Incidentally, it has been reported that treatment with 1.2 N NaOH at 90°C for 10 minutes results in more selective modification of adenine and cytosine, and particularly adenine, in nucleic acid (Methods in Enzymology, Vol.65, p537 (1980)). Since the alkali treatment of the invention is carried out using NaOH at no greater than a 0.2 N concentration, there is believed to be little chance of such base modification. Alkali-eluted nucleic acid was actually usable as a template for PCR reaction, and hybridization was possible even when treating the hybridization probe under the same conditions as for alkaline elution.

Alkaline elution is characterized by producing eluted nucleic acid with a large molecular weight. When elution is performed by heating in TE Buffer at 95°C for 20 minutes, the nucleic acid is collected as fragments of several kilobases to several tens of kilobases, whereas elution by heating in an alkali at 95°C for 5 minutes has produced fragments of several hundred kilobases or greater. Alkaline elution is very useful when it is desired to prepare large-sized genomic DNA. The alkaline elution method of the present invention is also effective for elution of nucleic acid from a nucleic acid adsorption filter prepared according to the present invention, for example, a nucleic acid adsorption filter manufactured by the method described in WO00/21973, U.S. Patent No. 5,187,083 or U.S. Patent No. 5,234,824.

The present inventors also examined elution under acidic conditions. It was found, as a result, that nucleic acid elution is accelerated by heating in a temperature range of 70-100°C in an acidic solution in the range of pH 3 to 5 and preferably pH 4 to 5. For example, nucleic acid was eluted from a filter in 10 minutes at 95°C, and the nucleic acid was usable as a template for PCR. However, because amplification by PCR occurred less readily than when the elution was performed in TE Buffer, it is possible that acid-eluted nucleic acid suffers some degree of damage. In fact, it is well known that glycoside bonds between deoxyribose and pyrimidine bases in DNA are stable under acidic conditions, while glycoside bonds with purine bases are hydrolyzed under acidic conditions, producing apurinic acid. The reaction employed is based on the Maxam-Gilbert DNA sequencing method, a base-specific chemical degradation method, and in this case, treatment is carried out for 60 minutes at 20°C in 0.1 M piperidine formate wherein pH is adjusted to 2 (Methods in Enzymology, Vol.65, p535 (1980)). The pH condition described for the present invention is in the range of pH 3 to 5, and therefore nucleic acid sequence damage may occur.

With alkaline elution, a procedure was necessary to restore the pH to neutral after elution. The present inventors discovered that a method of elution in the presence of a surfactant requires no such subsequent procedure. The nonwoven fabric-adsorbed nucleic acid elutes upon heating in water or under low-salt conditions, but the nucleic acid elution is further accelerated by addition of a surfactant thereto. Compared to elution with TE Buffer, the surfactant had the effect of lowering the elution temperature and shortening the elution time. Since non-ionic surfactants and amphoteric surfactants do not inhibit PCR or hybridization at concentrations of about 1%, they are highly useful as eluents to give eluted nucleic acid which can be used directly.

The present inventors further discovered a method of accomplishing elution in a short time without the need for temperature control during the elution. Elution of nonwoven fabric-adsorbed nucleic acid by treatment with active oxygen is highly useful as it allows the adsorbed DNA to be eluted in simpler manner and in shorter time as compared with elution with TE Buffer or elution with an alkali. It is well known that the phosphoric acid ester bonds of nucleic acid residues are broken by active oxygen causing fragmentation of the nucleic acid, and it is believed that the nucleic acid adsorbed on the filter readily elutes from the filter by fragmentation. The use of metal ion-added hydrogen peroxide could fragment and elute a filter-adsorbed genome in a shorter time as compared with the case wherein a metal ion was not added. With an increased concentration of hydrogen peroxide, the time required for fragmentation of the genomic DNA was shortened and elution could be performed in a shorter time.

It is known that active oxygen acting on nucleic acid not only cleaves the phosphoric acid ester bonds of nucleic acid residues but also damages especially the base portions of the nucleic acid, and therefore by conducting the active oxygen treatment in a short time or promptly removing the active oxygen from the eluted nucleic acid solution, it is possible to minimize the degree of damage to the nucleic acid. The method of removing the active oxygen may be, for example, a method of using a nucleic acid purification column such as NucleoSpin™ (Marcherey-Nagel) to remove the active oxygen in the nucleic acid effluent, a method of using an ultrafiltration membrane to purify and concentrate only the nucleic acid, or a method of purifying the nucleic acid by adding an appropriate amount of alcohol in the presence of a salt to precipitate the nucleic acid, commonly known as the ethanol precipitation method. When it is desired to produce active oxygen in the presence of a metal ion, a metal ion chelating agent may be added to the active oxygen reaction solution containing the metal ion in order to prevent generation of the active oxygen. An alternative is to add an active oxygen eliminator (radical scavenger) to reduce cleavage and damage to the nucleic acid by active oxygen. For example, superoxide dismutase (SOD) is known as a superoxide scavenger, and ascorbic acid and glutathione are well known as hydroxy radical and superoxide scavengers. Genomic DNA eluted with active oxygen treatment was actually purified with a NucleoSpin™ column with success, and was usable as a template for PCR reaction, while hybridization was also possible even when treating the hybridization probe under the same conditions as for active oxygen elution.

As an even more direct method, it was shown that genomic DNA eluted from a nonwoven fabric was usable as a target for hybridization, or as a probe. Specifically, a genomic DNA preparation was biotin-labeled and hybridized with genomic DNA eluted from a nonwoven fabric, or genomic DNA eluted from a nonwoven fabric was biotin-labeled and hybridized with a genomic DNA preparation. The results demonstrated that nucleic acid obtained by both heated elution in TE Buffer or alkali solution and elution with active oxygen can be used both as a target and a probe.

Another feature of the invention is that the nucleic acid-adsorbed nonwoven fabric can be directly used for nucleic acid amplification such as PCR, or nucleic acid sequence detection, without elution of the nucleic acid. Specifically, nucleic acid extraction/purification and nucleic acid amplification or nucleic acid extraction/purification and nucleic acid sequence detection can be accomplished on the same filter. This allows the total procedure, from nucleic acid extraction to examination, to be greatly simplified, while the treatment time is also notably shortened, as no elution procedure is necessary. If nonwoven fabrics are arranged in an array, or nucleic acid is spotted in an array fashion on the same nonwoven fabric, multidetection can be easily performed. The steps from nucleic acid extraction and purification to nucleic acid amplification according to the present invention may be followed by analysis of the target nucleic acid sequence by an examination method such as electrophoresis or a DNA microarray, or by using a mass spectrometer.

A nucleic acid adsorbed onto a nonwoven fabric according to the invention is characterized by its difficult release, and this allows hybridization of probes or nucleic acid extension reaction on the nonwoven fabric without special immobilization protocols. Analysis by scanning electron microscopy reveals fibrous nucleic acid adsorbed onto the fiber surface of the nonwoven fabric. The molecular weight of fibrous nucleic acid seen under an electron microscope is huge, and it is believed that the nucleic acid strands of such long length are very resistant to release because of their interaction with the nonwoven fabric fibers at multiple points. The nucleic acid-adsorbing filter of the invention is also characterized by its difficult release even upon heating if $Mg^{2+}$ or a salt such as NaCl is added to the solution. Consequently, the nucleic acid remains adsorbed onto the filter even under heat denaturing or alkali denaturing conditions commonly employed to convert double-stranded DNA to single-stranded DNA, so that hybridization can be carried out without special immobilization protocols such as UV irradiation. In a nucleic acid amplification reaction, for example, in a PCR reaction conducted by adding to the PCR reaction solution small pieces of nonwoven fabric onto which the genomic DNA is adsorbed for use of the genomic DNA as the template, the PCR amplification product is recovered in the solution.

It is yet another feature of the invention that DNA extension reaction using the adsorbed nucleic acid as a template occurs on the nonwoven fabric. Unlike the PCR amplification product described above, DNA extension chains remain adsorbed to the nonwoven fabric without being released into the solution. This aspect may be utilized to construct a highly sensitive detection system. Addition of labeled nucleotides as the substrates for DNA polymerase during the nucleic acid extension reaction allows the extension reaction to be detected based on incorporation of the labeled nucleotides in the newly synthesized DNA. The labeled nucleotides used may be, for example, fluorescent-labeled nucleotides or digoxigenin (DIG)-labeled nucleotides (Roche Diagnostics). In the case of a DIG system, detection may be accomplished by fluorescence, luminescence or color development in combination with labeled anti-DIG antibody, as is well known. The pyrophosphoric acid produced during DNA synthesis may also be detected instead of labeled nucleotides. The pyrophosphoric acid may be detected by conversion to ATP by a reaction similar to Pyrosequencing and luminescence with a luciferin-luciferase system, or by measuring turbidity upon formation of magnesium pyrophosphate.

According to the invention, the nucleic acid adsorbed on the nonwoven fabric may also be labeled directly. This provides an advantage over labeling of nucleic acid in solution form, in that the unreacted labeling reagent on the nonwoven fabric can be easily removed by washing. Since the labeled nucleic acid on the nonwoven fabric readily elutes by the method described for the invention, it may be used for detection reactions such as hybridization.

The nonwoven fabric of the invention allows treatment of large numbers of cells per unit area or large-volume samples, and therefore the amount of immobilized nucleic acid per unit area (nucleic acid density) can be easily increased. Because a larger amount of nucleic acid for examination facilitates detection, this method is particularly useful in cases requiring high sensitivity, such as infection diagnosis, or in cases of examination where nucleic acid amplification is not desirable and samples are relatively easily obtained.

Brief Description of the Drawings

Fig. 1 shows 0.7% agarose electrophoresis of nucleic acids purified from blood. Lane 1: 1 kb DNA ladder (GibcoBRL); Lane 2: Marker7GT (Nippon Gene); Lane 3: nucleic acid purified with HM-3-coated A040C01; Lane 4: A040C01; Lane 5: P020A (EL); Lane 6: P020C; Lane 7: P090D; Lane 8: N05070; Lane 9: E05070.

Fig. 2 shows 2% agarose electrophoresis of a PCR amplification product. The G3PDH gene was amplified by PCR using nucleic acid purified from blood as a template. Lane 1: 100 bp DNA ladder (GibcoBRL); Lane 2: negative control; Lane 3: positive control; Lane 4: HM-3-coated A040C01; Lane 5: A040C01; Lane 6: P020A (EL); Lane 7: P020C; Lane 8: P090D; Lane 9: N05070; Lane 10: E05070.

Fig. 3 shows 0.7% agarose electrophoresis of nucleic acid amplified from *E. coli.* Lane 1: 1kb DNA ladder (GibcoBRL); Lane 2: Marker7GT (Nippon Gene); Lane 3: nucleic acid purified with HM-3-coated A040C01.

Fig. 4 shows 3% agarose electrophoresis of a PCR amplification product. The ribosomal protein L25 gene was amplified by PCR using nucleic acid purified from *E. coli* as a template. Lane 1: BioMarker Low (BioVentures); Lane 2: negative control; Lane 3: positive control; Lane 4: nucleic acid purified with HM-3-coated A040C01.

Fig. 5 shows blood treatment times. ■: room temperature, +Proteinase K; □: room temperature, -Proteinase K; ●: 50°C, +Proteinase K; ○: 50°C, -Proteinase K.

Fig. 6 shows leukocyte adsorption rate on nonwoven fabrics, and corresponding DNA recovery yields.

Fig. 7 shows DNA elution under alkaline conditions. A nucleic acid-adsorbed nonwoven fabric was immersed in TE Buffer, 0.2N NaOH or 0.05N NaOH and heated at 95°C for 5-20 minutes, and the amount of eluted DNA was measured. ♦: TE Buffer; ■: 0.2N NaOH; ▲: 0.05N NaOH.

Fig. 8 shows 0.7% agarose electrophoresis of nucleic acid eluted under alkaline conditions. Lane 1: 1 kb DNA ladder (GibcoBRL); Lane 2: Marker7GT (Nippon Gene); Lane 3: TE Buffer, 95°C, 20 min; Lane 4: 0.2N NaOH, 95°C, 5 min; Lane 5: 0.2N NaOH, 95°C, 10 min; Lane 6: 0.2N NaOH, 95°C, 20 min; Lane 7: 0.05N NaOH, 95°C, 5 min; Lane 8: 0.05N NaOH, 95°C, 10 min; Lane 9: 0.05N NaOH, 95°C, 20 min.

Fig. 9 shows 2% agarose electrophoresis of a PCR amplification product. The G3PDH gene was amplified by PCR using nucleic acid eluted under alkaline conditions as a template. Lane 1: 100 bp DNA ladder (GibcoBRL); Lane 2: negative control; Lane 3: positive control; Lane 4: TE Buffer, 95°C, 20 min; Lane 5: 0.2N NaOH, 95°C, 5 min;

Lane 6: 0.2N NaOH, 95°C, 10 min; Lane 7: 0.2N NaOH, 95°C, 20 min; Lane 8: 0.05N NaOH, 95°C, 5 min; Lane 9: 0.05N NaOH, 95°C, 10 min; Lane 10: 0.05N NaOH, 95°C, 20 min.

Fig. 10 shows the effect of temperature on alkaline elution. □: TE Buffer; ■: 0.2N NaOH; ▩: 0.05N NaOH.

Fig. 11 shows 0.7% agarose electrophoresis of nucleic acid eluted under acidic conditions. Lane 1: 1 kb DNA ladder (GibcoBRL); Lane 2: Marker7GT (Nippon Gene); Lane 3: TE Buffer, 95°C, 20 min; Lane 4: 10 mM Citrate (pH 4.5)/1 mM EDTA, 95°C, 10 min.

Fig. 12 shows 2% agarose electrophoresis of a PCR amplification product. The G3PDH gene was amplified by PCR using nucleic acid eluted under acidic conditions as a template. Lane 1: 100 bp DNA ladder (GibcoBRL); Lane 2: negative control; Lane 3: positive control; Lane 4: TE Buffer, 95°C, 20 min; Lane 5: 10 mM Citrate (pH 4.5)/1 mM EDTA, 95°C, 10 min.

Fig. 13 shows the effects of alkaline elution on a DNA probe. A DIG-labeled DNA probe was treated with 0.05N NaOH for 5 minutes at 95°C (spots 1,2) and untreated (3,4). The amounts of immobilized Lambda DNA were 10 ng (spots 1,3) and 1 ng (spots 2,4).

Fig. 14 shows 0.7% agarose electrophoresis of nucleic acid eluted with hydrogen peroxide. Elution was performed with 3% hydrogen peroxide containing 0.1 mM $CuCl_2$. Lane 1: 1 kb DNA ladder (GibcoBRL); Lane 2: hydrogen peroxide treatment, room temperature, 1 min; Lane 3: hydrogen peroxide treatment, room temperature, 2 min; Lane 4: hydrogen peroxide treatment, room temperature, 3 min; Lane 5: hydrogen peroxide treatment, room temperature, 5 min.

Fig. 15 shows 2% agarose electrophoresis of a PCR amplification product using hydrogen peroxide-eluted nucleic acid as a template. A human G3PDH partial sequence was amplified by PCR using nucleic acid eluted with 3% hydrogen peroxide containing 0.1 mM $CuCl_2$ as a template. Lane 1: 100 bp DNA ladder (GibcoBRL); Lane 2: hydrogen peroxide treatment, room temperature, 1 min; Lane 3: hydrogen peroxide treatment, room temperature, 2 min; Lane 4: hydrogen peroxide treatment, room temperature, 3 min; Lane 5: hydrogen peroxide treatment, room temperature, 5 min.

Fig. 16 shows 0.7% agarose electrophoresis of nucleic acid eluted with a reducing sugar and metal ion. Elution was performed with an active oxygen solution containing 100 mM D-ribose 5-phosphate and 0.1 mM $CuCl_2$. Lane 1: 1 kb DNA ladder (GibcoBRL); Lane 2: 50°C, 1 min; Lane 3: 50°C, 3 min; Lane 4: 50°C, 5 min; Lane 5: 50°C, 10 min; Lane 6: 50°C, 30 min; Lane 7: Elution with TE Buffer, 95°C, 20 min (control).

Fig. 17 shows 2% agarose electrophoresis of a PCR amplification product obtained using nucleic acid eluted with a reducing sugar as a template. A human G3PDH partial sequence was amplified by PCR using nucleic acid eluted with an active oxygen solution containing 100 mM D-ribose 5-phosphate and 0.1 mM $CuCl_2$ as a template. Lane 1: 100 bp DNA ladder (GibcoBRL); Lane 2: 50°C, 1 min; Lane 3: 50°C, 3 min; Lane 4: 50°C, 5 min; Lane 5: 100 bp DNA ladder; Lane 6: 50°C, 10 min; Lane 7: 50°C, 30 min.

Fig. 18 shows 0.7% agarose electrophoresis of nucleic acid eluted from a nonwoven fabric with a restriction enzyme. Lane 1: 1 kb DNA ladder (GibcoBRL); Lane 2: room temperature, 5 min; Lane 3: room temperature, 5 min (column purification); Lane 4: room temperature, 10 min; Lane 5: room temperature, 10 min (column purification); Lane 6: room temperature, 30 min; Lane 7: room temperature, 30 min (column purification); Lane 8: 37°C, 5 min; Lane 9: 37°C, 5 min (column purification); Lane 10: 37°C, 10 min; Lane 11: 37°C, 10 min (column purification); Lane 12: 37°C, 30 min; Lane 13: 37°C, 30 min (column purification); Lane 14: 1kb DNA ladder (GibcoBRL).

Fig. 19 shows the effect of active oxygen treatment on hybridization. Hybridization was performed using a DIG-labeled probe treated with final concentrations of 50 mM D-ribose 5-phophate and 25 μM $CuCl_2$, and DNA immobilized on a nylon membrane. DIG-labeled DNA probes without active oxygen treatment or treated in TE Buffer at 95°C for 20 minutes were used as controls.

Fig. 20 shows PCR performed using nonwoven fabric-adsorbed human genomic DNA as a template. Blood-extracted nucleic acid was adsorbed onto a nonwoven fabric and used for amplification of the G3PDH gene by PCR. Lane 1: 100 bp DNA ladder (GibcoBRL); Lane 2: negative control; Lane 3: positive control; Lane 4: HM-3-coated A040C01; Lane 5: A040C01; Lane 6: P020A (EL); Lane 7: P090D; Lane 8: N05070; Lane 9: E05070.

Fig. 21 shows PCR performed using nonwoven fabric-adsorbed *E. coli* genomic DNA as a template. Nucleic acid extracted from *E. coli* was adsorbed onto HM-3-coated A040C01 and used for amplification of the ribosomal protein L25 gene by PCR. Lane 1: BioMarker Low (BioVentures); Lane 2: negative control; Lane 3: positive control; Lane 4: HM-3-coated A040C01.

Fig. 22 shows detection of nonwoven fabric-adsorbed human genomic DNA. A radioisotope-labeled DNA probe was hybridized onto two different nonwoven fabrics HM-3-coated A040C01 and A040C01, onto which were adsorbed human genomic DNA extracted from 0.25 ml of blood. Hybond-N+ nylon membranes (Amersham Pharmacia Biotech) immobilizing 1 μg of human genomic DNA (hgDNA) and 1 μg of Lambda DNA (λDNA) were used as a control. The legend indicates the mixing proportions of the human G3PDH probe and Lambda DNA probe. All the mixtures had a total radioactivity of 448,000 cpm.

Fig. 23 shows scanning electron micrographs of nonwoven fabric-adsorbed nucleic acid.

A: Nonwoven fabric P03050 (control), B: Blood nucleic acid-adsorbed nonwoven fabric P03050.
Fig. 24 shows the examination of surfactants used in cytolytic solutions.
X-114 (Triton X-100), TOC (Nissan Dispanol TOC), X-100 (Triton X-100), CA630 (Igepal CA630), NS-208.5 (Nissan Nonion NS-208.5), HPC (hexadecylpyridinium chloride), HPB (hexadecylpyridinium bromide), HTAC (hexadecyltrimethylammonium chloride), HTAB (hexadecyltrimethylammonium bromide), CHAPS (3-[(3-Cholamidopropyl) dimethylammonio]-1-propanesulfonate), CHAPSO (3-[(3-Cholamidopropyl)dimethylammonio]-2-hydroxy-1-propanesulfonate), SDS (sodium dodecyl sulfate).
Fig. 25 shows the dependency on NaCl concentration of purified genomic DNA adsorption.
♦ A040C01; ▲ A066A; ■ A040B
Fig. 26 shows the dependency on $MgCl_2$ concentration of purified genomic DNA adsorption.
♦ A040C01; ▲ A066A; ■ A040B; ● E01030
Fig. 27 shows the dependency on $HPO_4^{2-}$ concentration of purified genomic DNA adsorption.
♦ A040C01; ▲ A066A; ■ A040B; ● A040C01/HM-3
Fig. 28 shows the dependency on $(NH_4)_2SO_4$ concentration of purified genomic DNA adsorption.
♦ A040C01; ■ A066A; ▲ E01030
Fig. 29 shows the effect of ethanol on purified genomic DNA adsorption.
Ethanol was added to 10 mM phosphoric acid buffer (pH 7.4) and the effect on purified genomic DNA adsorption was examined.
■ 10 mM phosphoric acid (pH 7.4); ▨ 10 mM phosphoric acid (pH 7.4)/10% ethanol; ▩ 10 mM phosphoric acid (pH 7.4) /20% ethanol; ⛶ 10 mM phosphoric acid (pH 7.4)/40% ethanol.
Fig. 30 shows shaking adsorption of purified genomic DNA (1).
■ 10 mM Tris (pH 8)/1 mM EDTA/50 mM NaCl; ▨ 10 mM Tris (pH 8)/2 mM $MgCl_2$; 50 m ⛶ M $Na_2HPO_4/NaH_2PO_4$ (pH 7.4).
Fig. 31 shows shaking adsorption of purified genomic DNA (2)
■ 10 mM phosphoric acid (pH 7.4); ▩ 10 mM phosphoric acid (pH 7.4)/0.2M ammonium sulfate; □10 mM phosphoric acid (pH 7.4)/0.5M ammonium sulfate; □10 mM phosphoric acid (pH 7.4)/1.0M ammonium sulfate.
Fig. 32 shows screening of nonwoven fabrics by DNA yields. Fresh blood was used as specimens. Gr1 to Gr5 represent different blood donors of the blood used.
Fig. 33 shows screening of nonwoven fabrics by DNA purity ($A_{260}/A_{280}$). The same samples as in Fig. 31 were used for measurement of the absorbance ratio $A_{260}/A_{280}$.
Fig. 34 shows nucleic acid purification from *E. coli*-added sputum by heat treatment (1). These are the results of detecting the *E. coli*-derived nucleic acid of Example 27. PCR amplification was followed by analysis by agarose gel electrophoresis.
Lane 1: DNA molecular weight marker; Lane 2: PCR amplification product of nucleic acid extract obtained from $10^5$ *E. coli*-added sputum specimen; Lane 3: PCR amplification product of nucleic acid extract obtained from $10^4$ *E. coli*-added sputum specimen; Lane 4: PCR amplification product of nucleic acid extract obtained from $10^3$ *E. coli*-added sputum specimen; Lane 5: PCR amplification product of nucleic acid extract obtained from *E. coli*-free sputum specimen.
Fig. 35 shows nucleic acid purification from *E. coli*-added sputum by heat treatment (2). These are the results of detecting the human-derived nucleic acid of Example 27. PCR amplification was followed by analysis by agarose gel electrophoresis.
Lane 1: DNA molecular weight marker; Lane 2: PCR amplification product of nucleic acid extract obtained from $10^5$. *E. coli*-added sputum specimen; Lane 3: PCR amplification product of nucleic acid extract obtained from $10^4$ *E. coli*-added sputum specimen; Lane 4: PCR amplification product of nucleic acid extract obtained from $10^3$ *E. coli*-added sputum specimen; Lane 5: PCR amplification product of nucleic acid extract obtained from *E. coli*-free sputum specimen.
Fig. 36 shows nucleic acid purification from *E. coli*-added sputum by reducing agent treatment (1). These are the results of detecting the *E. coli*-derived nucleic acid of Example 28. PCR amplification was followed by analysis by agarose gel electrophoresis.
Lane 1: DNA molecular weight marker; Lane 2: PCR amplification product of nucleic acid extract obtained from $10^5$ *E. coli*-added sputum specimen; Lane 3: PCR amplification product of nucleic acid extract obtained from $10^4$ *E. coli*-added sputum specimen; Lane 4: PCR amplification product of nucleic acid extract obtained from $10^3$ *E. coli*-added sputum specimen; Lane 5: PCR amplification product of nucleic acid extract obtained from *E. coli*-free sputum specimen.
Fig. 37 shows nucleic acid purification from *E. coli*-added sputum by reducing agent treatment (2). These are the results of detecting the human-derived nucleic acid of Example 28. PCR amplification was followed by analysis by agarose gel electrophoresis.
Lane 1: DNA molecular weight marker; Lane 2: PCR amplification product of nucleic acid extract obtained from $10^5$ *E. coli*-added sputum specimen; Lane 3: PCR amplification product of nucleic acid extract obtained from $10^4$ *E. coli*-added sputum specimen; Lane 4: PCR amplification product of nucleic acid extract obtained from $10^3$ *E. coli*-added sputum specimen; Lane 5: PCR amplification product of nucleic acid extract obtained from *E. coli*-free sputum specimen.

Fig. 38 shows nucleic acid extension reaction on a nonwoven fabric. The two primers bACT1 and bACT2 were hybridized to a Klenow Large Fragment enzyme reaction solution in amounts of 10 μg, 100 ng, 1 ng and 0 ng, respectively and an extension reaction was conducted at 37°C.

Fig. 39 shows amplification and detection of nucleic acid on a nonwoven fabric by the LAMP method. Bovine genomic DNA amplified with a Loopamp was subjected to 1% agarose gel electrophoresis. Lane 1: molecular weight marker; Lane 2: human genomic DNA-adsorbed nonwoven fabric as a template; Lane 3: bovine genomic DNA-adsorbed nonwoven fabric as a template; Lane 4: supernatant of bovine genomic DNA-adsorbed nonwoven fabric heat eluted in TE Buffer as a template; Lane 5: no template; Lane 6: kit-supplied control bovine genomic DNA as a template.

Fig. 40 shows hybridization of genomic DNA purified with nonwoven fabric (1). The test was conducted by the TE Buffer elution method and alkaline elution method. A 1 μl portion of DNA solution purified with a nonwoven fabric was dotted on a Hybond N+ membrane and used as the target for hybridization.

Fig. 41 shows hybridization of genomic DNA purified with nonwoven fabric (2). The test was conducted by the TE Buffer elution method and hydrogen peroxide elution method. A 1 μl portion of DNA solution purified with a nonwoven fabric was dotted on a Hybond N+ membrane and used as the target for hybridization.

Fig. 42 shows hybridization of genomic DNA purified with nonwoven fabric (3). The test was conducted by the TE Buffer elution method, alkaline elution method and hydrogen peroxide elution method, using labeled DNA purified with a nonwoven fabric as the hybridization probe.

Fig. 43 is a graph showing elution of nucleic acid using a surfactant. A 500 μl portion of an aqueous solution or TE Buffer containing 0.5% of a surfactant was added, and heating was carried out at 80°C for 20 minutes for elution of the nucleic acid adsorbed on the nonwoven fabric. The amount of nucleic acid eluted upon heating in TE Buffer at 95°C for 20 minutes was defined as 100%.

Fig. 44 shows electrophoresis of nucleic acid eluted with a surfactant. The eluted sample of Fig. 43 was concentrated. and purified with a NucleoSpin column and subjected to 0.7% agarose gel electrophoresis.

Fig. 45 shows electrophoresis of a PCR amplification product of nucleic acid eluted with a surfactant. The eluted sample of Fig. 43 was used as the template for PCR amplification of the G3PDH gene.

Fig. 46 shows electrophoresis of a PCR product. The surfactant used for genome elution was added to a PCR reaction system for PCR amplification of the G3PDH gene. A 1 kb ladder (GibcoBRL) was used as the DNA marker.

Fig. 47 shows hybridization using a Biotin Chem-Link-labeled nucleic acid probe. A Biotin Chem-Link-labeled nucleic acid probe 1 or 2 was hybridized to a membrane dotted with human genomic DNA or Lambda DNA.

## Examples

The present invention will now be explained more definitely through the following examples. However, these examples serve merely for the purpose of illustration and do not restrict the technical scope of the invention.

[Example 1] Purification of nucleic acid from fresh human blood with nonwoven fabric

Blood was taken from a healthy donor and heparin sodium (Shimizu Pharmaceutical) was added as an anticoagulant at 10 units per milliliter of blood. The leukocyte count of the blood was measured with a flow cytometer (FACS Calibur, Becton Dickinson) using a LeucoCOUNT Kit (Becton Dickinson). The leukocyte count in 0.25 ml of blood was 1.16 x $10^6$. The nonwoven fabric used was a product by Asahi Kasei Corp. The nonwoven fabric was cut into 12 mm-diameter disks, four of which were stacked and set in a filter holder (SWINNEX, MILLIPORE), with a 10 ml glass syringe set upstream and a suction pump set downstream in connection with the filter holder. The nonwoven fabric was initially washed with 3 ml of Digestion Buffer (10 mM Tris, pH 8; 100 mM NaCl; 25 mM EDTA; 0.5% SDS).

Next, 0.05 pg of Proteinase K (PCR-Grade, Roche) was added to 0.25 ml of the human blood, and after further adding 0.25 ml of 2x Digestion Buffer (20 mM Tris, pH 8; 200 mM NaCl; 50 mM EDTA; 1% SDS), the mixture was allowed to stand at room temperature for 5 minutes. This treatment resulted in complete disruption of the erythrocytes and leukocytes. The blood extract was applied to the nonwoven fabric and immediately subjected to suction filtration. The filter was then washed by pouring 8 ml of Digestion Buffer under suction.

Next, the nonwoven fabric was further washed by pouring 3 ml of 1 M NaCl-containing PBS/1 mM EDTA and finally 3 ml of TE Buffer (10 mM Tris, pH 8; 1 mM EDTA). The four nonwoven fabric disks were removed from the filter holder and placed in a lock-equipped Eppendorf tube, and 0.5 ml of TE Buffer was added. After incubating at 80°C for 1 hour, the $A_{260}$ (absorbance at 260 nm) and $A_{280}$ (absorbance at 280 nm) of the effluent were measured with a UV-1600 UV-visible light spectrophotometer (Shimadzu). An effluent with an $A_{260}/A_{280}$ ratio in the range of 1.8-2.0 is considered to be essentially pure nucleic acid. Table 1 shows a list of nonwoven fabrics which had $A_{260}/A_{280}$ ratios in the range of 1.8-2.0. The DNA concentrations were calculated by the following formula (using software bundled with UV1600: DNA/PROTEIN PROGRAM PACK Ver. 2.00).

$$\text{DNA concentration } (\mu g/ml) = K1*A_{260} - K2*A_{280}$$

(K1 = 62.90, K2 = 36.00)

Table 1

| Product name | Nonwoven fabric | Material | DNA (μg) | $A_{260}/A_{280}$ |
|---|---|---|---|---|
| | HM-3-coated A040C01 | polyethylene terephthalate | 5.7 | 1.86 |
| | A040C01 | polyethylene terephthalate | 4.3 | 2.01 |
| MICROWEB® | P020A(EL) | polypropylene | 2.3 | 1.94 |
| MICROWEB® | P020C | polypropylene | 3.8 | 1.94 |
| MICROWEB® | P090D | polypropylene | 4.0 | 1.91 |
| ELTAS® | N05070 | nylon | 4.7 | 1.93 |
| ELTAS® | E05070 | polyester | 5.1 | 1.88 |

[Example 2] Analysis of purified human nucleic acid

The purified nucleic acid obtained in Example 1 was subjected to 0.7% agarose electrophoresis and the sizes were confirmed. After adding 1.5 μl of 10x Loading Buffer (1% SDS, 50% glycerol, 0.05% Bromophenol Blue, TaKaRa) to 10 μl of the effluent of Example 1 and thoroughly mixing, the total amount was subjected to 0.7% agarose electrophoresis. After electrophoresis in a Mupid Minigel Migration Tank (Advance) at 50 V for 90 minutes, the gel was stained with ethidium bromide and photographed with a BioImage Gel Print 2000i/VGA. As shown in Fig. 1, the purified nucleic acid contained nucleic acid fragments of various sizes of from several kilobases to several tens of kilobases.

It was then confirmed that the purified DNA obtained in Example 1 could be used as a PCR template. A glyceraldehyde 3-Phosphate Dehydrogenase (G3PDH) 0.45 kb Control Amplimer Set by Clontech (Cat. No. 5405-3) was used for primers. The effluent of Example 1 was diluted 10-fold with distilled water and 5 μl thereof was added to a PCR reaction mixture (final concentration: 10 mM Tris-HCl, pH 8.3; 50 mM KCl; 1.5 mM $MgCl_2$; 0.2 mM dATP; 0.2 mM dGTP; 0.2 mM dCTP; 0.2 mM dTTP; 1.25 U AmpliTaq (Applied Biosystems); 0.5 pM G3PDH 3'-primer; 0.5 μM G3PDH 5'-primer) to a total volume of 50 μl.

The mixture was reacted in a DNA Thermal Cycler (Perkin Elmer) with 1 cycle of 94°C, 5 min; 30 cycles of 94°C, 30 sec, 55°C, 1 min, 72°C, 1.5 min; and then 72°C for 7 minutes. After completion of the PCR reaction, 1.5 μl of 10x Loading Buffer was added to 10 μl of the reaction solution and thoroughly mixed therewith, and the total amount was subjected to 2% agarose electrophoresis. After electrophoresis at 50 V for 45 minutes, the gel was stained with ethidium bromide and photographed with a BioImage Gel Print 2000i/VGA. The results are shown in Fig. 2. A 452 bp PCR product was amplified from all of the purified DNA fractions, indicating that they were usable as templates for PCR.

[Example 3] Purification of *E. coli* nucleic acid

HM-3-coated A040C01(Asahi Kasei) was cut into 12 mm-diameter disks, four of which were stacked and set in a filter holder (SWINNEX, MILLIPORE), with a 10 ml glass syringe set upstream and a suction pump set downstream from the filter holder. The nonwoven fabric disks were initially washed with 3 ml of Digestion Buffer (10 mM Tris, pH 8; 100 mM NaCl; 25 mM EDTA; 0.5% SDS).

After adding 50 μl of *E. coli* DH5 glycerol stock to 3 ml of LB medium (1 g Tryptone Peptone (DIFCO); 0.5 g Yeast Extract (DIFCO); 1 g NaCl; 200 μl 1N NaOH; 100 ml distilled water), the mixture was cultured at 37°C for 4.5 hours to obtain a culture solution with $A_{600}$ = 1.56. The *E. coli* density based on absorbance was considered to be approximately 6.2 x $10^9$ cells/ml. A 1.6 ml portion of the culture solution ($10^{10}$ *E. coli* cells) was taken and centrifuged at 15,000 rpm for 1 minute. The cell precipitate was suspended in 0.25 ml of LB medium. After adding 0.05 μg/2.6 μl of Proteinase K (PCR-Grade, Roche) and then 0.25 ml of 2x Digestion Buffer (20 mM Tris, pH 8; 200 mM NaCl; 50 mM EDTA; 1% SDS), the mixture was allowed to stand at room temperature for 5 minutes. The extract was placed on the previously prepared HM-3-coated A040C01 and immediately subjected to suction filtration. The filter was then washed by pouring 8 ml of Digestion Buffer under suction.

Next, the HM-3-coated A040C01 was further washed by pouring 3 ml of 1 M NaCl-containing PBS/1 mM EDTA and finally 3 ml of TE Buffer (10 mM Tris, pH 8; 1 mM EDTA). The four HM-3-coated A040C01 were removed from the filter holder and placed in a lock-equipped Eppendorf tube, and 0.5 ml of TE Buffer was added. After incubating at 80°C for 1 hour, the absorbance of the effluent was measured. The yield was 64.1 μg of purified nucleic acid with an $A_{260}/A_{280}$ ratio of 1.84.

[Example 4] Analysis of *E. coli* nucleic acid

The purified nucleic acid obtained in Example 3 was subjected to 0.7% agarose electrophoresis and the sizes were confirmed. After adding 1.5 μl of 10x Loading Buffer (1% SDS, 50% glycerol, 0.05% Bromophenol Blue, TaKaRa) to 10 μl of the effluent of Example 3 and thoroughly mixing, the total amount was subjected to 0.7% agarose electrophoresis. After electrophoresis in a Mupid Minigel Migration Tank (Advance) at 50 V for 90 minutes, the gel was stained with ethidium bromide and photographed with a BioImage Gel Print 2000i/VGA. As shown in Fig. 3, the purified nucleic acid contained nucleic acid fragments of various sizes of from several hundred bases to several tens of kilobases.

It was then confirmed that the purified DNA obtained in Example 3 could be used as a PCR template. As PCR primers, the following chemically synthesized sequences were ordered from Sigma Co.: the nucleic acid sequence from C at position 393 to A at position 413 ( SEQ ID NO:1) and the sequence complementary to the nucleic acid sequence from C at position 567 to A at position 587 ( SEQ ID NO:2), of gene rplY coding for the *E. coli* ribosomal protein L25. The length of the PCR amplification product was 195 bp. The effluent of Example 3 was diluted 4000-fold with distilled water and 10 μl thereof was added to a PCR reaction mixture for a total of 25 μl (final concentration: 60 mM Tris/15 mM $(NH_4)_2SO_4$, pH 10.0; 50 mM KCl; 3.5 mM $MgCl_2$; 0.2 mM dATP; 0.2 mM dGTP; 0.2 mM dCTP; 0.2 mM dTTP; 1.25 U TaKaRa Ex Taq (Takara Shuzo); 0.5 μM of each primer).

The mixture was reacted in a DNA Thermal Cycler (Perkin Elmer) with 1 cycle of 94°C, 2 min; 30 cycles of 94°C, 1 min, 55°C, 1 min, 72°C, 1 min; and then 72°C for 7 minutes. After completion of the PCR reaction, 1.5 μl of 10x Loading Buffer was added to 10 μl of the reaction solution and thoroughly mixed therewith, and the total amount was subjected to 3% NuSieve 3:1 agarose electrophoresis (BioWhittaker Molecular Applications). After electrophoresis at 100 V for 40 minutes with a Mupid Minigel Migration Tank (Advance), the gel was stained with ethidium bromide and photographed with a BioImage Gel Print 2000i/VGA. The results are shown in Fig. 4. A 195 bp PCR product was amplified, confirming that the mixture was usable as a template for PCR.

[Example 5] Purification of nucleic acid from cryopreserved human blood

Blood was taken from a healthy donor and heparin sodium (Shimizu Pharmaceutical) was added as an anticoagulant at 10 units per milliliter of blood. The leukocyte count of the blood was measured with a flow cytometer (FACS Calibur, Becton Dickinson) using a LeucoCOUNT Kit (Becton Dickinson). The leukocyte count in 0.25 ml of blood was $1.16 \times 10^6$.

A 0.25 ml portion of blood was placed in an Eppendorf tube and frozen at -80°C. After adding 0.25 ml of 2x Digestion Buffer (20 mM Tris, pH 8; 200 mM NaCl; 50 mM EDTA; 1% SDS) heated at 37°C and then 0.05 μg of Proteinase K (PCR-Grade, Roche) to 0.25 ml of the cryopreserved blood, the mixture was stirred in a vortex while periodically heating it in a 37°C water bath, to complete dissolution. After allowing the solution to stand at room temperature for 5 minutes, the blood extract was applied to the nonwoven fabric and immediately subjected to suction filtration. A nucleic acid was prepared thereafter according to the method described in Example 1. Fresh blood was treated in the same manner as a control. The results are shown in Table 2. Purified nucleic acid was obtained from the cryopreserved blood in the same manner as from the fresh blood.

Table 2

| | DNA (μg) | $A_{260}/A_{280}$ |
|---|---|---|
| Fresh blood | 4.4 | 1.92 |
| Cryopreserved blood | 3.0 | 1.90 |

[Example 6] Adsorption of genomic DNA onto nonwoven fabric

To $6.20 \times 10^7$ human peripheral leukocytes there were added 620 μl of Digestion Buffer (10 mM Tris, pH 8; 100 mM NaCl; 25 mM EDTA; 0.5% SDS) and Proteinase K (final concentration: 0.1 mg/ml, PCR-Grade, Roche) to suspend the leukocytes. After incubation at 50°C for 12 hours, an equivalent of phenol/chloroform/isoamyl alcohol = 25:24:1 (GibcoBRL) was added and thoroughly mixed therewith prior to centrifugation at 1700xg for 10 minutes. The supernatant was obtained and the same procedure was repeated twice. The supernatant was then transferred to a dialysis tube and dialyzed three times against 100 volumes of TE Buffer (10 mM Tris, pH 8; 1 mM EDTA) at 4°C. A

531 μg portion of genomic DNA with an $A_{260}/A_{280}$ ratio of 1.76 was obtained.

An HM-3-coated A040C01 nonwoven fabric was cut into 12 mm-diameter disks, four of which were stacked and set in a filter holder (SWINNEX, MILLIPORE). A 10 ml glass syringe was set upstream from the filter holder, and 3 ml of PBS was poured thereover using a TE-311 Terfusion Syringe Pump (Terumo) at a flow rate of 26.2 ml/hr to wash the nonwoven fabric disks. Next, 26.3 μg of the genomic DNA was dissolved in 1 ml of PBS and filtered with the HM-3-coated A040C01. The nonwoven fabric disks were then washed with 3 ml of PBS. All of the filtrates were recovered. Finally, the nonwoven fabric disks were removed from the filter holder and placed in a lock-equipped Eppendorf tube, and 0.5 ml of TE Buffer was added. After incubating at 80°C for 1 hour, the TE Buffer was recovered. The volumes and absorbance of all of the filtrates and effluents were measured to calculate the DNA yields, giving the results shown in Table 3. With filtration of 26.3 μg of DNA through the HM-3-coated A040C01, approximately 65% of the DNA was adsorbed and 51% of the DNA (13.5 μg) was recovered. The DNA yield was calculated by the following formula.

$$\text{Yield (\%)} = 100 * \text{recovered DNA } (\mu g)/26.3\ (\mu g)$$

Table 3

| | Total DNA | Filtration | Washing | Elution |
|---|---|---|---|---|
| DNA (μg) | 26.3 | 6.6 | 2.7 | 13.5 |
| Yield (%) | 100% | 25% | 10% | 51% |

[Example 7] Blood lysis conditions

An experiment was conducted under the same conditions as in Example 1, to examine the effects of blood treatment time, treatment temperature and presence of Proteinase K. HM-3-coated A040C01 was used as the nonwoven fabric. After adding Proteinase K and 2x Digestion Buffer to 0.25 ml of blood, the mixture was treated at room temperature or 50°C for 5-60 minutes and nucleic acid was then purified with the HM-3-coated A040C01. The results are shown in Fig. 5. With treatment at 50°C for 5-15 minutes in the absence of Proteinase K, the blood extract could not pass through the nonwoven fabric due to clogging, making it impossible to recover the nucleic acid. With treatment at room temperature for 5-60 minutes, the nucleic acid was successfully purified both with and without addition of Proteinase K.

[Example 8] Leukocyte adsorption capacity and nucleic acid adsorption capacity

HM-3-coated A040C01, A040C01, N05070 and E05070 were used as nonwoven fabrics. Each nonwoven fabric was cut into 12 mm-diameter disks, four of which were stacked and set in a filter holder (SWINNEX, MILLIPORE). A 10 ml glass syringe was connected upstream from the filter holder, and the glass syringe was set in a TE-311 Terfusion Syringe Pump (Terumo). The nonwoven fabric disks were pretreated by flowing through 3 ml of EtOH followed by 3 ml of PBS. The flow rate was set to 26.2 ml/hr with the syringe pump. After passing 1 ml of blood through the pretreated nonwoven fabric disks, they were washed with 6 ml of PBS. Finally, air was passed through the nonwoven fabric disks to force out all of the washing liquid. All of the emerging blood and washing liquid was recovered (as filtrate), and the volumes and leukocyte counts were measured. The leukocyte counts in the blood before filtration with the nonwoven fabric and in the filtrate were measured with a flow cytometer (FACS Calibur, Becton Dickinson) using a LeucoCOUNT Kit (Becton Dickinson). The leukocyte surface coverage was calculated by the following formula.

Leukocyte surface coverage (%) = 100 * (leukocyte count in 1

ml blood - leukocyte count in filtrate)/(leukocyte count in 1

ml blood)

Fig. 6 shows a plot of the leukocyte surface coverage determined above against the DNA yields shown in Table 1. No correlation was found between leukocyte adsorption capacity (leukocyte removal capacity) and nucleic acid adsorption capacity of the nonwoven fabrics.

[Example 9] Nucleic acid elution under alkaline conditions

An experiment was conducted under the same conditions as in Example 5 to examine the nucleic acid elution under alkaline conditions. The nonwoven fabric used was HM-3-coated A040C01 by Asahi Kasei Corp. A 0.25 ml portion of cryopreserved blood was applied to nonwoven fabric disks by the procedure of Example 5, and after finally washing the nonwoven fabric by pouring 3 ml of TE Buffer, the four nonwoven fabric disks were removed from the filter holder and placed in a lock-equipped Eppendorf tube, and then 0.5 ml of TE Buffer, 0.05 N NaOH or 0.2 N NaOH was added to immerse the nonwoven fabric prior to incubation in a heat block at 95°C for 5-20 minutes. After completion of the reaction, 3 M $NaH_2PO_4$ was added in a 1/10 volume (50 μl) with respect to the 0.2 N NaOH or in a 1/40 volume (12.5 μl) with respect to the 0.05 N NaOH for neutralization.

The amount of eluted DNA was assayed using an OliGreen® ssDNA quantitation kit (Product No. 0-11492, Molecular Probes). An 18-residue oligonucleotide supplied with the kit was used as the standard, and quantitation was performed with an ARVOsx-3 Fluorescent Plate Reader (Wallac Berthold Japan, Co., Ltd.) at Ex 485 nm, Em 535 nm. The effluent from the nonwoven fabric was diluted 10-fold with TE Buffer, and the RNA was removed by adding DNase-free RNase (Product No. 1119915, Roche) to a final concentration of 2.5 μg/ml and conducting the reaction at 37°C for 30 minutes. The treatment degraded the RNA and resulted in virtually no detection in the OliGreen assay system. This was confirmed using 16S and 23S rRNA products.

The measurement results are shown in Fig. 7. The amount of DNA eluted in TE Buffer at 95°C increased with reaction time up to 20 minutes (Fig. 7), reaching a plateau. On the other hand, the same amount of DNA eluted in 5 minutes at 95°C in the 0.05 N or 0.2 N NaOH alkali solution.

A 10 μl portion of the purified DNA was subjected to 0.7% agarose electrophoresis and the sizes were confirmed. As shown in Fig. 8, the sizes of the DNA eluted under alkaline conditions tended to be larger than the DNA eluted with TE Buffer. The sizes of the eluted DNA decreased with longer reaction times of 5 minutes, 10 minutes and 20 minutes at 95°C. Elution with 0.05 N NaOH produced larger DNA sizes than elution with 0.2 N NaOH, but in both cases the DNA sizes reached from several kilobases to several hundred kilobases.

It was then confirmed that the purified DNA eluted under alkaline conditions could be used as a PCR template. The PCR and its analysis were performed in the same manner as Example 2. A glyceraldehyde 3-phosphate dehydrogenase (G3PDH) 0.45 kb Control Amplimer Set by Clontech was used for primers. The results are shown in Fig. 9. A 452 bp PCR product was amplified even from the purified DNA eluted under alkaline conditions, indicating that the DNA was usable as a template for PCR.

[Example 10] Effect of temperature on alkaline elution

An HM-3-coated A040C01 nonwoven fabric having blood-derived nucleic acid adsorbed thereon was prepared according to the method of Example 9, and the effect of elution temperature was examined. The elution was performed with TE Buffer at 95°C for 20 minutes; with 0.05 N NaOH at 95°C or 70°C for 10 minutes; and with 0.2 N NaOH at 95°C, 70°C, 60°C, 50°C or 40°C for 10 minutes, and the amounts of the eluted DNA were compared. The amounts of DNA were assayed using an OliGreen® ssDNA quantitation kit in the same manner as in Example 9. As shown in Fig. 10, the amount of the eluted DNA increased with increasing temperature, reaching a notable level at 70°C and above.

[Comparative Example 1] DNA elution under acidic conditions

A HM-3-coated A040C01 nonwoven fabric having blood-derived nucleic acid adsorbed thereon was prepared according to the method of Example 9. The elution was performed with TE Buffer at 95°C for 10 minutes and 20 minutes or with 10 mM citrate (pH 4.5)/1 mM EDTA Buffer at 95°C for 10 minutes. After elution, 1 M Tris (pH 8.0) was immediately added to the effluent for neutralization. Next, the $A_{260}$ (absorbance at 260 nm) and $A_{280}$ (absorbance at 280 nm) of the effluent were measured with a UV-1600 UV-visible light spectrophotometer (Shimadzu), and the eluted nucleic acid amount was calculated. The results are shown in Table 4. The DNA concentrations were calculated by the following formula (using software bundled with UV1600: DNA/PROTEIN PROGRAM PACK Ver. 2.00).

$$\text{DNA concentration } (\mu g/ml) = K1*A_{260} - K2*A_{280}$$

(K1 - 62.90, K2 = 36.00)

Table 4

| | Eluted DNA (μg) |
|---|---|
| TE, 95°C, 10 min | 1.1 |
| TE, 95°C, 20 min | 6.4 |
| pH 4.5, 95°C, 10 min | 4.5 |

The eluted DNA was subjected to 0.7% agarose electrophoresis in the same manner as in Example 9 to confirm the sizes. The results are shown in Fig. 11. The sizes of the nucleic acid eluted at pH 4.5, 95°C, 10 min. were in the range of from several kilobases to several tens of kilobases. It was then confirmed that the purified DNA eluted under acidic conditions could be used as a PCR template. This was also accomplished by the same method as in Example 9. It was found that the DNA eluted under acidic conditions was also suitable for PCR (Fig. 12).

[Reference Example 1] Effect of alkaline elution on DNA probes

In order to evaluate whether DNA which is alkali-eluted from a nonwoven fabric can be used for hybridization, hybridization was carried out using digoxigenin-11-dUTP (DIG)-labeled DNA under the same conditions as those for alkaline elution. DIG labeling of the DNA was accomplished using a Roche PCR DIG Probe Synthesis Kit and PCR DIG Labeling Mix, and the detection of hybridization was accomplished using a Roche DIG-High Prime DNA Labeling/ Detection Kit. After preparing 50 μl of PCR reaction solution (PCR buffer containing 1.5 mM $MgCl_2$ (final concentration); 0.2 mM dATP; 0.2 mM dGTP; 0.2 mM dCTP; 0.19 mM dTTP; 0.01 mM digoxigenin-11-dUTP; 2.6 U Expand High Fidelity AmpliTaq) containing 1 ng Lambda DNA (Takara) and the chemically synthesized DNA primer of SEQ ID NO:3(final concentration: 0.4 μM) and DNA primer of SEQ ID NO:4 (final concentration: 0.4 μM), ordered from Nihon Bioservice, the solution was reacted in a DNA Thermal Cycler (Perkin Elmer) with 1 cycle of 94°C, 3 min; 30 cycles of 94°C, 30 sec, 60°C, 1 min, 72°C, 3 min; and then 72°C for 5 minutes. The labeled DNA was purified with a NucleoSpin column by Macherey-Nagel, yielding a 50 μl DNA solution. After then adding NaOH to a final concentration of 0.05 N or 0.2 N, the mixture was incubated in a heat block at 95°C for 5-20 minutes and placed under the same conditions as those for alkaline elution. The incubation was followed by neutralization by the addition of 3 M $NaH_2PO_4$ in a 1/10 volume with respect to 0.2 N NaOH or in a 1/40 volume with respect to 0.05 N NaOH. The DNA solution was again purified with a NucleoSpin column to obtain 50 μl of a DIG-labeled DNA probe for hybridization. Lambda DNA was blotted on a Hybond N+ membrane (Amersham-Pharmacia) at from 0.01 ng to 10 pg, and after alkali degeneration for single strand conversion, they were immobilized on the membrane by UV crosslinking. This was immersed in a DIG Easy Hyb (Roche) solution and subjected to prehybridization at 42°C for 3 hours, after which the previous heat-denatured single-stranded DIG-labeled DNA probe was added and incubation was performed at 42°C for 18 hours for hybridization. This was followed by twice washing for 5 minutes at room temperature using 2xSSC, 0.1% SDS, and then twice washing for 15 minutes at 68°C using 0.1XSSC, 0.1% SDS. After equilibrating for 1 minute with a washing buffer (final concentrations: 0.1 M maleic acid; 0.15 M NaCl; 0.3% Tween20, pH 7.5), the membrane was immersed for 1 hour in a blocking solution (Roche) diluted 10-fold with maleic acid buffer (final concentrations: 0.1 M maleic acid; 0.15 M NaCl, pH 7.5). After then adding 3 μl of alkali phosphatase-labeled anti-DIG antibody (Roche), the mixture was placed at room temperature for 30 minutes. The washing buffer was used for washing twice for 15 minutes at room temperature, followed by equilibration for 2 minutes with alkali phosphatase buffer (final concentrations: 0.1 M Tris, pH 9.5; 0.1 M NaCl; 50 mM $MgCl_2$) and addition of a 1/100 volume of CSPD ready-to-use (Roche) prior to incubation for 20 minutes at 37°C. After photosensitization for 30 minutes with a Hyper ECL film (Amersham-Pharmacia), it was developed with a developing machine (Konica). As a result, it was possible to detect a signal of the alkali solution-treated DNA probe with the same strength as that of the non-alkali solution-treated DNA probe (Fig. 13). These results suggest that DNA eluted under the alkaline conditions indicated in Example 9 is usable for hybridization.

[Example 11] Nucleic acid elution with hydrogen peroxide water

An experiment was conducted under the same conditions as those in Example 5 to examine nucleic acid elution with hydrogen peroxide water. The nonwoven fabric used was HM-3-coated A040C01 by Asahi Kasei Corp. A 0.25 ml portion of cryopreserved blood was applied to a nonwoven fabric by the procedure of Example 5, and after washing the nonwoven fabric by pouring 3 ml of 1 M NaCl-containing PBS/1 mM EDTA and 10 ml of purified water, 1 ml of an active oxygen solution comprising 3% hydrogen peroxide water and 0.1 mM $CuCl_2$ was added and a syringe was slowly pushed to impregnate the entire nonwoven fabric with the solution. After standing at room temperature for 1, 2, 3 and 5 minutes, the syringe was pushed to force out all of the radical solution, and the nucleic acid adsorbed onto the nonwoven fabric was eluted with 1 ml of TE Buffer. The nucleic acid was promptly purified with a NucleoSpin column (Macherey-Nagel), and the hydrogen peroxide and metal ion in the effluent were removed.

The amount of purified nucleic acid was quantitated by measurement at $OD_{260}$ nm. The purified nucleic acid was subjected to 0.7% agarose electrophoresis to determine the degree of fragmentation. The results are shown in Table 14. They indicated that nucleic acid adsorbed onto the filter eluted with hydrogen peroxide treatment for a short time.

It was then confirmed that the purified genomic DNA eluted with the radical solution could be used as a PCR template. The PCR and its analysis were performed in the same manner as in Example 2. Specifically, a Glyceraldehyde 3-Phosphate Dehydrogenase (G3PDH) 0.45 kb Control Amplimer Set by Clontech was used for primers. The results are shown in Fig. 15. They indicated that the purified genomic DNA eluted with hydrogen peroxide could be used as a template for nucleic acid amplification by PCR.

[Example 12] Nucleic acid elution with reducing sugar and metal ion

A HM-3-coated A040C01 nonwoven fabric having human nucleic acid adsorbed thereon was prepared by the same method as in Example 11. It was then removed from the column holder and transferred to a 24-well plate (SUMILON), and 250 µl each of 100 mM D-ribose 5-phosphate (SIGMA) and 0.1 mM $CuCl_2$ was added as radical solutions and the fabric was allowed to stand at 50°C while stirring. A 50 µl portion of 0.5 M EDTA was added after 1, 3, 5, 10 and 30 minutes to prevent active oxygen generation. A 10 µl portion of the product was subjected to 0.7% agarose gel electrophoresis. After electrophoresis at 50 V for 45 minutes, the gel was stained with ethidium bromide and photographed with a BioImage Gel Print 2000i/VGA. The results are shown in Fig. 16. They indicated that the filter-adsorbed nucleic acid had been eluted by the active oxygen generated by the divalent metal ion-added reducing sugar.

By the same method as in Example 11, it was confirmed that the genomic DNA eluted and purified with this radical solution could be used as a PCR template. The results are shown in Fig. 17. They indicated that nucleic acid amplification by PCR, using as the template purified human genomic DNA eluted from a filter by active oxygen generated by a divalent metal ion-added reducing sugar, was possible by active oxygen reaction in a short time.

[Example 13] Nucleic acid elution by restriction enzyme

A HM-3-coated A040C01 nonwoven fabric having human nucleic acid adsorbed thereon was prepared by the same method as in Example 11. It was then removed from the column holder and transferred to a 24-well plate (SUMILON), and then 50 µl of an enzyme solution containing 2.5 µl of restriction enzyme Sau3AI (Takara) was added and the fabric was allowed to stand at room temperature or at 37°C for 5, 10 and 30 minutes. A 50 µl portion was purified with a NucleoSpin column to obtain 50 µl of an eluted genome solution. A 10 µl portion of the product was subjected to 0.7% agarose gel electrophoresis. After electrophoresis at 50 V for 45 minutes, the gel was stained with ethidium bromide and photographed with a BioImage Gel Print 2000i/VGA. The results are shown in Fig. 18. They indicated that the filter-adsorbed genomic DNA had been cleaved by the restriction enzyme and had been eluted.

[Reference Example 2] Effect of active oxygen elution on DNA probe

By the same method as in Reference Example 1, it was confirmed whether the genomic DNA fragmented and eluted from the nonwoven fabric with active oxygen could be used for hybridization. A hybridization experiment was carried out using digoxigenin-11-dUTP (DIG)-labeled DNA. DIG labeling of the DNA was accomplished using a Roche PCR DIG Probe Synthesis Kit and PCR DIG Labeling Mix, and hybridization and detection were accomplished using a Roche DIG-High Prime DNA Labeling/Detection Kit. After preparing 50 µl of a PCR reaction solution containing 1 ng Lambda DNA (Takara) and the chemically synthesized DNA primers of SEQ ID NO:3 and SEQ ID NO:4 (final concentrations: 4 µM each), the solution was reacted in a DNA Thermal Cycler (Perkin Elmer) with 1 cycle of 94°C, 3 min; 30 cycles of 94°C, 30 sec, 60°C, 1 min, 72°C, 3 min; and then 72°C for 5 minutes. The labeled DNA was purified with a NucleoSpin column by Macherey-Nagel, yielding a 50 µl DIG-labeled DNA probe solution. A 50 µl portion of an active oxygen solution comprising 100 mM D-ribose 5-phosphate and 0.05 mM $CuCl_2$ was added to the DIG-labeled DNA probe, and the mixture was allowed to stand at 50°C for 2 and 10 minutes. After re-purification with a NucleoSpin column, the active oxygen in the solution was removed to obtain the active oxygen-treated DIG-labeled DNA probe. The procedure was subsequently conducted in the same manner as in Reference Example 1. The results are shown in Fig. 19. They indicated that an active oxygen-treated DIG-labeled DNA probe was also capable of hybridizing with DNA immobilized on a membrane.

[Example 14] PCR of human genomic DNA adsorbed on nonwoven fabric

The nonwoven fabrics HM-3-coated A040C01, A040C01, P020A(EL), P090D, E05070 and N05070 having human nucleic acid adsorbed thereon were prepared by treatment with 0.25 ml of cryopreserved blood (leukocyte count: 1.15 x $10^6$) by the same method as in Example 11. After washing the nonwoven fabric disks by pouring 3 ml of 1 M NaCl-containing PBS/1 mM EDTA, 3 ml of TE Buffer and finally 3 ml of purified water, the four nonwoven fabric disks were removed from the filter holder and the center of the nonwoven fabric at the upstream end (entrance) was cut out to a 3 mm x 3 mm rectangle and placed at the bottom of a 0.5 ml PCR tube.

A glyceraldehyde 3-phosphate dehydrogenase (G3PDH) 0.45 kb Control Amplimer Set by Clontech (Cat. No. 5405-3) was used as the PCR system. The nonwoven fabric pieces were placed in a PCR tube, and in the same manner as in Example 2, 50 µl of PCR reaction solution was added and the mixture was reacted in a DNA Thermal Cycler (Perkin Elmer) with 1 cycle of 94°C, 5 min; 30 cycles of 94°C, 30 sec, 55°C, 1 min, 72°C, 1.5 min; and then 72°C for 7 minutes. After completion of the PCR reaction, 1.5 µl of 10x Loading Buffer was added to 10 µl of the reaction solution and thoroughly mixed therewith, and the total amount was subjected to 2% agarose electrophoresis. After electrophoresis at 50 V for 45 minutes, the gel was stained with ethidium bromide and photographed with a BioImage Gel Print 2000i/VGA. The results are shown in Fig. 20. A 452 bp PCR product was amplified from all of the nucleic acid-adsorbed nonwoven fabrics, indicating that the human genomic DNA adsorbed on the nonwoven fabrics was usable as a template for PCR.

[Example 15] PCR of nonwoven fabric-adsorbed *E. coli* genomic DNA

After adding 50 µl of *E. coli* DH5 glycerol stock to 3 ml of LB medium, the mixture was cultured at 37°C for 3.5 hours to obtain a culture solution with $A_{600}$ = 0.9. The *E. coli* density based on absorbance was considered to be approximately 3.6 x $10^9$ cells/ml. A 1.4 ml portion of the culture solution was taken and centrifuged at 15,000 rpm for 1 minute. The cell precipitate was suspended in 0.25 ml of LB medium, and in the same manner as in Example 3, an *E. coli* nucleic acid-adsorbed HM-3-coated A040C01 was prepared. After washing the nonwoven fabric by pouring 3 ml of TE Buffer, the four nonwoven fabric disks were removed from the filter holder and the center of the nonwoven fabric at the upstream end (entrance) was cut out to a 3 mm x 3 mm rectangle and placed at the bottom of a 0.5 ml PCR tube. The same PCR system was used as in Example 4 for a PCR reaction targeted to gene rplY coding for the *E. coli* ribosomal protein L25, and the product was analyzed by agarose electrophoresis. The results are shown in Fig. 21. A 195 bp PCR product was amplified, thus confirming that nonwoven fabric-adsorbed *E. coli* genomic DNA can be used as a template for PCR.

[Example 16] Detection of nonwoven fabric-adsorbed human genomic DNA by probe hybridization

The nonwoven fabrics HM-3-coated A040C01 and A040C01 having human nucleic acid adsorbed thereon were prepared by treatment with 0.25 ml of cryopreserved blood (leukocyte count: 1.15 x $10^6$) by the same method as in Example 11. The nonwoven fabric disks were washed by pouring 3 ml of 1 M NaCl-containing PBS/1 mM EDTA and then 3 ml of TE Buffer.

The washed nonwoven fabric disks were then removed from the filter holder and transferred to a 24-well plate (SUMILON) for suspended cell culturing, and the genomic DNA adsorbed on the nonwoven fabric disks was converted to single-strands by alkali denaturation. After immediately adding 0.5 ml of a hybridization buffer (5x Denhardt's; 2xSSPE, 0.2% SDS; 10 ng/ml Salmon Sperm DNA (Sigma)) without immobilizing the genomic DNA on the membrane surface, it was allowed to stand at 65°C for 1 hour for pre-hybridization.

A radioisotope-labeled DNA probe for the human G3PDH gene and a radioisotope-labeled DNA probe for Lambda DNA were prepared by the following method. First, a G3PDH Control Amplimer Set by Clontech was used for the following PCR reaction. A PCR reaction mixture (final concentration: 10 mM Tris-HCl, pH 8.3; 50 mM KCl; 1.5 mM $MgCl_2$; 0.2 mM dATP; 0.2 mM dGTP; 0.2 mM dCTP; 0.2 mM dTTP; 1.25 U AmpliTaq (Applied Biosystems); 0.5 µM G3PDH 3'-primer; 0.5 µM G3PDH 5'-primer) was added at a final volume of 50 µl to a PCR tube containing 10 ng of Human Genomic DNA (GibcoBRL). For the Lambda DNA probe, a PCR reaction mixture (final concentration: 10 mM Tris-HCl, pH 8.3; 50 mM KCl; 1.5 mM $MgCl_2$; 0.2 mM dATP; 0.2 mM dGTP; 0.2 mM dCTP; 0.2 mM dTTP; 1.25 U AmpliTaq (Applied Biosystems)) was added at a final volume of 50 µl to a PCR tube containing 1 ng of Lambda DNA (Takara) and the chemically synthesized DNA primer of SEQ ID NO:3 (final concentration: 0.4 µM) and DNA primer of SEQ ID NO:4 (final concentration: 4 µM), ordered from Nihon Bioservice. The PCR reaction mixture was reacted in a DNA Thermal Cycler (Perkin Elmer) with 1 cycle of 96°C, 5 min; 30 cycles of 96°C, 30 sec, 60°C, 1 min, 72°C, 3 min; and then 72°C for 5 minutes. After completion of PCR, each PCR product was purified with a NucleoSpin Extract Kit (Macherey-Nagel) to obtain partial sequence DNA fragments of the human G3PDH gene and Lambda DNA. A 45 µl portion of TE Buffers containing 20 ng of each DNA fragment was allowed to stand at 96°C for 5 minutes, and was promptly placed in ice for 5 minutes for conversion to single-stranded DNA. After adding the labeling reaction mix of a Rediprime II DNA Labeling System (Amersham Pharmacia Biotech) and 1.85 MBq of Readyview [$\alpha$-$^{32}$P]dCTP (Amersham Pharmacia Biotech) and gently mixing, the mixture was placed at 37°C for 10 minutes for labeling. It was then purified with a BioSpin Column G-30 (BIO RAD) to remove the unreacted [$\alpha$-$^{32}$P]dCTP, and allowed to stand again at 96°C for 5 minutes and placed in ice for 5 minutes for conversion to single-stranded DNA, to obtain a radioisotope-labeled human G3PDH DNA probe and Lambda DNA probe.

A portion of each radioisotope-labeled probe was taken for measurement of the radioactivity with a scintillation counter, and the human G3PDH probe and Lambda DNA probe were mixed in a proportion varied to an equivalent total radioactivity, after which they were added to the previous pre-hybridization solution and the mixture was allowed to stand at 65°C for 18 hours for hybridization. Following hybridization, the nonwoven fabric disks were transferred to a fresh 24-well plate, 0.5 ml of 2xSSC, 1% SDS was added for washing at 65°C for 10 minutes, and then 0.5 ml of 0.1xSSC, 1% SDS was added for washing at 65°C for 10 minutes, i.e., a total of two washings. After washing, the nonwoven fabric disks were removed and transferred to a vial bottle containing 5 ml of liquid scintillator, and the residual radioactivity of the nonwoven fabric disks was measured with a scintillation counter. The results are shown in Fig. 22. Genomic DNA was detected on the nonwoven fabric disks using the G3PDH probe, with the level of detected radioactivity lower with a lower proportion of human G3PDH probe, indicating detection of specific hybridization between the human G3PDH probe and the G3PDH gene among the human genomic DNA on the nonwoven fabric disks.

[Example 17] Release of nucleic acid adsorbed on nonwoven fabric

The nonwoven fabric HM-3-coated A040C01 having human nucleic acid adsorbed thereon was prepared by treatment with 0.25 ml of cryopreserved blood (leukocyte count: 1.15 x $10^6$) by the same method as in Example 11. The nonwoven fabric disks were washed by pouring 3 ml of purified water, and then the four nonwoven fabric disks were removed from the filter holder and placed in a 1.5 ml Eppendorf tube. Five sets were prepared, and each set was treated at the respective temperatures, for the respective times and with addition of the respective solutions listed in Table 5. A heat block was used for the 95°C treatment. The relative elution (%) is the amount of nucleic acid eluted under the conditions listed with respect to 100% as the amount of nucleic acid eluted with treatment in TE Buffer at 95°C for 20 minutes. The results in Table 5 indicate that the nucleic acid is not readily released in solution containing $Mg^{2+}$ or high concentration salts even when heated at 95°C, and that the nucleic acid is also not readily released at room temperature under alkaline denaturation conditions.

Table 5

| Solution | Temperature (°C) | Time (min) | Relative elution (%) |
|---|---|---|---|
| TE | 95 | 20 | 100 |
| 10 mM Tris (pH 8.0)/1 mM $MgCl_2$ | 95 | 20 | 5 |
| 10 mM Tris (pH 8.0)/10 mM $MgCl_2$ | 95 | 20 | 3 |
| 1 M NaCl/1 mM EDTA-containing PBS | 95 | 20 | 4 |
| 0.05 N NaOH | 25 | 10 | 2 |

[Example 18] Electron micrographs of nonwoven fabric-adsorbed nucleic acid

The nonwoven fabric P03050 was used to prepare a nucleic acid-adsorbed filter in the same manner as in Example 14. After pouring purified water to wash the nonwoven fabric disks, the four nonwoven fabric disks were removed from the filter holder and the nonwoven fabric disk at the upstream end (entrance) was freeze-dried using a DC-41 freeze dryer (Yamato). A nonwoven fabric set was also prepared by the same procedure except for pouring of the blood, and this was also freeze-dried as a control. The dried nonwoven fabric was cut into an approximately 5 mm square and fixed to an SEM sample stage using carbon paste. After air drying, an OPC-80 Osmium Plasma Coater (Nippon Laser & Electronics) was used for osmium plasma coating to a thickness of 20 nm to prepare a microscopy sample. The sample was observed by SEM using an S-900 field emission scanning electron microscope (Hitachi) at an acceleration voltage of 1 kV. The photograph shown in Fig. 23 clearly shows that the nucleic acid was fibrous and had adsorbed onto the surface of the nonwoven fabric fibers.

[Example 19] Investigation of surfactants

An experiment was conducted under the same conditions as in Example 5 to study the types of surfactants used for nucleic acid extraction. The nonwoven fabric used was a HM-3-coated A040C01 product by Asahi Kasei Corp. The leukocyte count in 0.25 ml of cryopreserved blood was 1.50 x $10^6$. To 0.25 ml of cryopreserved blood there was added 0.25 ml of 2x Digestion Buffer (20 mM Tris, pH 8; 200 mM NaCl; 50 mM EDTA; 1% surfactant) heated to 37°C. The surfactant was one selected from among the following surfactants. Specifically, the surfactant used was the anionic surfactant Sodium dodecyl sulfate (SDS), the amphoteric surfactant 3-[(3-Cholamidopropyl) dimethylammonio]-1-propanesulfonate (CHAPS) or 3-[(3-Cholamidopropyl) dimethylammonio]-2-hydroxy-1-propanesulfonate (CHAPSO), the non-ionic surfactants Polyethyleneglycol tert-octylphenyl ether (Triton X-114), Polyethyleneglycol tert-octylphenyl ether (Triton X-100), Polyoxyethylene alkyl ether (Nissan Dispanol TOC), (Octylphenoxy)polyethoxyethanol (Igepal CA630) or Nonoxynol-8.5 (Nissan Nonion NS-208.5), or the cationic surfactants Hexadecylpyridinium Chloride (HPC), Hexadecylpyridinium Bromide (HPB), Hexadecyltrimethylammonium Chloride (HTAC) or Hexadecyltrimethylammonium Bromide (HTAB). After adding 2x Digestion Buffer and further adding 0.05 μg of Proteinase K (PCR-Grade, Roche), the mixture was stirred in a vortex while periodically heating it in a 37°C water bath, to complete dissolution. After allowing the solution to stand at room temperature for 5 minutes, the blood extract was applied to the nonwoven fabric disks and immediately subjected to suction filtration. The filter was then washed by pouring 8 ml of Digestion Buffer containing the same surfactant.

The nonwoven fabric disks were washed by pouring 3 ml of 1M NaCl-containing PBS/1 mM EDTA and 3 ml of TE Buffer (10 mM Tris, pH 8; 1 mM EDTA). The four nonwoven fabrics were removed from the filter holder and placed in a lock-equipped Eppendorf tube, and the nonwoven fabric disks were immersed in 0.5 ml of TE Buffer and then incubated with a heat block at 95°C for 20 minutes.

The amount of DNA eluted from the nonwoven fabric disks was assayed using an OliGreen® ssDNA quantitation kit in the same manner as in Example 9. As shown in Fig. 24, the nucleic acid could be purified when using an anionic surfactant, amphoteric surfactant or non-ionic surfactant, but no nucleic acid could be recovered when using a cationic surfactant.

[Comparative Example 2] Porous filters other than nonwoven fabrics

An experiment was conducted under the same conditions as in Example 1 to examine whether porous filters other than nonwoven fabrics can be used for nucleic acid purification. As porous filters there were used a porous polyurethane sheet (IMUGUARD III-RC Main Filter, Terumo) and a polycarbonate track-etched membrane with a pore size of 8 μm and coated with a SiCN thin film as the top layer (OIA flow through membrane, US BioStar), while a HM-3-coated A040C01 nonwoven fabric by Asahi Kasei Corp. was used as a control.

The filters were cut into 12 mm-diameter disks, and one each of the porous polyurethane sheet or the IOA flow through membrane, and four of the A040C01/HM-3 nonwoven fabric disks, were set in a filter holder (SWINNEX, MILLIPORE). A 10 ml glass syringe was set upstream and a suction pump was set downstream in connection with the filter holder. The filter was initially washed with 3 ml of Digestion Buffer (10 mM Tris, pH 8; 100 mM NaCl; 25 mM EDTA; 0.5% SDS).

Next, 0.05 μg of Proteinase K (PCR-Grade, Roche) was added to 0.25 ml of human blood (leukocyte count: 1.62 x $10^6$), and after further adding 0.25 ml of 2x Digestion Buffer (20 mM Tris, pH 8; 200 mM NaCl; 50 mM EDTA; 1% SDS), the mixture was allowed to stand at room temperature for 5 minutes. The blood extract was applied to the filter and immediately subjected to suction filtration. The experiment was subsequently conducted in the same manner as Example 1, and the amount of eluted nucleic acid was measured based on absorbance. As shown in Table 6, the porous polyurethane sheet was able to purify the nucleic acid, although the nucleic acid yield and purity were inferior compared to the A040C01/HM-3 nonwoven fabric, but purification of the nucleic acid was not possible with the OIA flow through membrane. This indicated that even some porous filters with pore sizes of about 8 μm cannot be used for nucleic acid purification.

Table 6

| Porous filter | DNA (μg) | $A_{260}/A_{280}$ |
|---|---|---|
| Porous polyurethane sheet | 5.2 | 1.70 |
| OIA flow through membrane | 0.2 | 1.21 |
| A040C01/HM-3 | 8.6 | 1.95 |

[Example 20] Investigation of purified genomic DNA adsorption conditions (1) -- Effect of salts

Purified genomic DNA was used to examine the effect of the solution composition on DNA adsorption onto nonwoven fabrics. The genomic DNA used was the sample purified in Example 6. One of the nonwoven fabrics A040C01, HM-3-coated A040C01, A066A, A040B or E01030 of Asahi Kasei Corp. was cut into 12 mm-diameter disks, four of which were stacked and set in a filter holder (SWINNEX, MILLIPORE). The nonwoven fabric disks were then washed with 3 ml of ethanol and then with 3 ml of TE Buffer, and finally equilibrated by pouring 3 ml of Sample Buffer. A 10 ml glass syringe was placed upstream from the filter holder and set in a HARVARD APPARATUS Model 55-2219 syringe pump (HARVARD APPARATUS Inc.).

The Sample Buffer used to dissolve the purified genomic DNA was 10 mM Tris (pH 8)/1 mM EDTA/0-1000 mM NaCl, 10 mM Tris (pH 8)/0-100 mM $MgCl_2$, 0-100 mM $Na_2HPO_4/NaH_2PO_4$ (pH 7.4), or 10 mM $Na_2HPO_4/NaH_2PO_4$ (pH 7.4)/0-1000 mM $(NH_4)_2SO_4$. Approximately 800 ng of the purified genomic DNA was suspended in 5 ml of Sample Buffer and passed through the nonwoven fabric at a flow rate of 26.2 ml/hr. After washing with an additional 6 ml of Sample Buffer, 3 ml of TE Buffer was poured. Finally the nonwoven fabric was removed from the filter holder and placed in a lock-equipped Eppendorf tube, and 0.5 ml of TE Buffer was added. After incubating at 95°C for 30 minutes, the TE Buffer was collected.

The amount of DNA eluted from the nonwoven fabric was assayed using an OliGreen® ssDNA quantitation kit in the same manner as in Example 9. An 18-residue oligonucleotide supplied with the kit was used as the standard, and quantitation was performed with a SPECTRA MAX GEMINI XS Fluorescent Plate Reader (Molecular Devices) at Ex 485 nm, Em 535 nm. The results are shown in Figs. 25, 26, 27 and 28. It was demonstrated that salts such as NaCl, $MgCl_2$, phosphates and $(NH_4)_2SO_4$ accelerate genomic DNA adsorption.

[Example 21] Study of purified genomic DNA adsorption conditions (2) -- Effect of ethanol

The effect of ethanol on DNA adsorption onto nonwoven fabrics was examined by the same method as in Example 20. Genomic DNA was purified by the same method as in Example 6. The nonwoven fabrics used were A040C01, A066A and E01030 by Asahi Kasei Corp. The Sample Buffer used to dissolve the purified genomic DNA was 10 mM $Na_2HPO_4/NaH_2PO_4$ (pH 7.4)/0-40% ethanol. Approximately 800 ng of the purified genomic DNA was suspended in 5 ml of Sample Buffer and passed through the nonwoven fabric at a flow rate of 26.2 ml/hr. After washing with an additional 6 ml of Sample Buffer, 3 ml of TE Buffer was poured. Finally, the nonwoven fabric was removed from the filter holder and placed in a lock-equipped Eppendorf tube, and 0.5 ml of TE Buffer was added. After incubating at 95°C for 30 minutes, the TE Buffer was recovered.

The amount of DNA eluted from the nonwoven fabric was assayed using an OliGreen® ssDNA quantitation kit. As shown in Fig. 29, the DNA yield was reduced by the addition of ethanol to the Sample Buffer dissolving the purified genomic DNA.

[Example 22] DNA shaking adsorption

A PET nonwoven fabric or PET sheet membrane was added to the purified genomic DNA solution and shaken to determine whether the DNA adsorbed onto it. The genomic DNA was purified by the same method as in Example 6. The nonwoven fabrics used were A040C01, A066A or E01030 of Asahi Kasei Corp. Four disks of the PET nonwoven fabric or PET sheet membrane which had been cut into 12 mm-diameter disks were placed in a 15 ml polypropylene test tube (IWAKI). Three ml of ethanol was added to the test tube to wet the nonwoven fabric disks. After suctioning off the ethanol, 3 ml of TE Buffer was added to wash the nonwoven fabric disks, and this was also suctioned off in the same manner. Finally, the nonwoven fabric disks were treated with 3 ml of Sample Buffer in the same manner. The Sample Buffer used was 10 mM Tris (pH 8)/1 mM EDTA/50 mM NaCl, 10 mM Tris (pH 8)/2 mM $MgCl_2$, 50 mM $Na_2HPO_4$/ $NaH_2PO_4$ (pH 7.4), or 10 mM $Na_2HPO_4/NaH_2PO_4$ (pH 7.4)/0.2-1.0 M $(NH_4)_2SO_4$.

Next, 5 ml of Sample Buffer was added to the nonwoven fabric disk-containing test tube, and approximately 800 ng of the purified genomic DNA was added and thoroughly stirred therewith. The test tube was mounted in a MIX-ROTAR VMR-5 (IUCHI) for treatment at 80 rpm for 30 minutes. After suctioning off the DNA solution in the test tube, 6 ml of Sample Buffer was added and the same treatment was repeated at 80 rpm for 15 minutes. After removing off the Sample Buffer, 3 ml of TE Buffer was added and the same treatment was again repeated at 80 rpm for 15 minutes. The nonwoven fabric disks were removed out of the test tube and placed in a lock-equipped Eppendorf tube, 0.5 ml of TE Buffer was added and incubation was performed at 95°C for 30 minutes. The amount of DNA eluted from the nonwoven fabric disks was assayed using an OliGreen® ssDNA quantitation kit. As shown in Fig. 30, the genomic DNA had adsorbed onto the PET nonwoven fabric and was recovered, but it did not adsorb onto the PET sheet membrane. This indicated that the form of the nonwoven fabric is important for DNA adsorption.

Fig. 31 shows the effect of adding ammonium sulfate with respect to 10 mM phosphoric acid. Ammonium sulfate resulted in particularly notable acceleration of DNA adsorption with the nonwoven fabric E01030. With shaking adsorption, in contrast to the filtering adsorption of Example 20, improved DNA adsorption was exhibited by E01030 rather than A040C01, with approximately 80% of the DNA being recovered. This indicated that the optimum type of nonwoven fabric for nucleic acid purification differs depending on the adsorption conditions.

[Example 23] Screening of nonwoven fabrics

An experiment was conducted in the same manner as in Example 1 to screen for nonwoven fabrics that can be used for DNA purification. Blood was taken from 5 healthy donors, with leukocyte counts of 0.94-1.91 x $10^6$ in 0.25 ml of blood. The list of nonwoven fabrics used is shown in Table 7. All were products of Asahi Kasei Corp. The mean pore size was measured with a mercury porosimeter and the mean fiber size was calculated from an SEM photograph.

After adding 0.05 µg of Proteinase K to 0.25 ml of human blood and further adding 0.25 ml of 2x Digestion Buffer, the mixture was allowed to stand at room temperature for 5 minutes. This was followed by the same treatment as in Example 1, and finally the nonwoven fabric disks were washed by pouring 3 ml of TE Buffer. The four nonwoven fabric disks were removed from the filter holder and placed in a lock-equipped Eppendorf tube, and 0.5 ml of TE Buffer was added. After incubating at 80°C for 1 hour, the TE Buffer was recovered.

Table 7

| Brand name | Product No. | Material | Thickness (mm) | Mean pore size (µm) | Mean fiber size (µm) |
|---|---|---|---|---|---|
| | A040C01/HM-3 | PET/HM-3 Coat | 0.235 | | |
| | A040C01 | PET | 0.235 | 10 | 1.2 |
| | A066A | PET | 0.400 | 13 | 1.7 |
| Bemliese | PS140 | cellulose | 0.080 | | |
| Bemliese | TS327 | cellulose | 0.270 | | |
| Bemliese | TS507 | cellulose | 0.320 | | |
| Bemliese | TS100 | cellulose | 0.400 | 45 | 12 |
| HYBRID Bemliese | QT409 | cellulose | 0.290 | | |
| HYBRID Bemliese | RK629 | cellulose | 0.420 | | |
| MICROWEB | A040B | polyester | 0.130 | 10 | |
| MICROWEB | A040C | polyester | 0.100 | | |
| MICROWEB | A045A | polyester | 0.130 | | |
| MICROWEB | A080A | polyester | 0.180 | | |
| MICROWEB | A090D | polyester | 0.370 | | |
| MICROWEB | P020A(EL) | polypropylene | 0.140 | | |
| MICROWEB | P020B(EL) | polypropylene | 0.200 | | |
| MICROWEB | P020C | polypropylene | 0.170 | | |
| MICROWEB | P050D(EL) | polypropylene | 0.470 | | |
| MICROWEB | P090C | polypropylene | 0.660 | | |
| MICROWEB | P090D | polypropylene | 0.740 | | |
| ELTAS | N05020 | nylon | 0.130 | | |
| ELTAS | N05030 | nylon | 0.170 | | |
| ELTAS | N05040 | nylon | 0.190 | | |

Table 7 (continued)

| Brand name | Product No. | Material | Thickness (mm) | Mean pore size (μm) | Mean fiber size (μm) |
|---|---|---|---|---|---|
| ELTAS | N05050 | nylon | 0.210 | | |
| ELTAS | N05070 | nylon | 0.250 | | |
| ELTAS | E01012 | polyester | 0.090 | 66 | 14 |
| ELTAS | E01015 | polyester | 0.110 | | |
| ELTAS | E01020 | polyester | 0.130 | | |
| ELTAS | E01025 | polyester | 0.170 | | |
| ELTAS | E01030 | polyester | 0.200 | 130 | 12 |
| ELTAS | E01040 | polyester | 0.250 | | |
| ELTAS | E01050 | polyester | 0.290 | | |
| ELTAS | E01070 | polyester | 0.360 | | |
| ELTAS | E05070 | polyester | 0.230 | | |
| ELTAS | P03015 | polypropylene | 0.140 | 67 | |
| ELTAS | P03020 | polypropylene | 0.190 | | |
| ELTAS | P03025 | polypropylene | 0.210 | | |
| ELTAS | P03040 | polypropylene | 0.340 | | |
| ELTAS | P03050 | polypropylene | 0.400 | 75 | 20 |
| ELTAS | P03070 | polypropylene | 0.500 | | |
| SHALERIA | C1050 | 90% acryl; 10% polyester | 0.350 | | |
| SHALERIA | C3040 | 70% acryl; 30% polyester | 0.260 | | |
| SHALERIA | CR050 | 65% acryl; 35% rayon | 0.320 | | |
| SHALERIA | RC040S | 35% acryl; 65% rayon | 0.270 | | |
| SMASH | Y15050 | polyester | 0.160 | 58 | |
| SMASH | Y15100 | polyester | 0.250 | | |
| SMASH | Y15150 | polyester | 0.340 | | |
| SMASH | Y15200 | polyester | 0.440 | | |
| SMASH | Y15250 | polyester | 0.530 | | |

After diluting the amount of nucleic acid contained in each recovered solution 10-fold with TE Buffer, it was assayed using an OliGreen® ssDNA quantitation kit. The results are shown in Fig. 32. The nucleic acid purity was determined by measuring the $A_{260}$ (absorbance at 260 nm) and $A_{280}$ (absorbance at 280 nm) of the effluent with a UV-1600 UV-visible light spectrophotometer (Shimadzu), and calculating $A_{260}/A_{280}$. The results are shown in Fig. 33. Although the nucleic acid yields and purities differed, nucleic acid purification was successfully accomplished with these nonwoven fabrics having different specifications and materials.

[Example 24] RNA in nucleic acid purified with nonwoven fabric

The presence of RNA in the *E. coli* nucleic acid prepared in Example 3 was confirmed with an OliGreen® ssDNA quantitation kit. The OliGreen assay system detects RNA as well as DNA. Thus, by comparing the assay value after RNase treatment of the nucleic acid solution for selective degradation of the RNA with the assay value without RNase treatment, it is possible to estimate the amount of RNA present in the nucleic acid solution. This was confirmed by a control experiment using rRNA.

A 150 µl portion of effluent from the nonwoven fabric was taken, and bovine pancreas DNase-free RNase (Product No. 1,119,915, Roche) was added thereto at 0.375 µg/0.75 µl (final concentration: 2.5 µg/ml). As a control, the same treatment was carried out with addition of TE Buffer instead of the RNase solution. After incubation at 37°C for 30 minutes, the mixture was cooled on ice and diluted 40-fold with TE Buffer, and 100 µl thereof was assayed with OliGreen. The amount of RNase-untreated nucleic acid was 75.3 µg, but with RNase treatment the amount was reduced to 10.0 µg. This indicated that the nucleic acid solution contained approximately 10 µg of DNA and approximately 65 µg of RNA, and that both DNA and RNA could be purified by the nonwoven fabric.

[Example 25] Heat treatment of specimens

Sputum was sampled from volunteers, and after mixing samples from 6 persons, the mixture was dispensed into Eppendorf tubes at 0.2 ml each and cryopreserved at -20°C. Cells were transferred from *E. coli* DH5 (Toyobo) glycerol stock into LB medium (1 g Tryptone Peptone (Difco), 0.5 g yeast extract (Difco), 1 g NaCl; 200 µl 1 N NaOH; 100 ml distilled water) with a plastic disposable platinum loop. The liquid was cultured at 37°C for 16 hours to obtain a culture solution with $A_{600}$ = 3.5. The *E. coli* density of the culture solution was estimated to be about 1.4 x $10^{10}$ cells/ml based on absorbance. The culture solution was used to prepare 2 x $10^6$ cell/ml, 2 x $10^5$ cell/ml and 2 x $10^4$ cell/ml cell suspensions. The previously dispensed sputum samples were dissolved and 50 µl of each suspension was added to the containers to prepare *E. coli*-added sputum samples.

The nonwoven fabric used was A040C01 of Asahi Kasei Corp. The nonwoven fabric was cut into 12 mm-diameter disks, four of which were stacked and set in a filter holder (SWINNEX, MILLIPORE), with a 10 ml glass syringe set upstream and a suction pump set downstream in connection with the filter holder. The nonwoven fabric disks were initially washed with 3 ml of Digestion Buffer (10 mM Tris, pH 8; 100 mM NaCl; 25 mM EDTA; 0.5% SDS).

After adding 0.25 ml of 2x Digestion Buffer (20 mM Tris, pH 8; 200 mM NaCl; 50 mM EDTA; 1% SDS) to each *E. coli*-added sputum sample, it was treated at 98°C for 1 minute. Upon cooling, 0.05 mg of Proteinase K (PCR-Grade, Roche) was added and the mixture was treated for 5 minutes in a 37°C water bath with periodic stirring. The *E. coli*-added sputum sample extract was applied to the nonwoven fabric disks and subjected to suction filtration. The filter was then washed by pouring 8 ml of Digestion Buffer under suction. Finally, the nonwoven fabric disks were washed by pouring 3 ml of 1 M NaCl-containing PBS, 1 mM EDTA and 3 ml of TE Buffer (10 mM Tris, pH 8; 1 mM EDTA).

The four nonwoven fabric disks were removed from the filter holder and the nonwoven fabric disk at the upstream end (entrance) was removed and placed in an Eppendorf tube. After adding 0.2 ml of TE Buffer (10 mM Tris, pH 8; 1 mM EDTA), treatment was carried out at 95°C for 20 minutes for elution. The effluent was used for a PCR reaction and the *E. coli* and human DNA was detected.

Detection of the *E. coli* DNA in the effluent was performed in the following manner. As PCR primers, the following chemically synthesized sequences were ordered from Invitrogen: the nucleic acid sequence from g at position 1283 to a at position 1302 ( SEQ ID NO:5) and the sequence complementary to the nucleic acid sequence from t at position 2229 to g at position 2248 ( SEQ ID NO:6), of the gene coding for the *E. coli* ProteinPII protein. A 10 µl portion of the effluent was added to the PCR reaction solution for a total of 25 µl (final concentration: 10 mM Tris/HCl, pH 8.3; 50 mM KCl; 1.5 mM $MgCl_2$; 0.2 mM dATP, 0.2 mM dGTP; 0.2 mM dCTP; 0.2 mM dTTP; 1.25 U Taq (Sigma); 0.5 µM of each primer). The mixture was reacted in a DNA Thermal Cycler (Perkin Elmer) with 1 cycle of 94°C, 2 min; 40 cycles of 94°C, 30 sec, 55°C, 30 sec, 72°C, 1 min; and then 72°C for 5 minutes.

Detection of the human DNA in the effluent was performed in the following manner. As PCR primers, the following chemically synthesized sequences were ordered from Invitrogen: the nucleic acid sequence from c at position 483 to c at position 502 ( SEQ ID NO:7) and the sequence complementary to the nucleic acid sequence from c at position 1039 to c at position 1058 ( SEQ ID NO:8), of the gene coding for the human HGFR (Hepatocyte Growth Factor Receptor) protein. A 10 µl portion of the effluent was added to the PCR reaction mixture for a total of 25 µl (final concentration: 10 mM Tris/HCl, pH 8.3; 50 mM KCl; 1.5 mM $MgCl_2$; 0.2 mM dATP, 0.2 mM dGTP; 0.2 mM dCTP; 0.2 mM dTTP; 1.25 U Taq (Sigma); 0.5 µM of each primer). The mixture was reacted in a DNA Thermal Cycler (Perkin Elmer) with 1 cycle of 94°C, 2 min; 40 cycles of 94°C, 30 sec, 60°C, 30 sec, 72°C, 1 min; and then 72°C for 5 minutes. After completion of each PCR, 1.5 µl of 10x Loading Buffer was added to 10 µl of each reaction mixture and mixed thoroughly, and the total amount was subjected to 1.5% agarose (GibcoBRL) electrophoresis. After electrophoresis in a Mupid Minigel Migration Tank (Advance) at 100 V for 30 minutes, the gel was stained with ethidium bromide and photographed with a BioImage Gel Print 2000i/VGA. The results are shown in Figs. 34 and 35. As can be seen from Fig. 34, when the *E. coli*-added sputum sample contained *E. coli* cells in an amount of $10^3$ or greater, a 966 bp *E. coli*-derived PCR product was amplified. As shown in Fig. 35, a 577 bp human-derived PCR product was detected regardless of the amount of *E. coli* added. It was thus confirmed that the cellular DNA in the samples was extracted and purified by heat treatment of the samples followed by nucleic acid purification with a nonwoven fabric, and that it was detectable, as illustrated in this example.

[Example 26] Reduction treatment of specimens

After adding 0.25 ml of 2x Digestion Buffer (20 mM Tris, pH 8; 200 mM NaCl; 50 mM EDTA; 1% SDS) to the *E. coli*-added sputum samples prepared according to Example 25, 5 μl of a 10% dithiothreitol solution was added and the mixture was treated at room temperature for 2 minutes while stirring. Next, 0.05 mg of Proteinase K (PCR-Grade, Roche) was added and the mixture was treated for 5 minutes in a 37°C water bath with periodic stirring. The *E. coli*-added sputum sample extract was applied to an A040C01 nonwoven fabric and subjected to suction filtration. The filter was then washed by pouring 8 ml of Digestion Buffer under suction. Finally, the nonwoven fabric disks were washed by pouring 3 ml of 1 M NaCl-containing PBS/1 mM EDTA and 3 ml of TE Buffer (10 mM Tris, pH 8; 1 mM EDTA) .

The four nonwoven fabric disks were removed from the filter holder and the nonwoven fabric disk at the upstream end (entrance) was removed and placed in an Eppendorf tube. After adding 0.2 ml of TE Buffer (10 mM Tris, pH 8; 1 mM EDTA), treatment was carried out at 95°C for 20 minutes for elution. The effluent was used for a PCR reaction in the same manner as in Example 25 and the *E. coli* and human DNA was detected. The results are shown in Figs. 36 and 37. As can be seen from Fig. 36, when the *E. coli*-added sputum sample contained *E. coli* cells in an amount of $10^3$ or greater, a 966 bp *E. coli*-derived PCR product was amplified. As shown in Fig. 37, a 577 bp human-derived PCR product was detected regardless of the amount of *E. coli* added. It was thus confirmed that the cellular DNA in the samples was extracted and purified by reduction treatment of the samples followed by nucleic acid purification with a nonwoven fabric, and that it was detectable as illustrated in this example.

[Comparative Example 3] Purification of nucleic acid from *E. coli*-added sputum samples without heat treatment or reduction treatment

*E. coli*-added sputum samples and nucleic acid-adsorbed filters were prepared in the same manner as in Example 25. After adding 0.25 ml of 2x Digestion Buffer (20 mM Tris, pH 8; 200 mM NaCl; 50 mM EDTA; 1% SDS) to the *E. coli*-added sputum samples, 0.05 mg of Proteinase K (PCR-Grade, Roche) was added without the heat treatment conducted in Example 25 or the reduction treatment conducted in Example 26, and the mixture was treated for 5 minutes in a 37°C water bath with periodic stirring. In this example, unlike in Examples 25 and 26, the sputum failed to uniformly dissolve to clarity, but remained as non-uniform lumps creating a turbid state.

Next, the *E. coli*-added sputum extract was applied to a nonwoven fabric for suction filtration. The lumps of the sputum components which remained undissolved attached onto the surface of the nonwoven fabric, impeding the suction filtration. With continued suction, the liquid portion was suction filtered out but the sputum component lumps attached to the nonwoven fabric surface could not be completely filtered off, making it difficult to continue the procedure.

[Example 27] Nucleic acid extension reaction on nonwoven fabric

Genomic DNA adsorbed on a nonwoven fabric was used as a template to determine whether nucleic acid extension reaction occurs on nonwoven fabrics. DIG labeling of the DNA was accomplished using a PCR DIG Probe Synthesis Kit (Roche) and PCR DIG Labeling Mix (Roche), and hybridization and detection were accomplished using a DIG-High Prime DNA Labeling/Detection Kit (Roche).

The nonwoven fabric HM-3-coated A040C01 having human nucleic acid adsorbed thereon was prepared by treatment with 0.25 ml of cryopreserved blood (leukocyte count: $1.15 \times 10^6$) by the same method as in Example 11. After washing the nonwoven fabric disks by pouring 3 ml of TE Buffer, the nonwoven fabric disks were placed in a 24-well plate containing 0.2 ml of DIG Easy Hyb Buffer (Roche) and allowed to stand at 42°C for 2 hours. DNA primer bACT1 listed as SEQ ID NO:9 and DNA primer bACT2 listed as SEQ ID NO:10 were then added, which were chemically synthesized sequences ordered from Nihon Bioservice, and the mixture was allowed to stand at 42°C for 18 hours. After removing the excess primers by washing with a suitable washing solution, 200 μl of an enzyme reaction solution (PCR buffer containing 1.5 mM $MgCl_2$ (final concentration); 0.2 mM dATP; 0.2 mM dGTP; 0.2 mM dCTP; 0.19 mM dTTP; 0.01 mM digoxigenin-11-dUTP; 5 U Klenow Large Fragment (BioLabs)) was added and the mixture was allowed to stand at 37°C for 18 hours. It was then washed with a suitable washing solution and reacted with alkali phosphatase-labeled anti-DIG antibody, and the DIG incorporated during the nucleic acid extension reaction was detected with an NBT/BCIP substrate. The results are shown in Fig. 38. The detectable signal increased with the amount of primer added, indicating that nucleic acid extension reaction occurred on the nonwoven fabric disks in a primer-dependent manner.

[Example 28] Amplification and detection of nucleic acid on nonwoven fabric by LAMP method

Genomic DNA adsorbed on a nonwoven fabric was used as a template to determine whether nucleic acid amplification was possible by the LAMP method. The LAMP method was performed using a Loopamp Bovine Embryo Sexing Kit (Eiken Chemical Co., Ltd.). First, bovine genomic DNA was adsorbed onto an A040C01 of Asahi Kasei Corp. as a nonwoven fabric, by the following method. After adding 0.25 ml of 2x Digestion Buffer (20 mM Tris, pH 8; 200 mM NaCl; 50 mM EDTA; 1% SDS) heated to 37°C to 0.25 ml of bovine cryopreserved blood (Nihon Biotest Laboratory Co., Ltd.), 0.05 μg of Proteinase K (PCR-Grade, Roche) was further added and the mixture was stirred in a vortex while periodically heating it in a 37°C water bath, to complete dissolution. After allowing the solution to stand at room temperature for 5 minutes, the blood extract was applied to the nonwoven fabric and immediately subjected to suction filtration. The filter was then washed by pouring 8 ml of Digestion Buffer under suction. Next, the nonwoven fabric was further washed by pouring 3 ml of 1 M NaCl-containing PBS/1 mM EDTA and 3 ml of TE Buffer (10 mM Tris, pH 8; 1 mM EDTA). The nonwoven fabric was cut into a 3 mm square and placed in a 0.5 ml microtube. There were then added 40 μl of Loopamp Reaction Mix II and 1 μl of Bst DNA polymerase, prior to incubation at 63°C. After one hour, the turbidity of the amplification solution was examined, and a portion thereof was subjected to agarose electrophoresis. The results indicated that nucleic acid amplification by LAMP occurred with the bovine genomic DNA adsorbed onto the nonwoven fabric as a template (Fig. 39).

[Example 29] Hybridization of eluted genomic DNA

It was investigated whether genomic DNA obtained by the elution methods described in Examples 9 and 11 can be used for hybridization. First, to 1 μg of human genomic DNA (Clontech) there were added 1 μl of Biotin Chem-Link (Roche) and water to a total volume of 20 μl, and after incubation at 85°C for one hour, 5 μl of reaction terminating solution was added to obtain a biotin-labeled genomic DNA solution. Next, the genomic DNA obtained by each elution method was dotted on a Hybond N+ nylon filter (Amersham-Pharmacia), and then denatured with an alkali solution and immobilized by UV crosslinking. An Easy Hyb hybridization solution (Roche) was added for pre-hybridization at 42°C for one hour, after which the previously prepared biotin-labeled genomic DNA was added and hybridization was performed at 42°C for 18 hours. After washing and reacting with alkali phosphatase-labeled avidin, the alkali phosphatase substrate CSPD (Roche) was added for reaction, and X-ray film exposure was conducted for an appropriate time period to detect the signal. As shown in Figs. 40 and 41, all of the genomic DNA obtained by elution with TE Buffer, alkali and hydrogen peroxide was usable for hybridization.

[Example 30] Hybridization using eluted genomic DNA as probe

It was investigated whether genomic DNA obtained by the elution methods described in Examples 9 and 11 can be used as hybridization probes. First, to 1 μg of each nonwoven fabric-eluted human genomic DNA there were added 1 μl of Biotin Chem-Link (Roche) and water to a total volume of 20 μl, and after incubation at 85°C for one hour, 5 μl of reaction terminating solution was added to obtain a biotin-labeled eluted genomic DNA solution. Next, human genomic DNA (Clontech) and λDNA (Takara) were dotted on a Hybond N+ nylon filter (Amersham-Pharmacia), and then denatured with an alkali solution and immobilized by UV crosslinking. An Easy Hyb hybridization solution (Roche) was added for pre-hybridization at 42°C for one hour, after which each of the previously prepared biotin-labeled genomic DNA samples was added and hybridization was performed at 42°C for 18 hours. After washing and reacting with alkali phosphatase-labeled avidin, the alkali phosphatase substrate CSPD (Roche) was added for reaction, and X-ray film exposure was conducted for an appropriate time period to detect the signal. The results are shown in Fig. 42. All of the genomic DNA obtained by elution with TE Buffer, alkali and hydrogen peroxide was usable as hybridization probes.

[Example 31] Nucleic acid elution with surfactants

The nonwoven fabric A040C01 having human nucleic acid adsorbed thereon was prepared by treatment with 0.25 ml of cryopreserved blood (leukocyte count: $1.16 \times 10^6$) by the same method as in Example 11. After washing nonwoven fabrics by pouring 5 ml of TE Buffer, each of the four nonwoven fabric disks were removed from the filter holder and placed in a 1.5 ml Eppendorf tube. The amphoteric surfactants CHAPS and CHAPSO and the non-ionic surfactants Triton X-114, Triton X-100, Nissan Dispanol TOC, Igepal CA630 and Nissan Nonion NS-208.5 from the surfactants used in Example 19 were selected, and 0.5 ml of their 0.5% aqueous solutions were added. Another system was prepared with addition of 0.5 ml of TE Buffer. The mixtures were heated at 80°C for 20 minutes and the nucleic acid adsorbed onto each nonwoven fabric was eluted. The amount of DNA eluted from the nonwoven fabric was assayed using an OliGreen® ssDNA quantitation kit and with 10-fold dilution of the effluent with TE Buffer, in the same manner as in Example 9. The results are shown in Fig. 43. The amount of nucleic acid eluted upon heating in TE Buffer at 95°C for 20 minutes was defined as 100%. All of the surfactants accelerated nucleic acid elution in comparison to using TE Buffer.

Fig. 44 shows the results of electrophoresis of the eluted nucleic acid. The eluted nucleic acid was concentrated with a NucleoSpin column, and 10 μl thereof was subjected to 0.7% agarose gel electrophoresis. The nucleic acid which eluted in the presence of the surfactants had the same molecular weight as the nucleic acid which eluted with TE Buffer. It was then confirmed whether the purified DNA which had eluted in the presence of the surfactants could be used as PCR templates. PCR analysis was conducted in the same manner as in Example 2, but using a Glyceraldehyde 3-Phosphate Dehydrogenase (G3PDH) Control Amplimer Set by Clontech (Cat. No. 5406-3) for primers. The results are shown in Fig. 45. A 983 bp PCR product was also amplified from the purified DNA which had been eluted with the surfactants, indicating that it was usable as a PCR template.

[Reference Example 3] Effect of surfactants on PCR

The effects of anionic surfactants, amphoteric surfactants and non-ionic surfactants on PCR were examined.

As PCR primers there were added the synthetic DNA of SEQ ID NO:11 as the 5' primer and the synthetic DNA of SEQ ID NO:12 as the 3' primer, at respective final concentrations of 0.4 μM, to a PCR reaction solution (final concentration: 10 mM Tris-HCl, pH 8.3; 50 mM KCl; 1.5 mM $MgCl_2$; 0.2 mM dATP; 0.2 mM dGTP; 0.2 mM dCTP; 0.2 mM dTTP; 0.5 U AmpliTaq (Applied Biosystems)) containing 5 ng of human genomic DNA (Clontech).

The anionic surfactant SDS, the amphoteric surfactants CHAPS and CHAPSO and the non-ionic surfactants Triton X-114, Triton X-100, Nissan Dispanol TOC, Igepal CA630 and Nissan Nonion NS-208.5 used in Example 19 were each added thereto to final concentrations of 1%, 0.5% and 0.1%, and the PCR reaction solutions were adjusted to 20 μl.

Each mixture was reacted in a DNA Thermal Cycler (Perkin Elmer) with 1 cycle of 94°C, 3 min; 30 cycles of 94°C, 30 sec, 60°C, 1 min, 72°C, 3 min; and then 72°C for 7 minutes. After completion of the PCR reaction, 1 μl of 10x Loading Buffer was added to 5 μl of the reaction solution and thoroughly mixed therewith, and the total amount was subjected to 1.5% agarose electrophoresis. After electrophoresis at 50 V for 45 minutes, the gel was stained with ethidium bromide and photographed with a BioImage Gel Print 2000i/VGA. The results are shown in Fig. 46.

No effect on PCR was exhibited by the surfactants at the concentrations used for elution, except for SDS. SDS had an effect on PCR even at a 0.1% concentration.

[Example 32] Biotinylation of nonwoven fabric-adsorbed nucleic acid

It was investigated whether nonwoven fabric-adsorbed nucleic acid can be directly labeled. The nonwoven fabric A040C01 having human nucleic acid adsorbed thereon was prepared by treatment with 0.25 ml of cryopreserved blood (leukocyte count: $1.16 \times 10^6$) by the same method as in Example 11. It was cut into 4 equal sections which were placed in a 0.5 ml microtube, and then 95 μl of water and 5 μl of Biotin Chem-Link (Roche) were added, and biotinylation was performed at 85°C for one hour. The supernatant was transferred to a separate tube, and after standing at 95°C for 5 minutes for single-strand conversion of the nucleic acid, it was cooled on ice to prepare Probe 1. The nonwoven fabric which had been reacted with Biotin Chem-Link was transferred to a tube containing 100 μl of TE Buffer and placed at 95°C for 20 minutes, for simultaneous elution of the nucleic acid from the nonwoven fabric and single-strand conversion of the nucleic acid. This was then cooled on ice to prepare Probe 2. From 100 ng to 0.1 ng of human genomic DNA (Clontech) and Lambda DNA (Takara) were dotted on a Hybond N+ membrane (Amersham-Pharmacia) and subjected to alkali denaturation and immobilization by UV crosslinking. This was transferred to a suitable sealed container, and Easy Hyb (Roche) was added. After standing at 42°C for one hour, 20 μl of Probe 1 or Probe 2 was added to 1 ml of Easy Hyb, and the probe was subsequently hybridized at 42°C for 18 hours. 2xSCC and 0.1% SDS were then used for washing twice at room temperature for 5 minutes, after which 0.1xSCC and 0.1% SDS were used for washing twice at 68°C for 15 minutes. Subsequent equilibration for 1 minute using a washing buffer (final concentration: 0.1 M maleic acid; 0.15 M NaCl; 0.3% Tween20, pH 7.5) was followed by immersion for one hour in a blocking solution (Roche) diluted 10-fold with maleic acid buffer (final concentration: 0.1 M maleic acid; 0.15 M NaCl, pH 7.5). After then adding 1 μl of alkali phosphatase-conjugated avidin (CAL BIOCHEM) to 5 ml of the liquid, the mixture was allowed to stand at room temperature for 30 minutes for gradual permeation. Washing was performed twice with a washing buffer at room temperature for 15 minutes, and followed by equilibration for 2 minutes with alkali phosphatase buffer (final concentration: 0.1 M Tris, pH 9.5; 0.1 M NaCl; 50 mM $MgCl_2$), after which a 2/100 volume of NBT/BCIP (Roche) was added for color development reaction. The results are shown in Fig. 47. Both Probes 1 and 2 hybridized to the human genomic DNA dotted on the Hybond N+ membrane, indicating that the nucleic acid trapped on the nonwoven fabric had been biotin-labeled.

Industrial Applicability

According to the method of the present invention it is possible to easily purify nucleic acids from samples containing cells such as leukocytes or bacteria. The invention may be applied for rapid elution of nucleic acids from nucleic acid-adsorbing filters. The method of the invention also allows rapid purification of nucleic acids from samples containing cells such as leukocytes or bacteria using nonwoven fabrics, and direct amplification or nucleic acid sequence detection of the nucleic acids on the nonwoven fabrics. It is thereby possible to simplify the steps from sample treatment to nucleic acid amplification and nucleic acid sequence detection, allowing treatment to be accomplished in a shorter time.

Sequence List (without sequence data)

SEQ ID NO:1: Synthetic DNA
SEQ ID NO:2: Synthetic DNA
SEQ ID NO:3: Synthetic DNA
SEQ ID NO:4: Synthetic DNA
SEQ ID NO:5: Synthetic DNA
SEQ ID NO:6: Synthetic DNA
SEQ ID NO:7: Synthetic DNA
SEQ ID NO:8: Synthetic DNA
SEQ ID NO:9: Synthetic DNA
SEQ ID NO:10: Synthetic DNA
SEQ ID NO:11: Synthetic DNA
SEQ ID NO:12: Synthetic DNA

## Claims

1. A method of preparing cellular nucleic acid from a cell-containing sample, which method comprises:

   (a) a step of disrupting cells to prepare a cell extract;
   (b) a step of contacting the cell extract with a nonwoven fabric to adsorb the nucleic acid in the cell extract onto the nonwoven fabric; and
   (c) a step of eluting the nucleic acid from the nonwoven fabric.

2. The method of claim 1, wherein the nucleic acid adsorbed onto the nonwoven fabric is heated in a temperature range of 40°C to 100°C to elute the nucleic acid.

3. The method of claim 1 or 2, wherein the nucleic acid adsorbed onto the nonwoven fabric is treated under alkaline conditions of pH 12 or higher to elute the nucleic acid.

4. The method of claim 1 or 2, wherein the nucleic acid adsorbed onto the nonwoven fabric is fragmented for elution.

5. The method of claim 4, wherein active oxygen is used to fragment the nucleic acid.

6. The method of claim 5, wherein the active oxygen used is generated by adding a divalent metal ion to a reducing sugar.

7. The method of claim 5, wherein the active oxygen is hydrogen peroxide.

8. The method of claim 5, wherein the active oxygen used is generated by adding a divalent metal ion to hydrogen peroxide.

9. The method of claim 4, wherein an enzyme is used to fragment the nucleic acid.

10. The method of claim 1 or 2, wherein the nucleic acid adsorbed onto the nonwoven fabric is treated with a surfactant for elution.

11. The method of claim 10, wherein the surfactant is a non-ionic surfactant or amphoteric surfactant.

12. A method of preparing and amplifying cellular nucleic acid from a cell-containing sample, which method comprises:

    (a) a step of disrupting cells to prepare a cell extract;
    (b) a step of contacting the cell extract with a nonwoven fabric to adsorb the nucleic acid in the cell extract onto said nonwoven fabric;
    (c) a step of adding a nucleic acid amplification solution to said nonwoven fabric and using the nucleic acid adsorbed onto said nonwoven fabric as the template for amplification of the nucleic acid; and
    (d) a step of recovering said reaction solution.

13. The method of claim 12, wherein the nucleic acid is amplified by a PCR or LAMP method.

14. A method of preparing cellular nucleic acid from a cell-containing sample and detecting a specific nucleic acid sequence, which method comprises:

(a) a step of disrupting cells to prepare a cell extract;
(b) a step of contacting the cell extract with a nonwoven fabric to adsorb the nucleic acid in the cell extract onto said nonwoven fabric;
(c) a step of adding a solution for the detection of a nucleic acid sequence to nonwoven fabric for reaction; and
(d) a step of measuring the reaction solution.

15. A method of preparing cellular nucleic acid from a cell-containing sample and detecting a specific nucleic acid sequence, which method comprises:

(a) a step of disrupting cells to prepare a cell extract;
(b) a step of contacting the cell extract with a nonwoven fabric to adsorb the nucleic acid in the cell extract onto said nonwoven fabric;
(c) a step of adding a nucleic acid sequence detection solution to said nonwoven fabric for reaction; and
(d) a step of measuring the surface of said nonwoven fabric.

16. The method of claim 14 or 15, wherein the nucleic acid sequence is detected by a PCR or LAMP method.

17. The method of claim 15, wherein the specific nucleic acid sequence is detected by hybridization using a probe.

18. The method of claim 14 or 15, wherein the specific nucleic acid sequence is detected by nucleic acid extension reaction using primers and a polymerase.

19. The method of claim 18, wherein the detection is accomplished by incorporating a labeled nucleotide during the nucleic acid extension reaction.

20. The method of claim 18, wherein pyrophosphoric acid produced by the nucleic acid extension reaction is detected.

21. A method of preparing a cellular nucleic acid-adsorbed nonwoven fabric, which method comprises:

(a) a step of disrupting cells to prepare a cell extract; and
(b) a step of contacting the cell extract with a nonwoven fabric to adsorb the nucleic acid in the cell extract onto the nonwoven fabric.

22. A method of preparing cellular nucleic acid from a cell-containing sample and labeling the nucleic acid, which method comprises:

(a) a step of disrupting cells to prepare a cell extract;
(b) a step of contacting the cell extract with a nonwoven fabric to adsorb the nucleic acid in the cell extract onto the nonwoven fabric; and
(c) a step of adding a nucleic acid-labeling solution to said nonwoven fabric for reaction.

23. The method of claim 22, wherein the nucleic acid is labeled with biotin, fluorescence or a hapten.

24. The method of claim 22 or 23, wherein the nucleic acid adsorbed onto the nonwoven fabric is itself labeled.

25. The method of any one of claims 1 to 24, wherein the nonwoven fabric material is polyester, polypropylene or nylon.

26. The method of any one of claims 1 to 24, wherein the nonwoven fabric material is polyethylene terephthalate.

27. The method of any one of claims 1 to 26, wherein the nonwoven fabric pore size is 2-150 μm.

28. The method of any one of claims 1 to 27, wherein the nonwoven fabric fiber size is 0.3-20 μm.

29. The method of any one of claims 1 to 28, wherein a cell extract not containing a viscosity enhancer, chaotropic agent or alcohol is used.

**30.** The method of any one of claims 1 to 29, wherein a salt-containing cell extract is used.

**31.** The method of claim 30, wherein the salt in the cell extract is a sodium salt, potassium salt, magnesium salt, calcium salt, ammonium salt, phosphate, sulfate or hydrochloride.

**32.** The method of claim 30, wherein the salt in the cell extract is sodium chloride, and its concentration is from 10 mM to 1000 mM.

**33.** The method of claim 30, wherein the salt in the cell extract is magnesium chloride, and its concentration is from 1 mM to 100 mM.

**34.** The method of claim 30, wherein the salt in the cell extract is sodium phosphate, and its concentration is from 2 mM to 100 mM.

**35.** The method of claim 30, wherein the salt in the cell extract is ammonium sulfate, and its concentration is from 20 mM to 1000 mM.

**36.** The method of any one of claims 1 to 35, wherein a cytolytic solution containing an anionic surfactant, an amphoteric surfactant or a non-ionic surfactant is used.

**37.** The method of any one of claims 1 to 36, wherein the step of disrupting cells to prepare a cell extract is carried out in a temperature range of 80°C to 110°C.

**38.** The method of any one of claims 1 to 37, wherein the step of disrupting cells to prepare a cell extract is carried out in the presence of a reducing agent.

**39.** The method of claim 38, wherein the reducing agent is a thiol group-containing compound.

**40.** The method of any one of claims 1 to 39, wherein the method of contacting the cell extract with a nonwoven fabric is filtration.

**41.** The method of any one of claims 1 to 40, wherein the step of adsorbing the nucleic acid in the cell extract onto the nonwoven fabric is followed by a step of washing the nonwoven fabric.

**42.** The method of claim 41, wherein the nonwoven fabric is washed with a solution not containing a chaotropic agent or alcohol.

**43.** The method of claim 41 or 42, wherein the nonwoven fabric is washed with a cytolytic solution.

**44.** The method of any one of claims 41 to 43, wherein the nonwoven fabric is washed with a salt solution having a concentration range of 0.5 M to 2 M.

**45.** A kit for preparation of cellular nucleic acid from a cell-containing sample, which kit comprises:

(a) a device incorporating a nonwoven fabric; and (b) a solution containing either a cytolytic solution or a nucleic acid eluent.

**46.** A kit for extraction of nucleic acid from cells and preparation of a nonwoven fabric having said nucleic acid adsorbed thereonto, which kit comprises:

(a) a device incorporating a nonwoven fabric; and (b) a solution containing either a cytolytic solution or a washing solution.

**47.** A method of eluting nucleic acid adsorbed on solid surface by treating it under alkaline conditions of pH 12 or higher.

**48.** A method of eluting nucleic acid adsorbed on solid surface by treating it with active oxygen.

**49.** A method of eluting nucleic acid adsorbed on solid surface by treating it with a surfactant.

**50.** A nonwoven fabric which has adsorbed nucleic acid.

FIG. 1

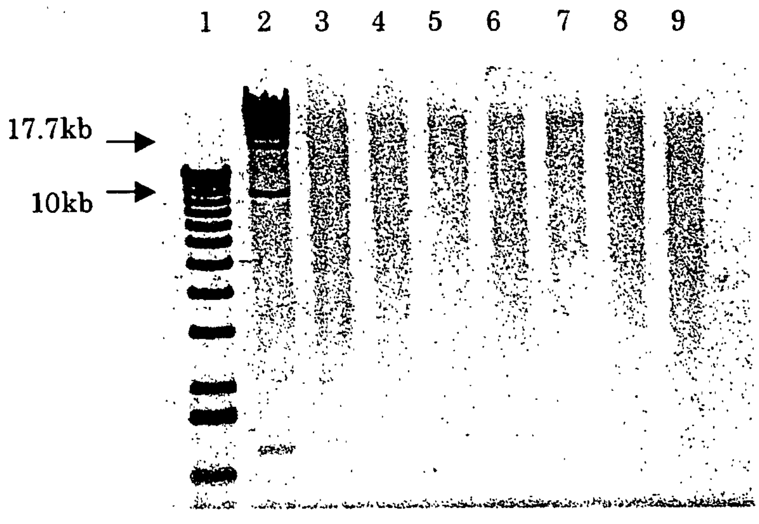


FIG. 2

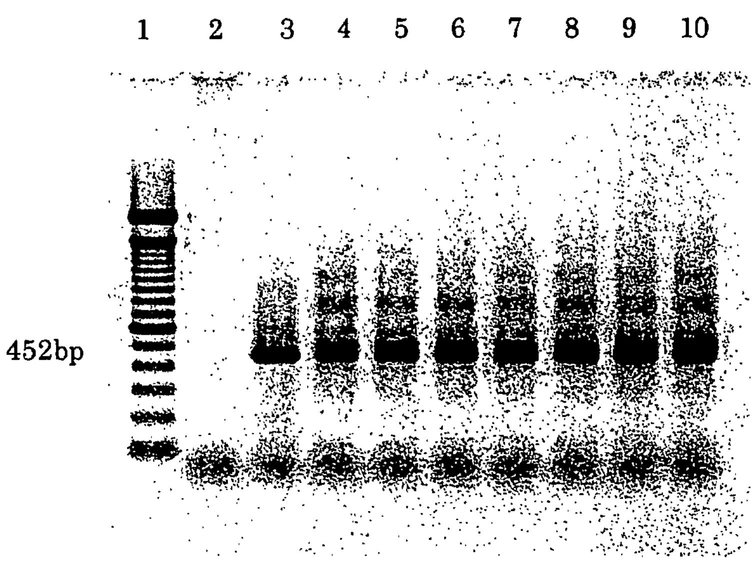

FIG. 3

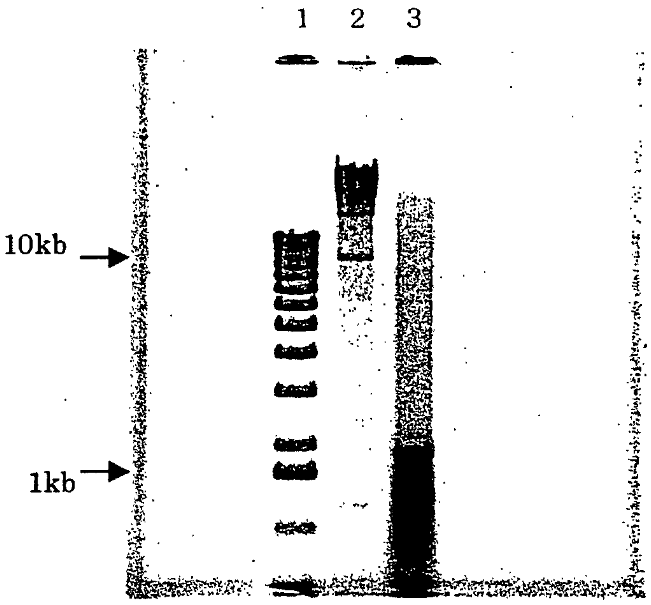


FIG. 4

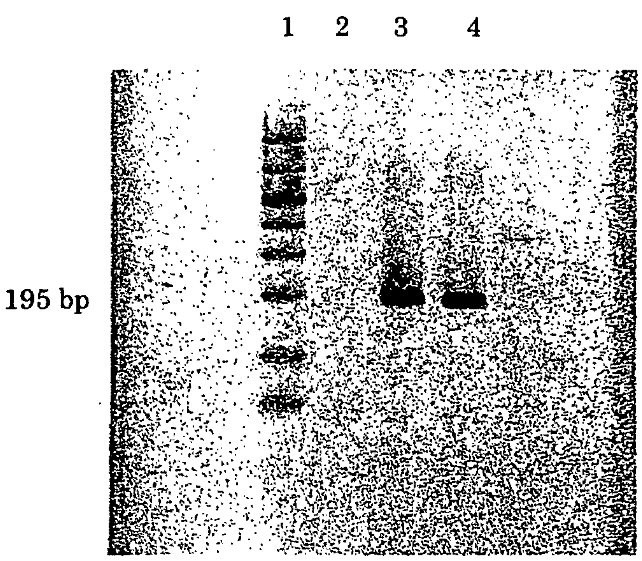

FIG. 5

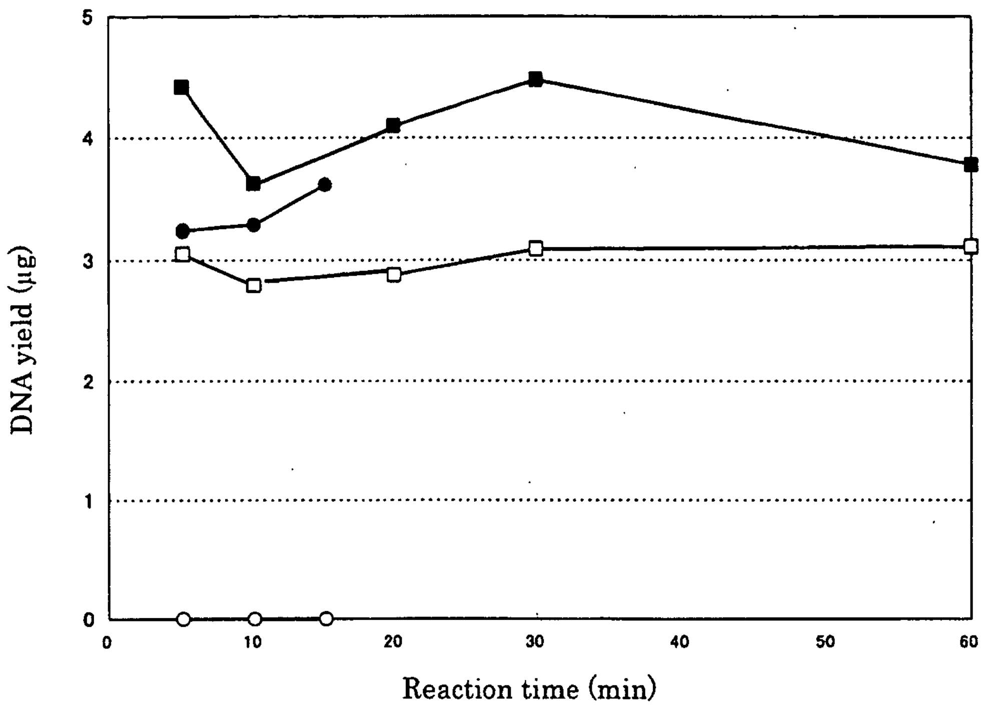
5
4
3
2
1
0
DNA yield (μg)
0
10
20
30
40
50
60
Reaction time (min)

FIG. 6

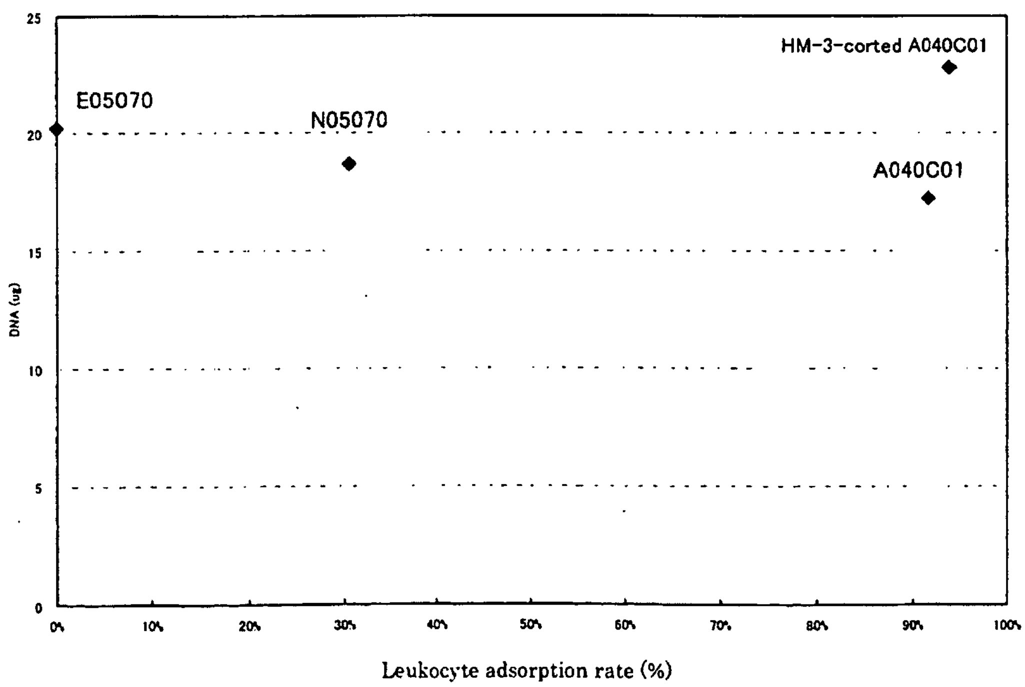
25
20
15
10
5
0
DNA (μg)
E05070
N05070
HM-3-corted A040C01
A040C01
0%
10%
20%
30%
40%
50%
60%
70%
80%
90%
100%
Leukocyte adsorption rate (%)

FIG. 7

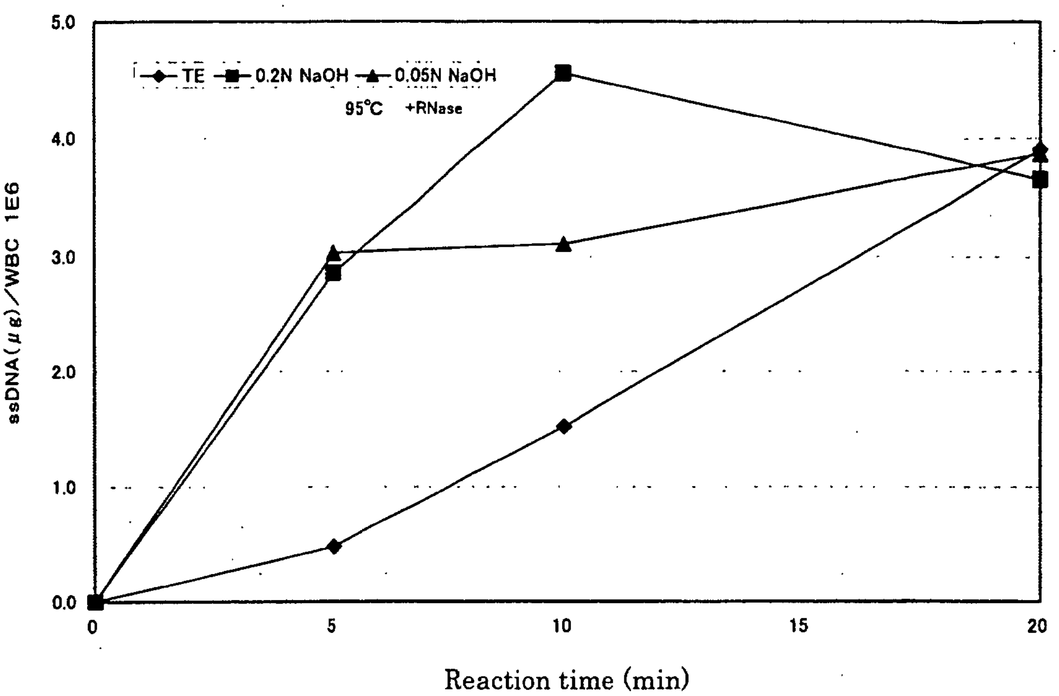
5.0
4.0
3.0
2.0
1.0
0.0
0
5
10
15
20
TE
0.2N NaOH
0.05N NaOH
95°C +RNase
ssDNA(μg)/WBC 1E6
Reaction time (min)

FIG. 8

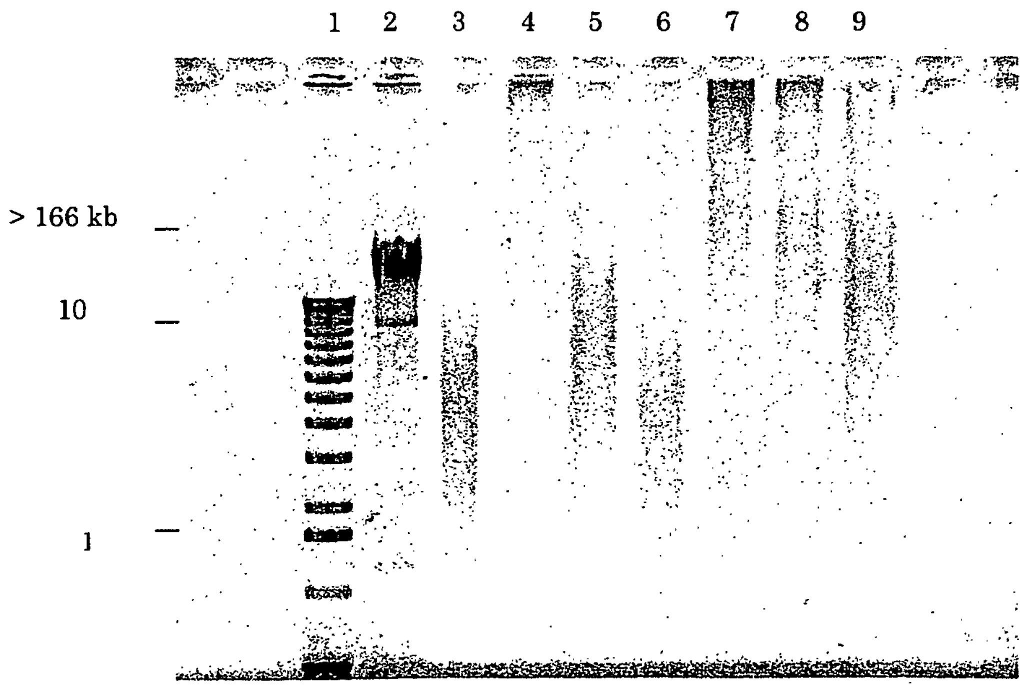
1 2 3 4 5 6 7 8 9
> 166 kb
10
1

FIG. 9

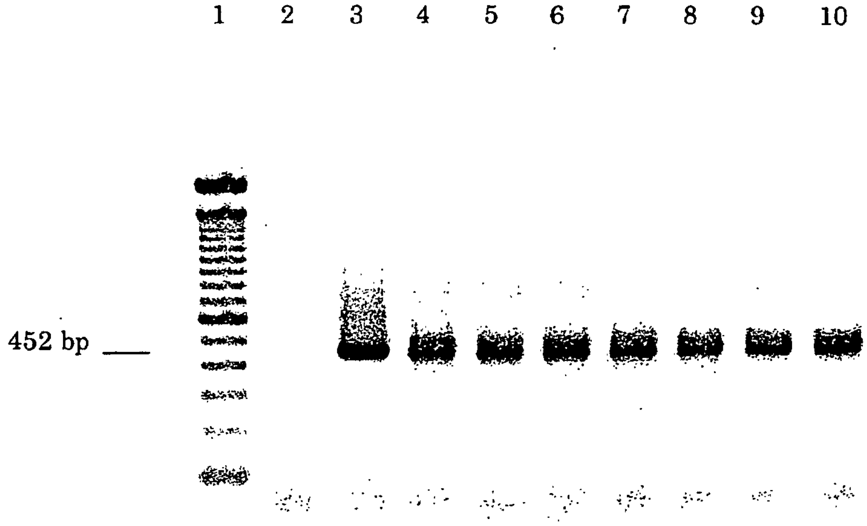
1 2 3 4 5 6 7 8 9 10
452 bp

FIG. 10

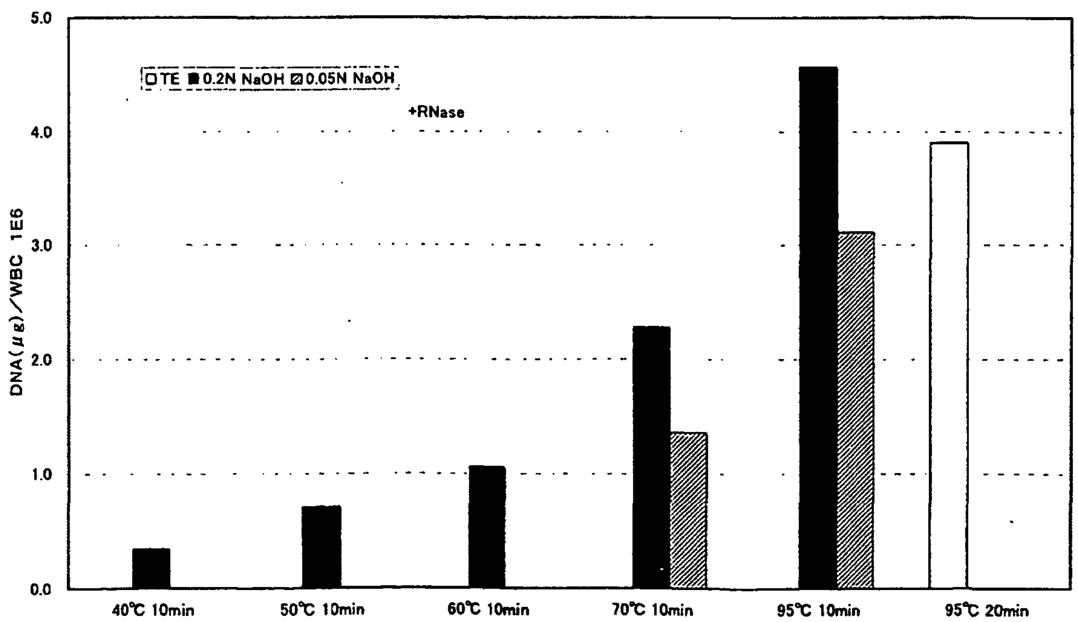
5.0
4.0
3.0
2.0
1.0
0.0
DNA(μg)/WBC 1E6
TE 0.2N NaOH 0.05N NaOH
+RNase
40℃ 10min
50℃ 10min
60℃ 10min
70℃ 10min
95℃ 10min
95℃ 20min

FIG. 1 1

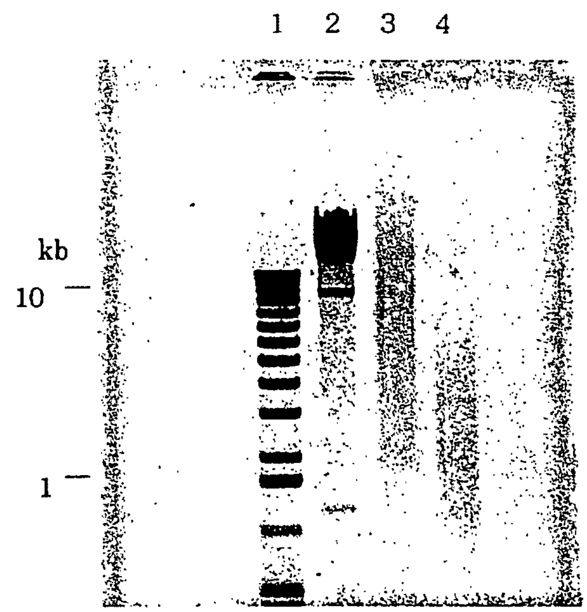


FIG. 1 2

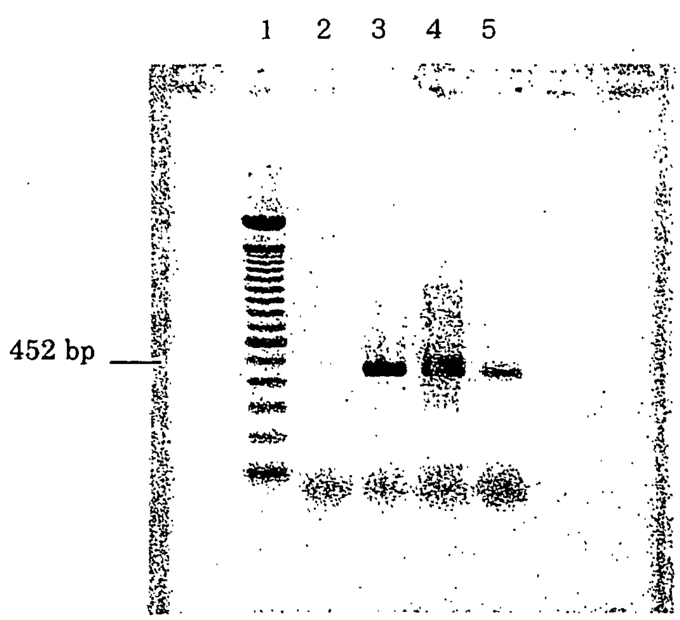

FIG. 1 3

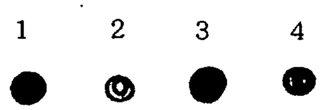


FIG. 1 4

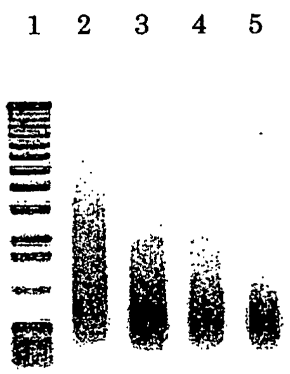

FIG. 15

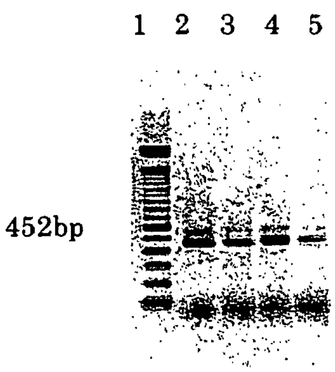


FIG. 16

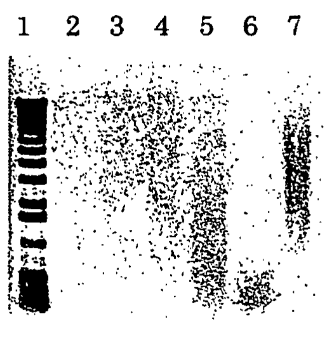

FIG. 1 7

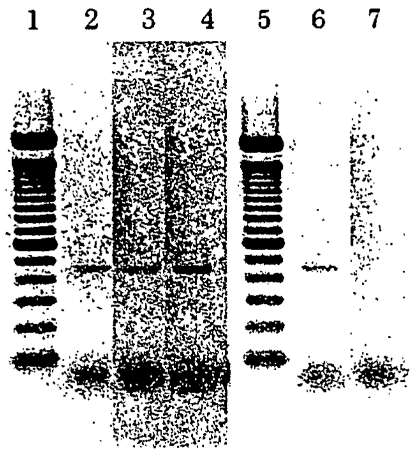


FIG. 1 8

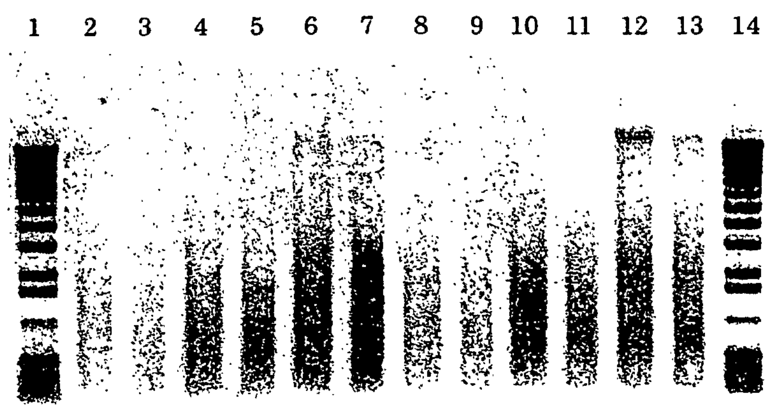

FIG. 19

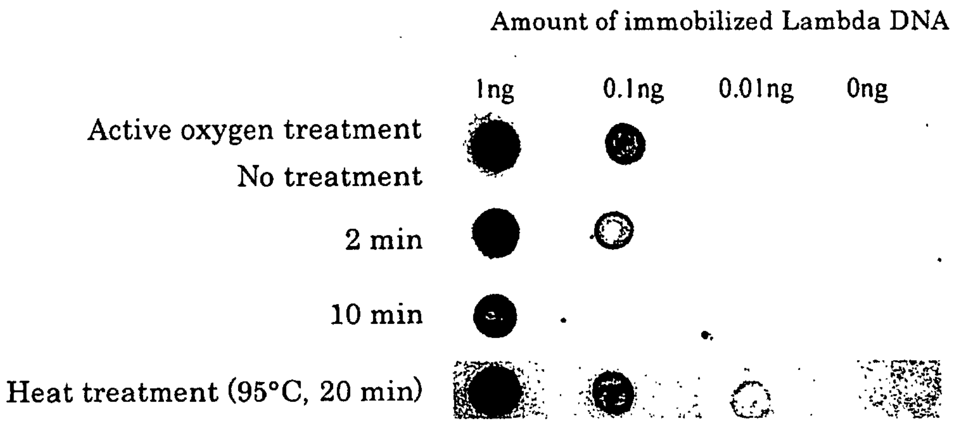
Amount of immobilized Lambda DNA
1ng
0.1ng
0.01ng
0ng
Active oxygen treatment
No treatment
2 min
10 min
Heat treatment (95°C, 20 min)

FIG. 20

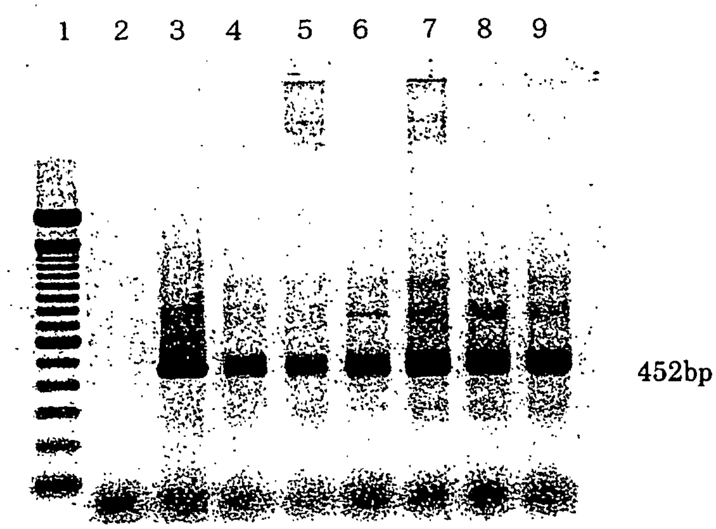
1 2 3 4 5 6 7 8 9
452bp

FIG. 2 1

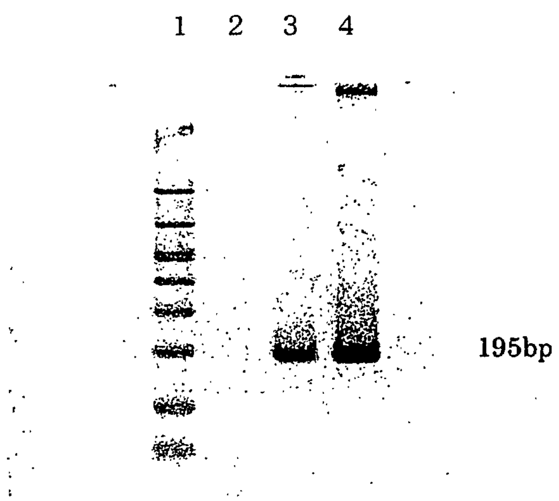
1 2 3 4
195bp

FIG. 2 2

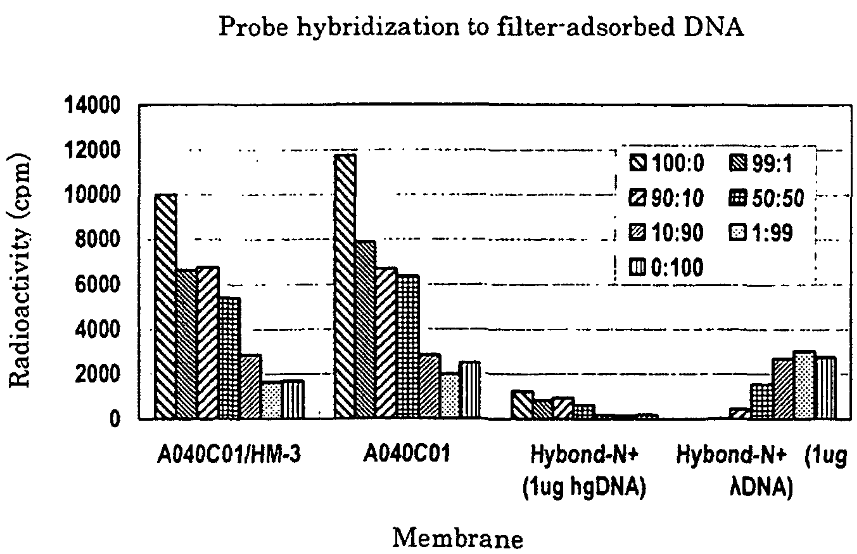
Probe hybridization to filter-adsorbed DNA
Radioactivity (cpm)
14000
12000
10000
8000
6000
4000
2000
0
100:0
99:1
90:10
50:50
10:90
1:99
0:100
A040C01/HM-3
A040C01
Hybond-N+ (1ug hgDNA)
Hybond-N+ (1ug λDNA)
Membrane

FIG. 2 3 A

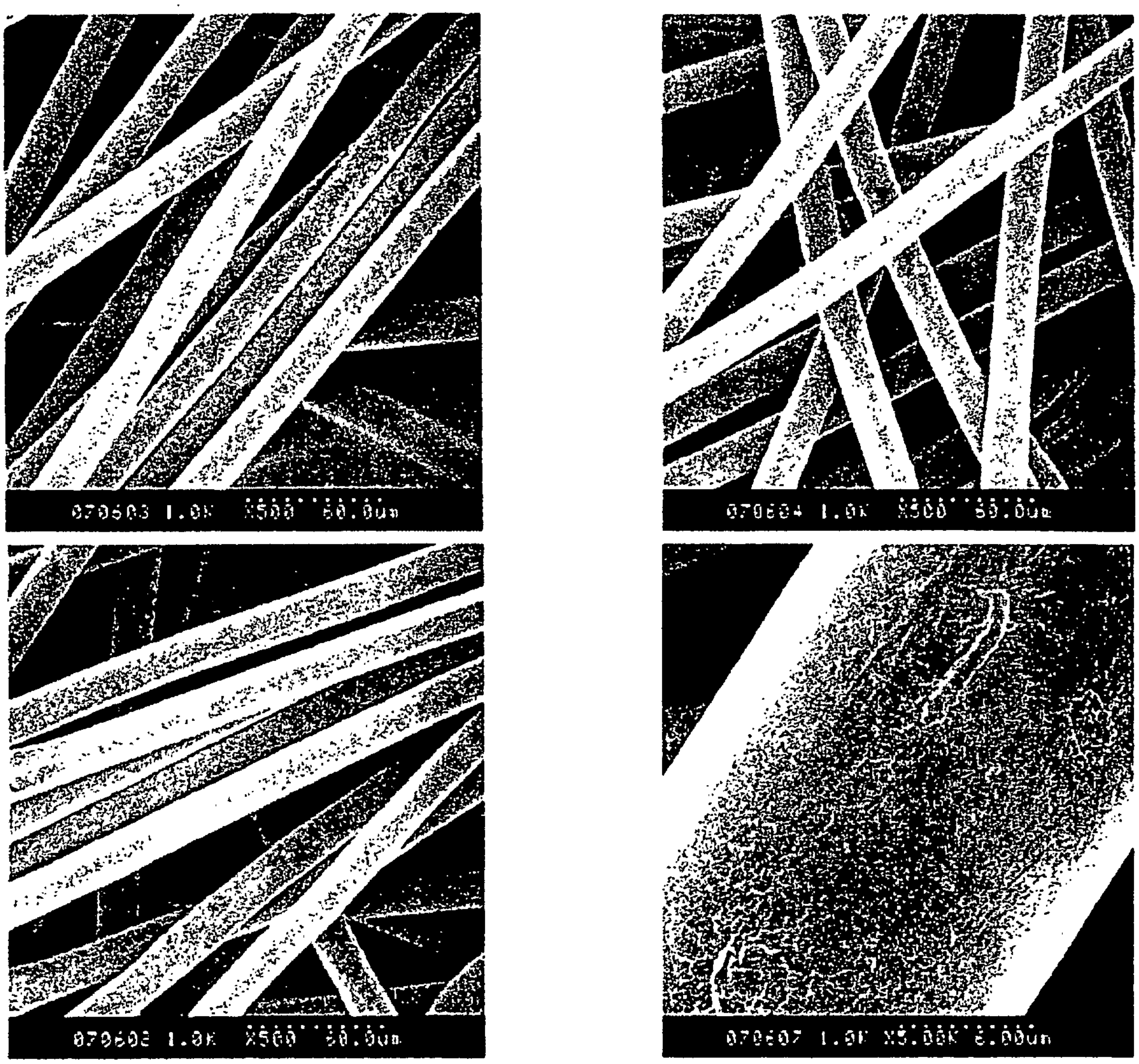
070603 1.0K X500 60.0um
070604 1.0K X500 60.0um
070602 1.0K X500 60.0um
070607 1.0K X5.00K 6.00um

FIG. 2 3 B

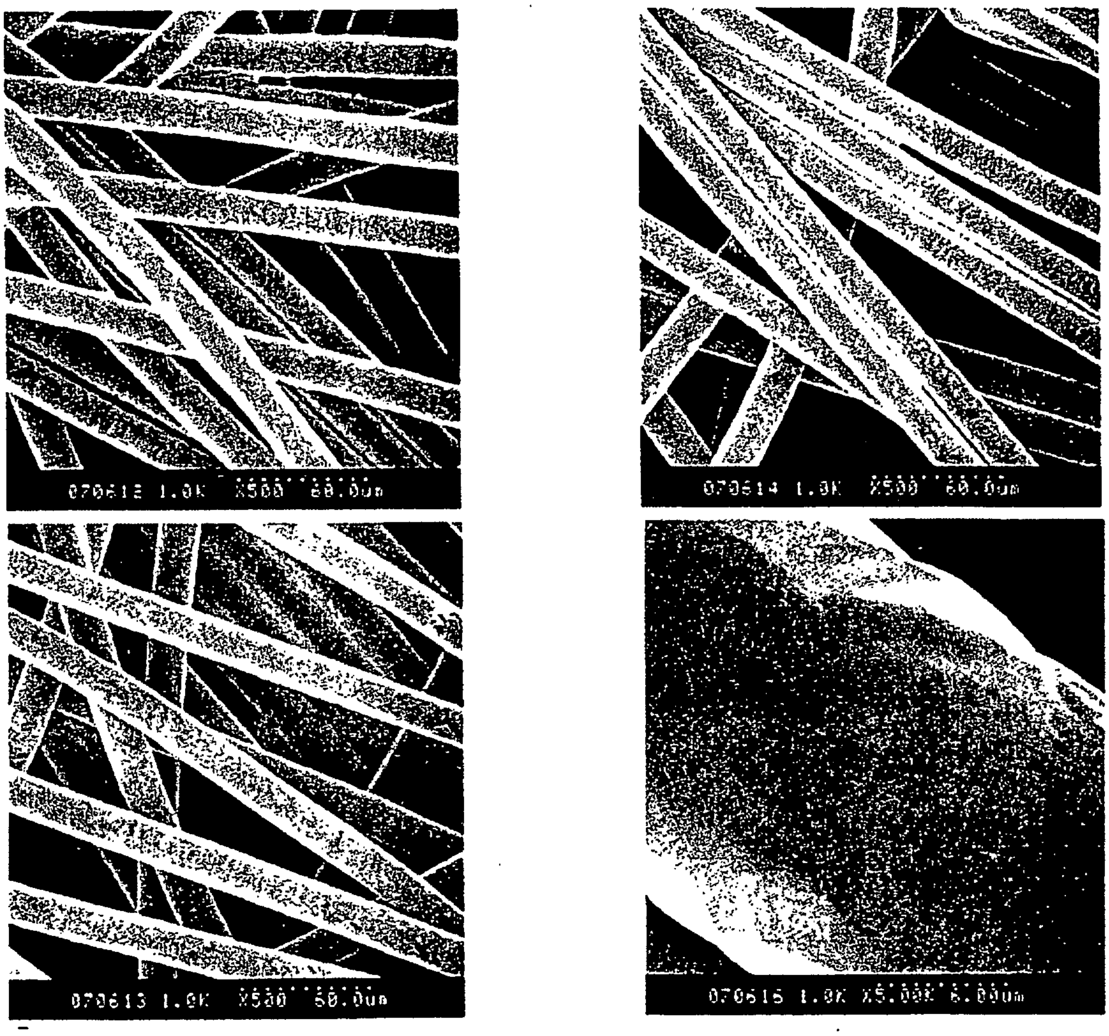
070614 1.0K X500 60.0um
070613 1.0K X500 60.0um
070616 1.0K X5.00K 6.00um

FIG. 24

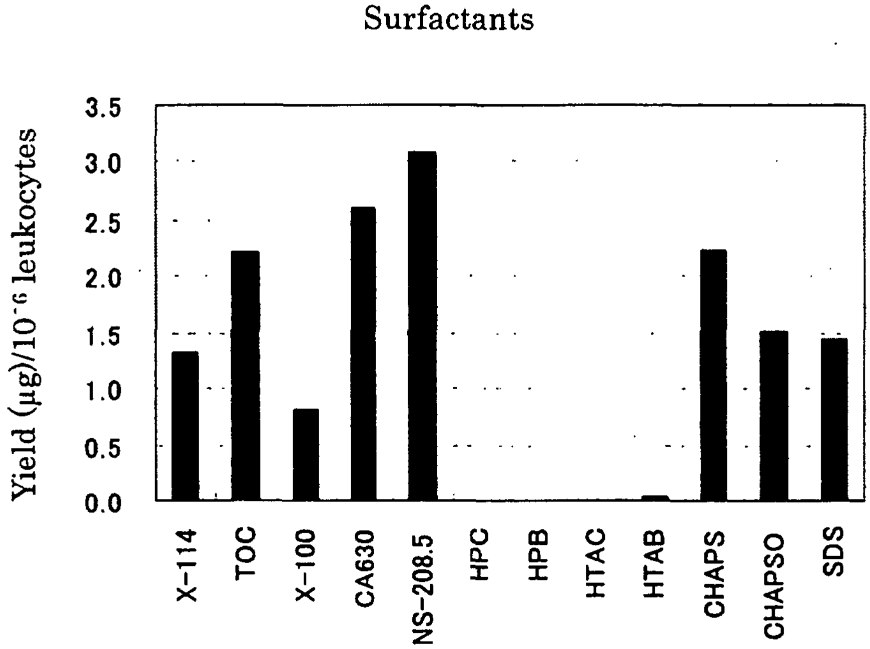
Surfactants
Yield (μg)/10^-6 leukocytes
3.5
3.0
2.5
2.0
1.5
1.0
0.5
0.0
X-114
TOC
X-100
CA630
NS-208.5
HPC
HPB
HTAC
HTAB
CHAPS
CHAPSO
SDS

FIG. 25

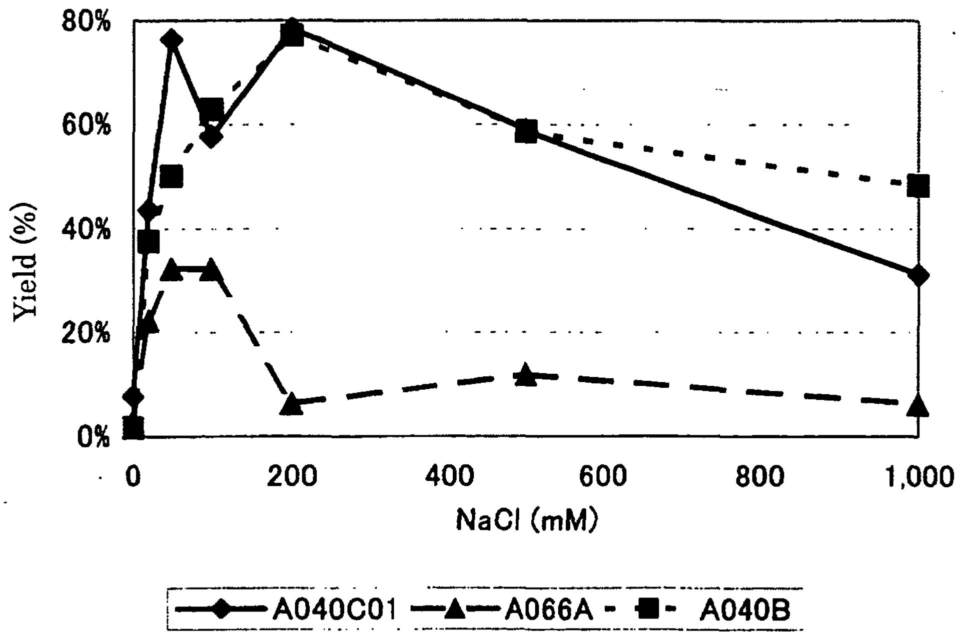
80%
60%
40%
20%
0%
Yield (%)
0
200
400
600
800
1,000
NaCl (mM)
A040C01
A066A
A040B

FIG. 2 6

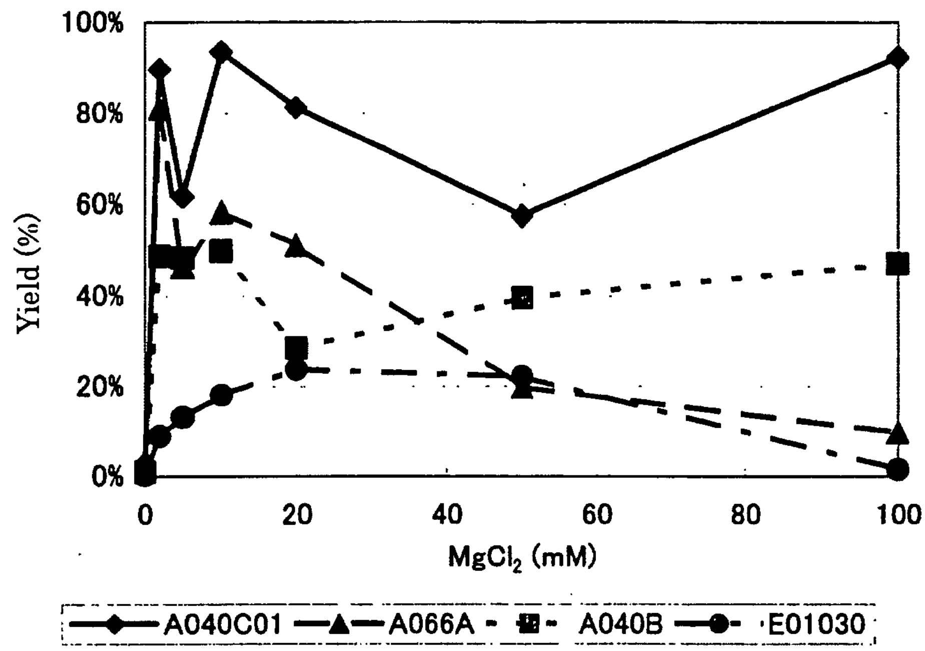
100%
80%
60%
40%
20%
0%
Yield (%)
0
20
40
60
80
100
MgCl2 (mM)
A040C01
A066A
A040B
E01030

FIG. 2 7

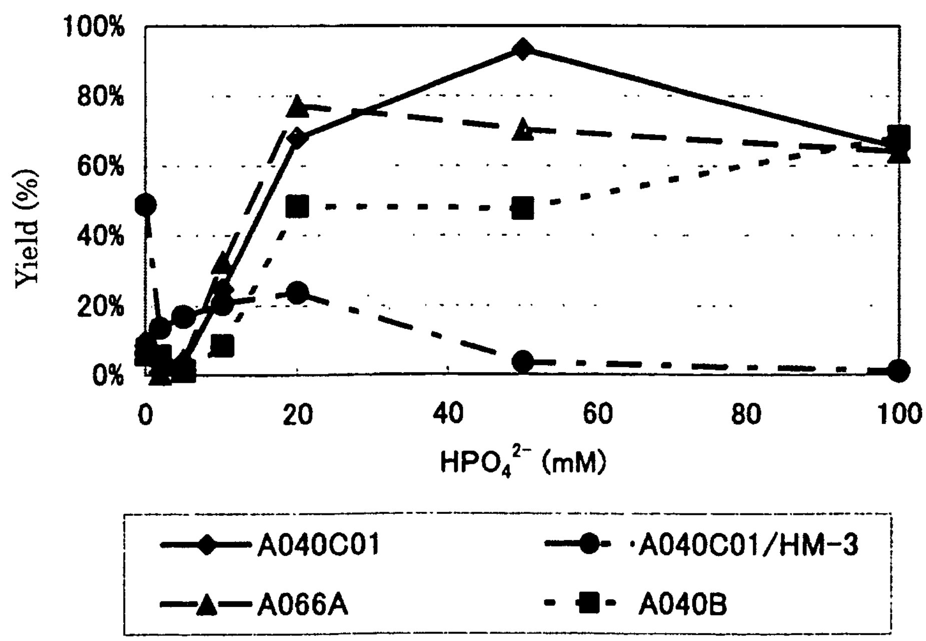
100%
80%
60%
40%
20%
0%
Yield (%)
0
20
40
60
80
100
HPO4 2- (mM)
A040C01
A040C01/HM-3
A066A
A040B

FIG. 2 8

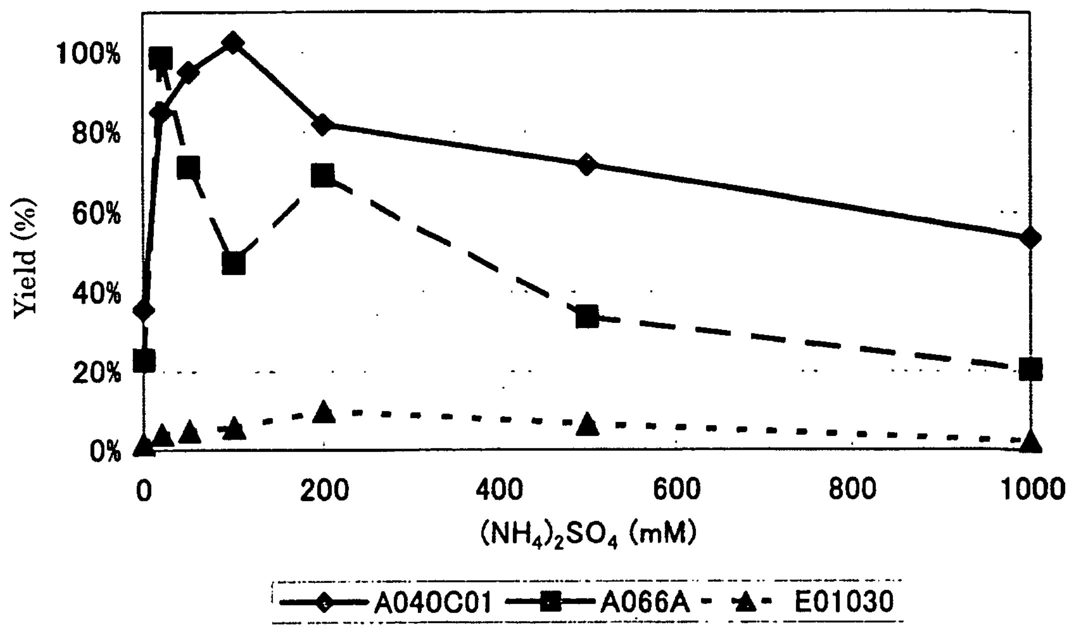
100%
80%
60%
40%
20%
0%
Yield (%)
0
200
400
600
800
1000
$(NH_4)_2SO_4$ (mM)
A040C01
A066A
E01030

FIG. 2 9

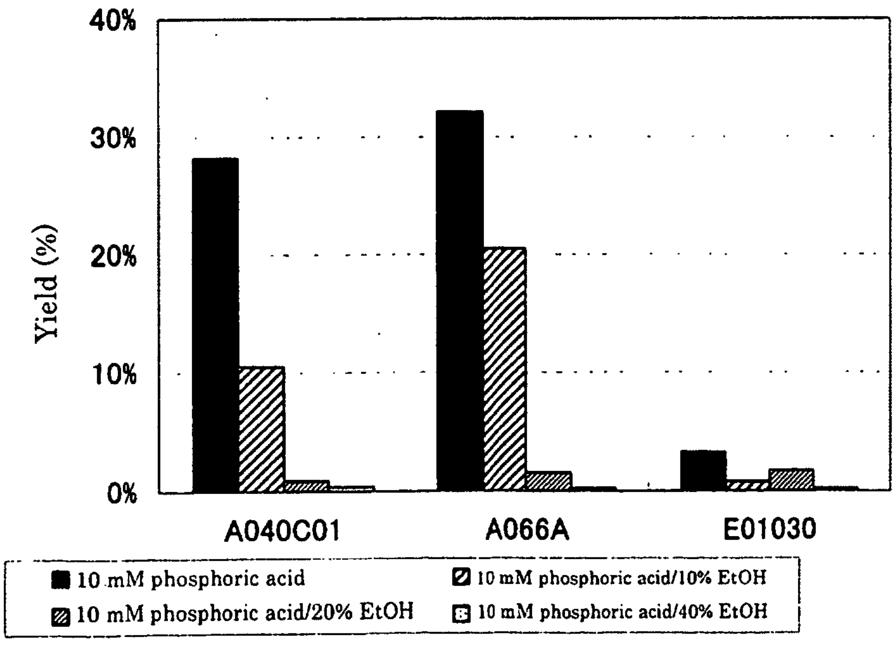
40%
30%
20%
10%
0%
Yield (%)
A040C01
A066A
E01030
10 mM phosphoric acid
10 mM phosphoric acid/10% EtOH
10 mM phosphoric acid/20% EtOH
10 mM phosphoric acid/40% EtOH

FIG. 30

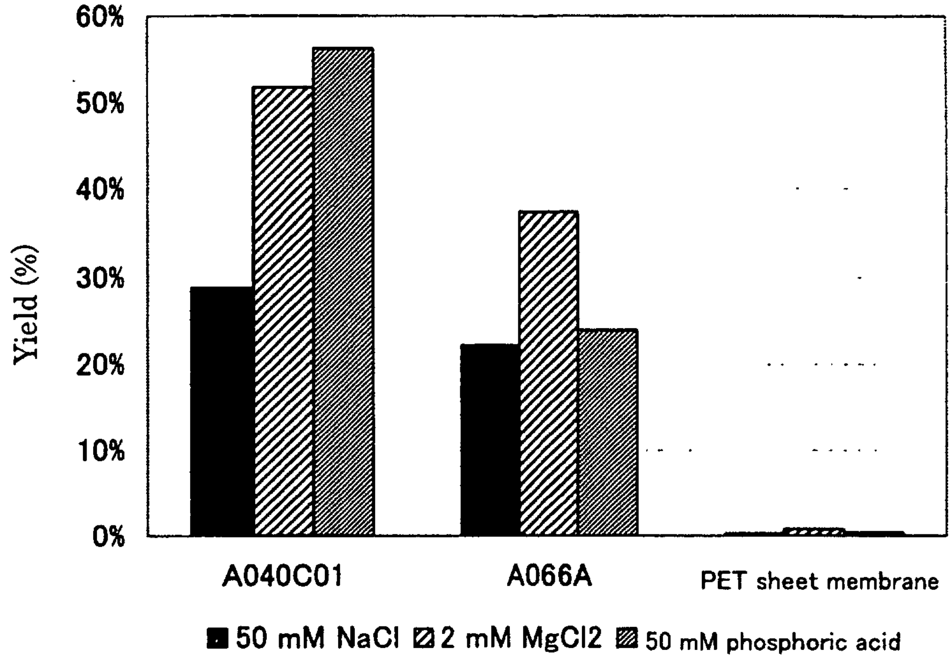
60%
50%
40%
30%
20%
10%
0%
Yield (%)
A040C01
A066A
PET sheet membrane
50 mM NaCl
2 mM MgCl2
50 mM phosphoric acid

FIG. 31

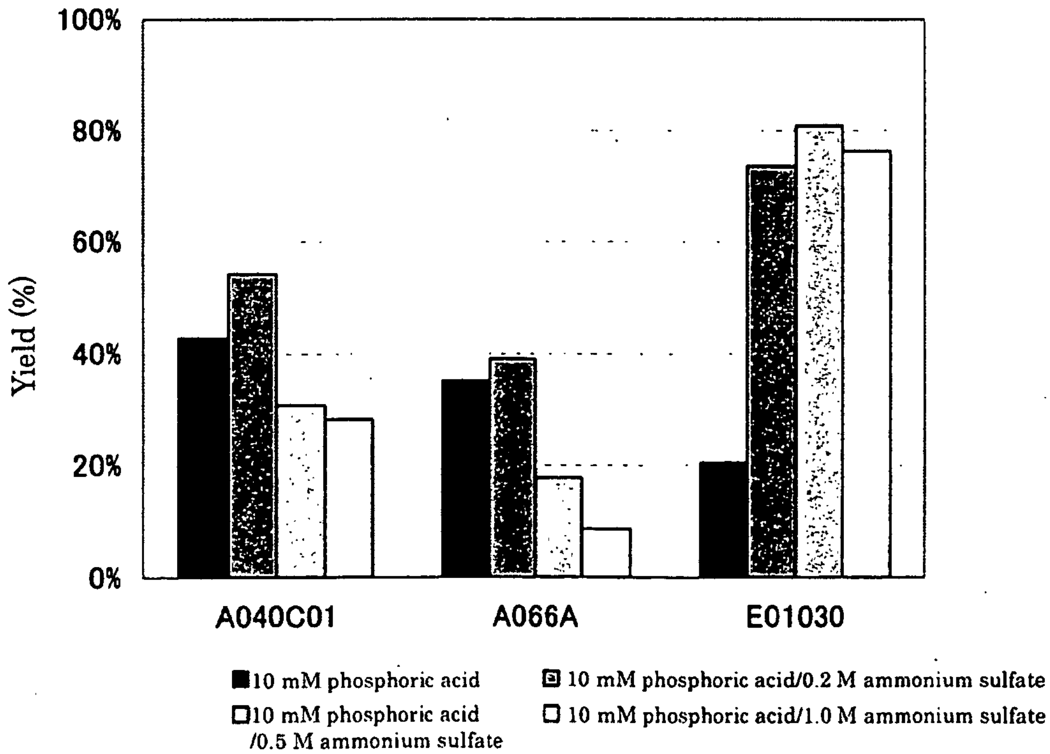
100%
80%
60%
40%
20%
0%
Yield (%)
A040C01
A066A
E01030
10 mM phosphoric acid
10 mM phosphoric acid/0.2 M ammonium sulfate
10 mM phosphoric acid /0.5 M ammonium sulfate
10 mM phosphoric acid/1.0 M ammonium sulfate

FIG. 3 2

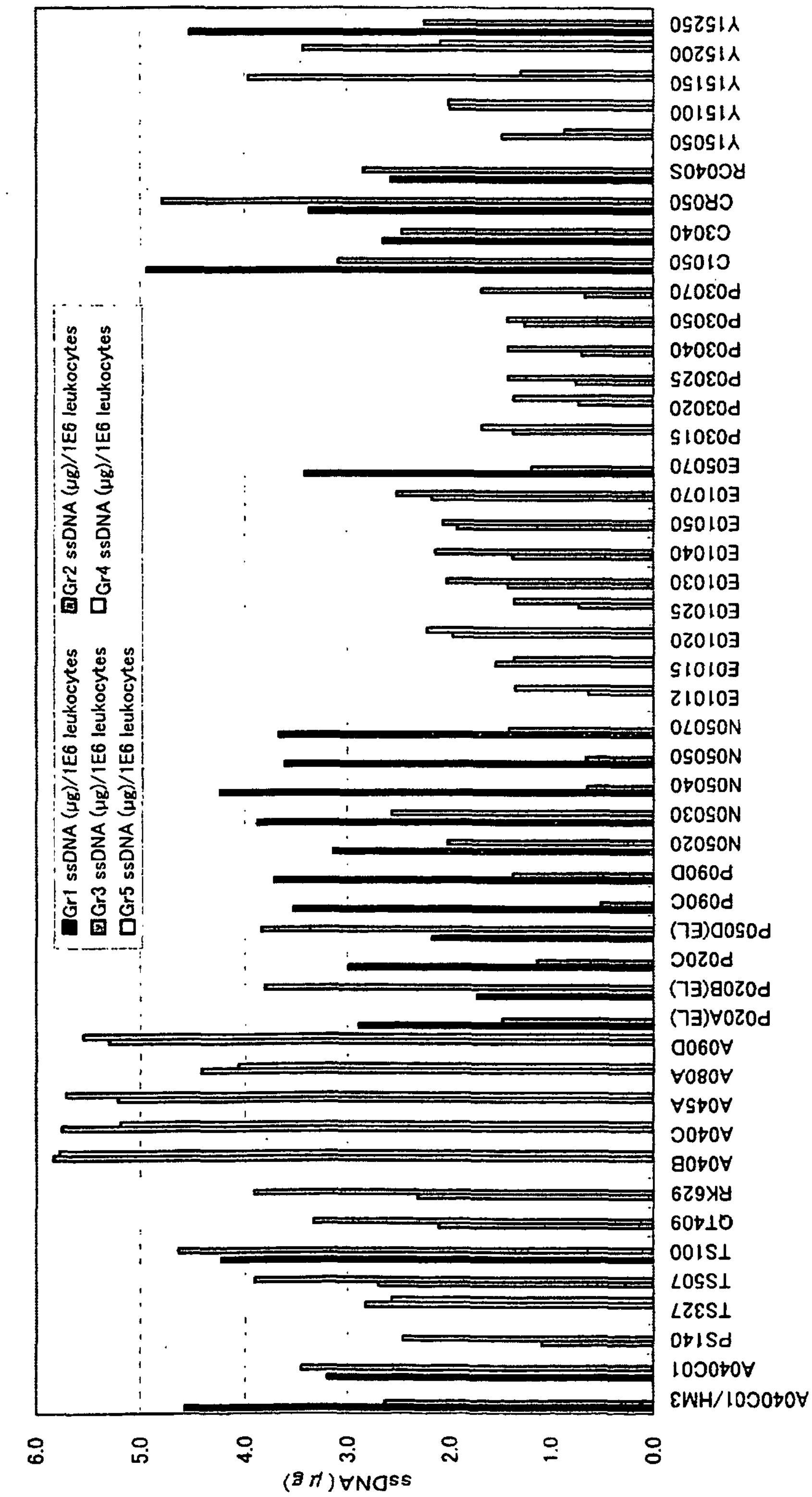
Gr1 ssDNA (μg)/1E6 leukocytes
Gr2 ssDNA (μg)/1E6 leukocytes
Gr3 ssDNA (μg)/1E6 leukocytes
Gr4 ssDNA (μg)/1E6 leukocytes
Gr5 ssDNA (μg)/1E6 leukocytes
ssDNA(μg)
0.0
1.0
2.0
3.0
4.0
5.0
6.0
A040C01/HM3
A040C01
PS140
TS327
TS507
TS100
QT409
RK629
A040B
A040C
A045A
A080A
A090D
P020A(EL)
P020B(EL)
P020C
P050D(EL)
P090C
P090D
N05020
N05030
N05040
N05050
N05070
E01012
E01015
E01020
E01025
E01030
E01040
E01050
E01070
E05070
P03015
P03020
P03025
P03040
P03050
P03070
C1050
C3040
CR050
RC040S
Y15050
Y15100
Y15150
Y15200
Y15250

FIG. 3 3

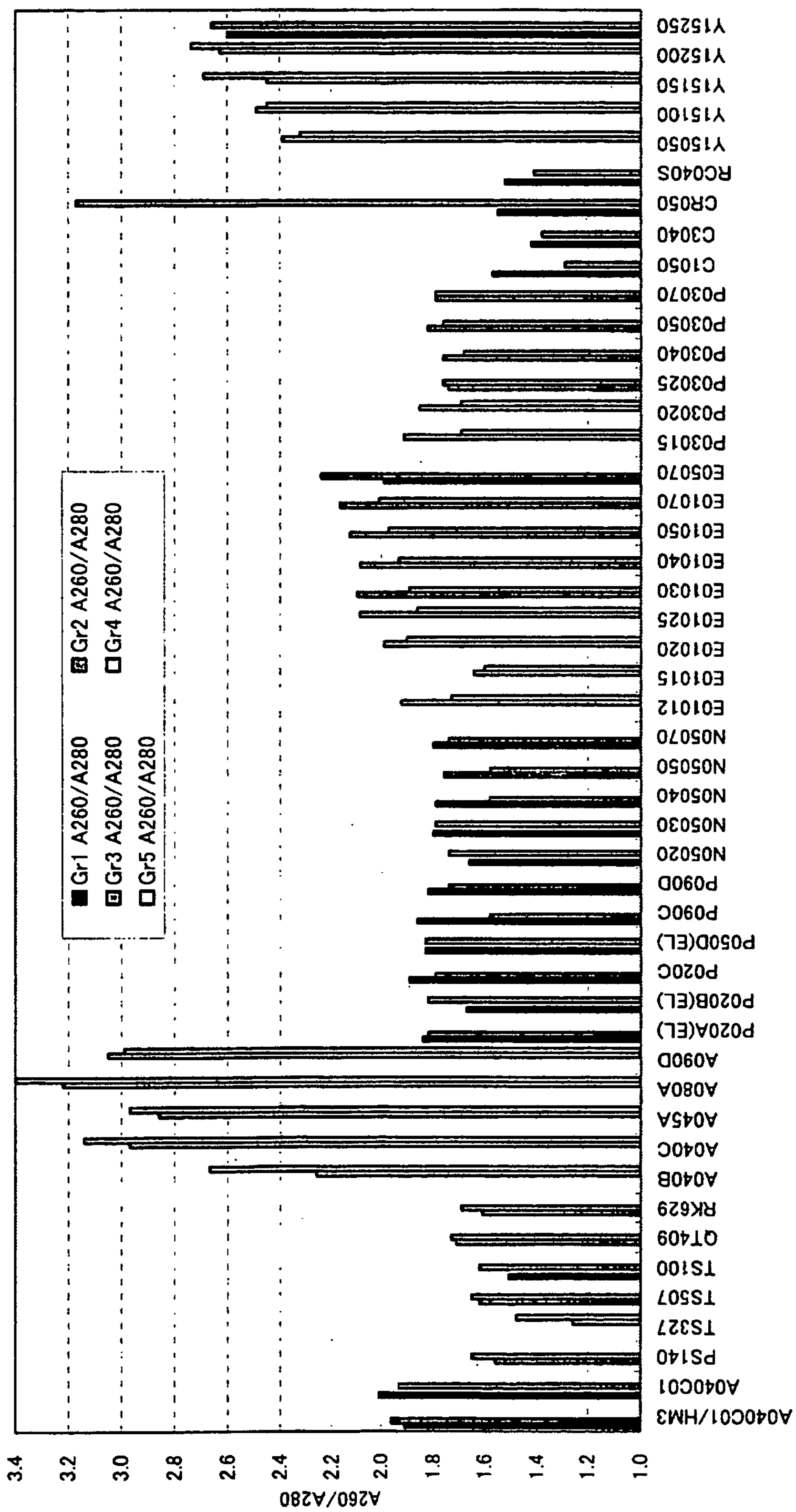
Gr1 A260/A280
Gr2 A260/A280
Gr3 A260/A280
Gr4 A260/A280
Gr5 A260/A280
A260/A280
3.4
3.2
3.0
2.8
2.6
2.4
2.2
2.0
1.8
1.6
1.4
1.2
1.0
A040C01/HM3
A040C01
PS140
TS327
TS507
TS100
QT409
RK629
A040B
A040C
A045A
A080A
A090D
P020A(EL)
P020B(EL)
P020C
P050D(EL)
P090C
P090D
N05020
N05030
N05040
N05050
N05070
E01012
E01015
E01020
E01025
E01030
E01040
E01050
E01070
E05070
P03015
P03020
P03025
P03040
P03050
P03070
C1050
C3040
CR050
RC040S
Y15050
Y15100
Y15150
Y15200
Y15250

FIG. 3 4

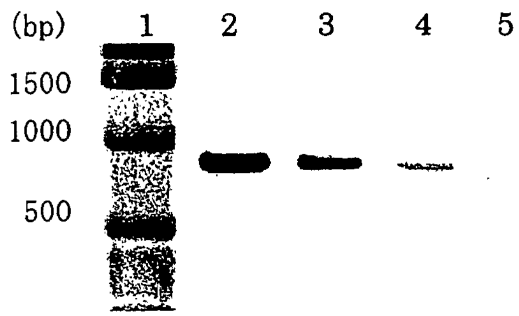


FIG. 3 5

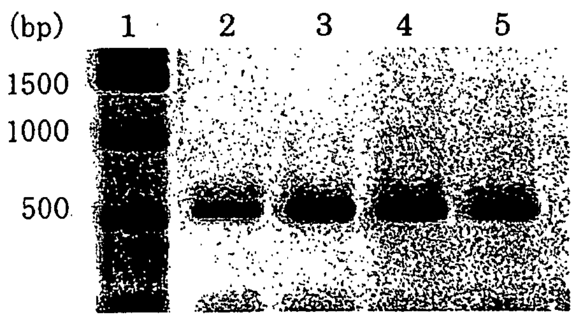


FIG. 3 6

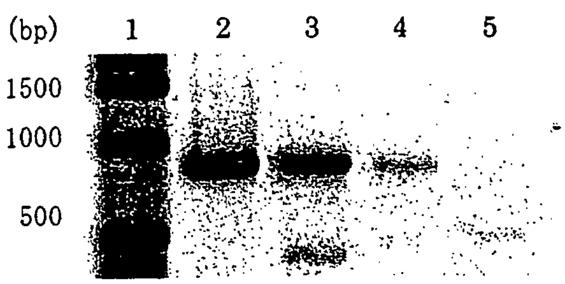

FIG. 37

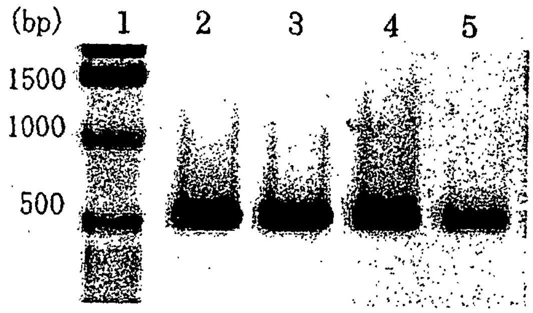


FIG. 38

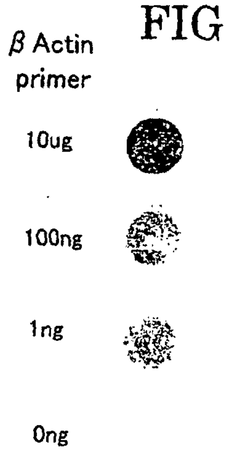


FIG. 39

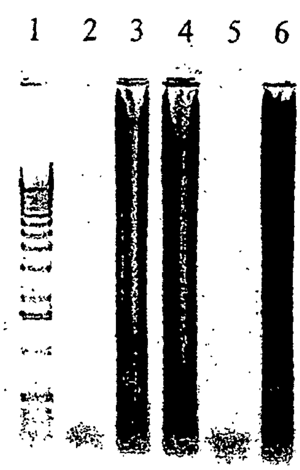

FIG. 4 0

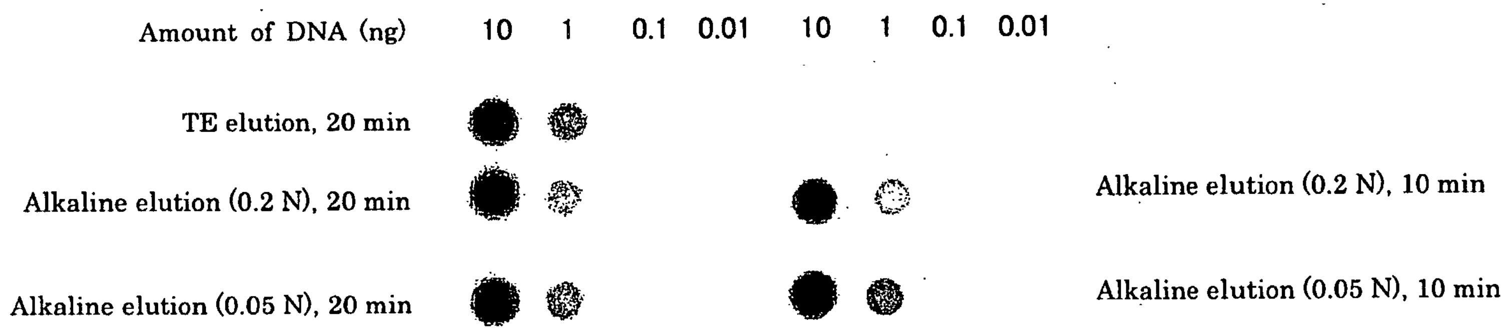
Amount of DNA (ng)
10
1
0.1
0.01
10
1
0.1
0.01
TE elution, 20 min
Alkaline elution (0.2 N), 20 min
Alkaline elution (0.05 N), 20 min
Alkaline elution (0.2 N), 10 min
Alkaline elution (0.05 N), 10 min

FIG. 4 1

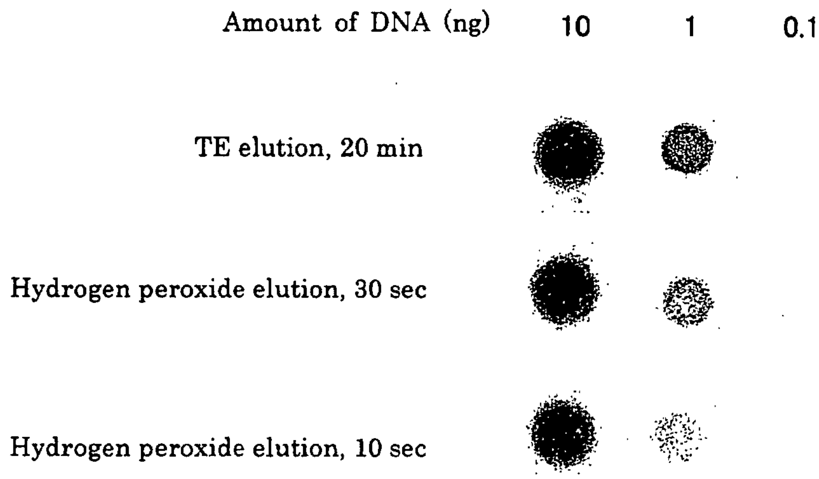
Amount of DNA (ng)
10
1
0.1
TE elution, 20 min
Hydrogen peroxide elution, 30 sec
Hydrogen peroxide elution, 10 sec

FIG. 4 2

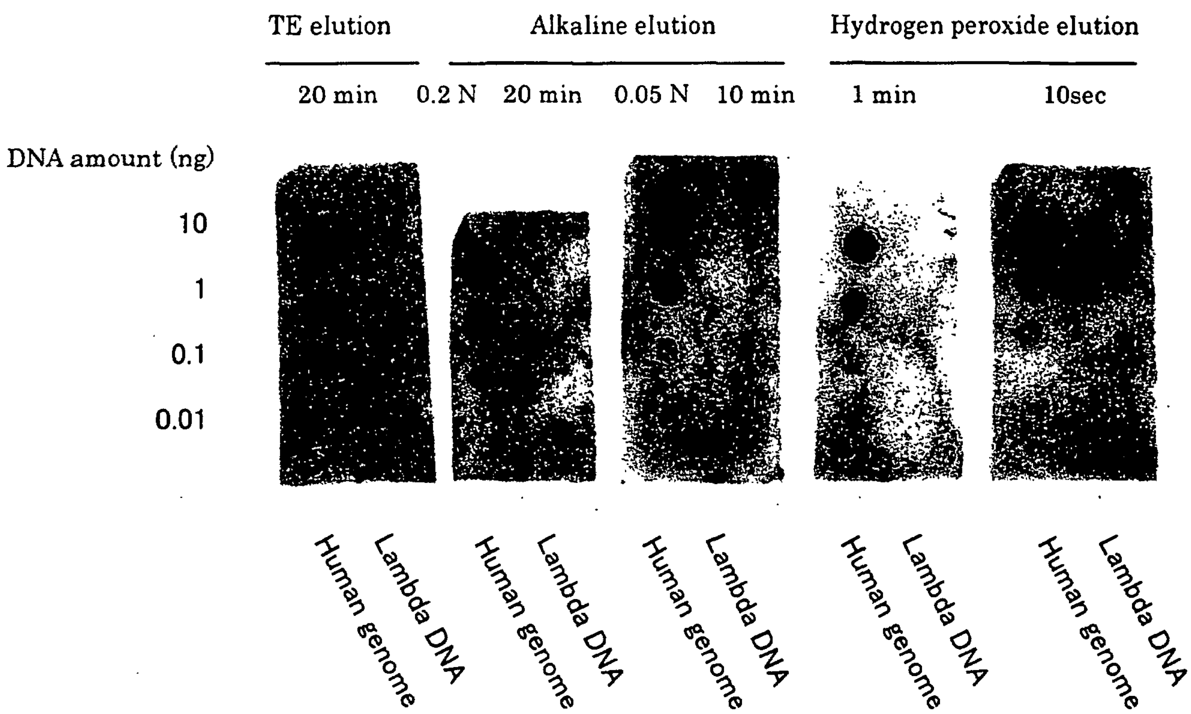
TE elution
Alkaline elution
Hydrogen peroxide elution
20 min
0.2 N 20 min
0.05 N 10 min
1 min
10sec
DNA amount (ng)
10
1
0.1
0.01
Human genome
Lambda DNA
Human genome
Lambda DNA
Human genome
Lambda DNA
Human genome
Lambda DNA
Human genome
Lambda DNA

FIG. 4 3

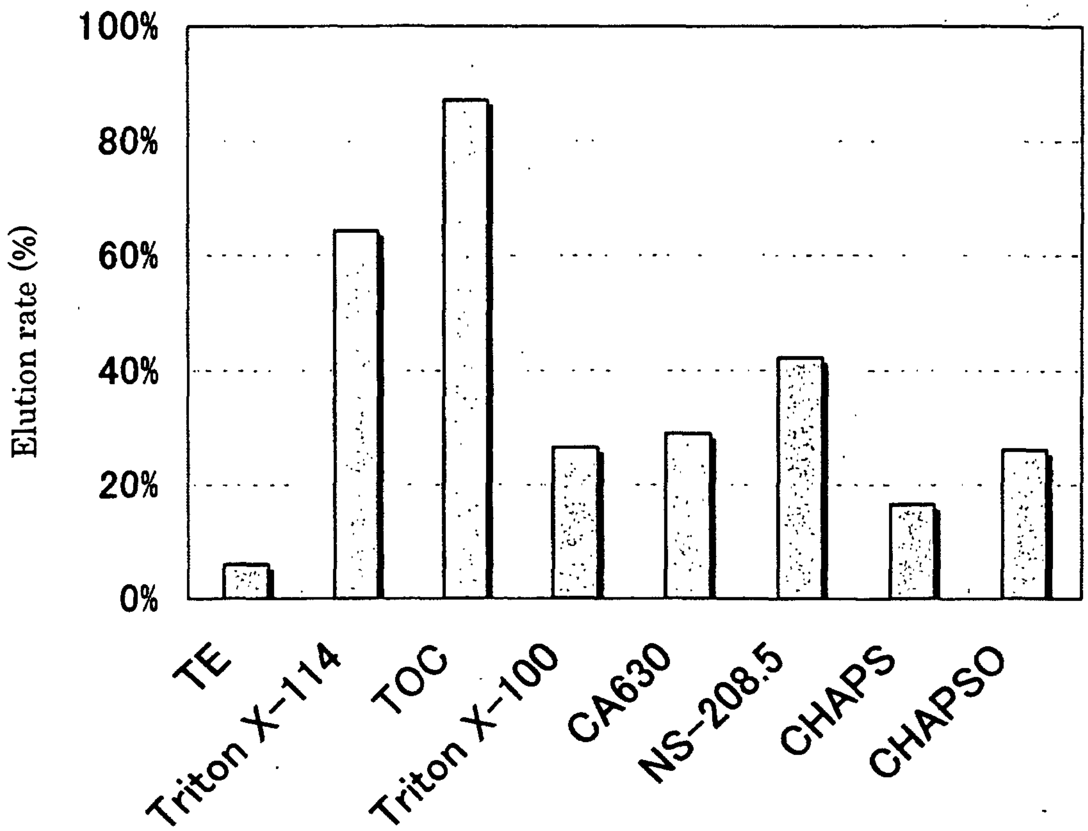
100%
80%
60%
40%
20%
0%
Elution rate (%)
TE
Triton X-114
TOC
Triton X-100
CA630
NS-208.5
CHAPS
CHAPSO

FIG. 4 4

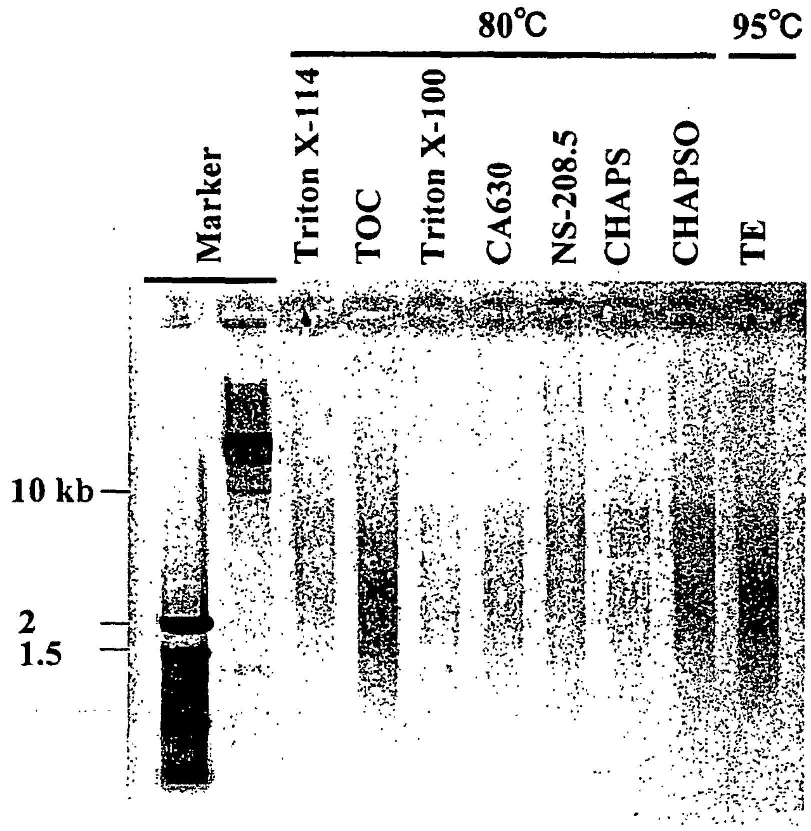
80°C
95°C
Marker
Triton X-114
TOC
Triton X-100
CA630
NS-208.5
CHAPS
CHAPSO
TE
10 kb
2
1.5

FIG. 45

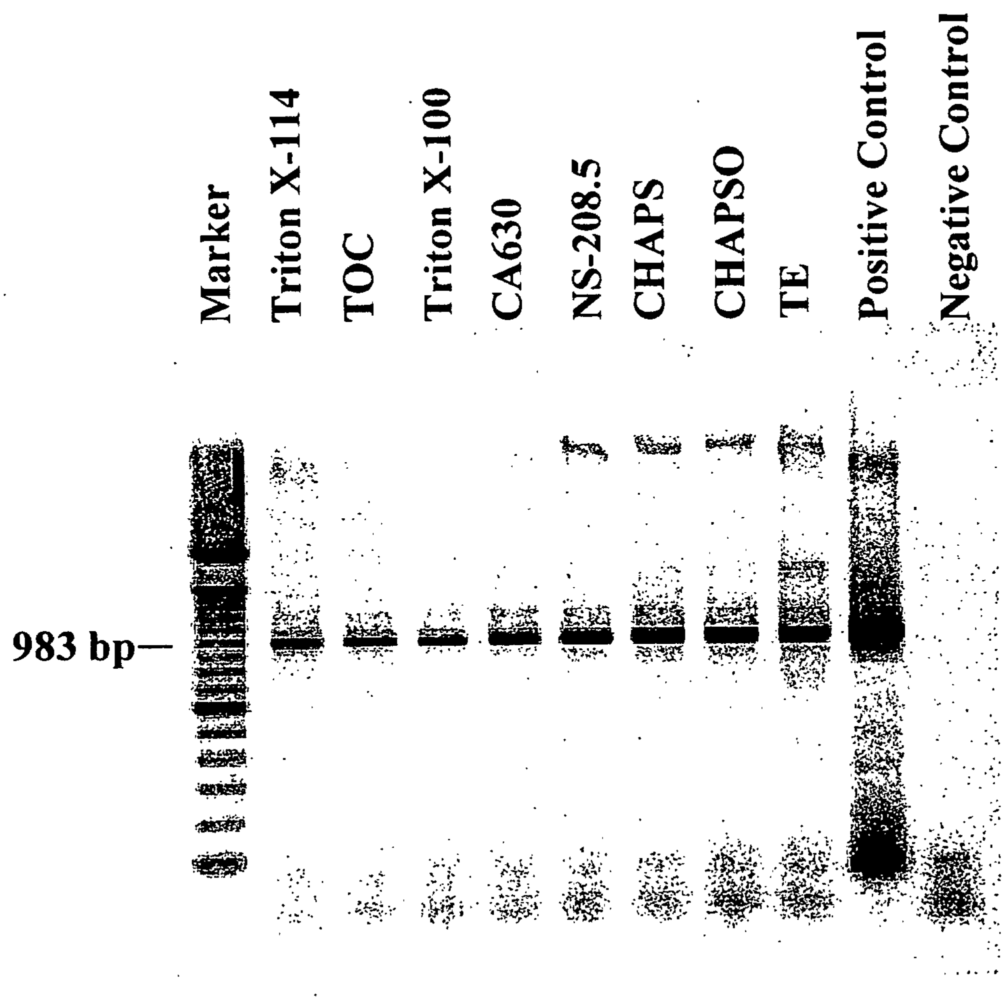
Marker
Triton X-114
TOC
Triton X-100
CA630
NS-208.5
CHAPS
CHAPSO
TE
Positive Control
Negative Control
983 bp—

FIG. 46

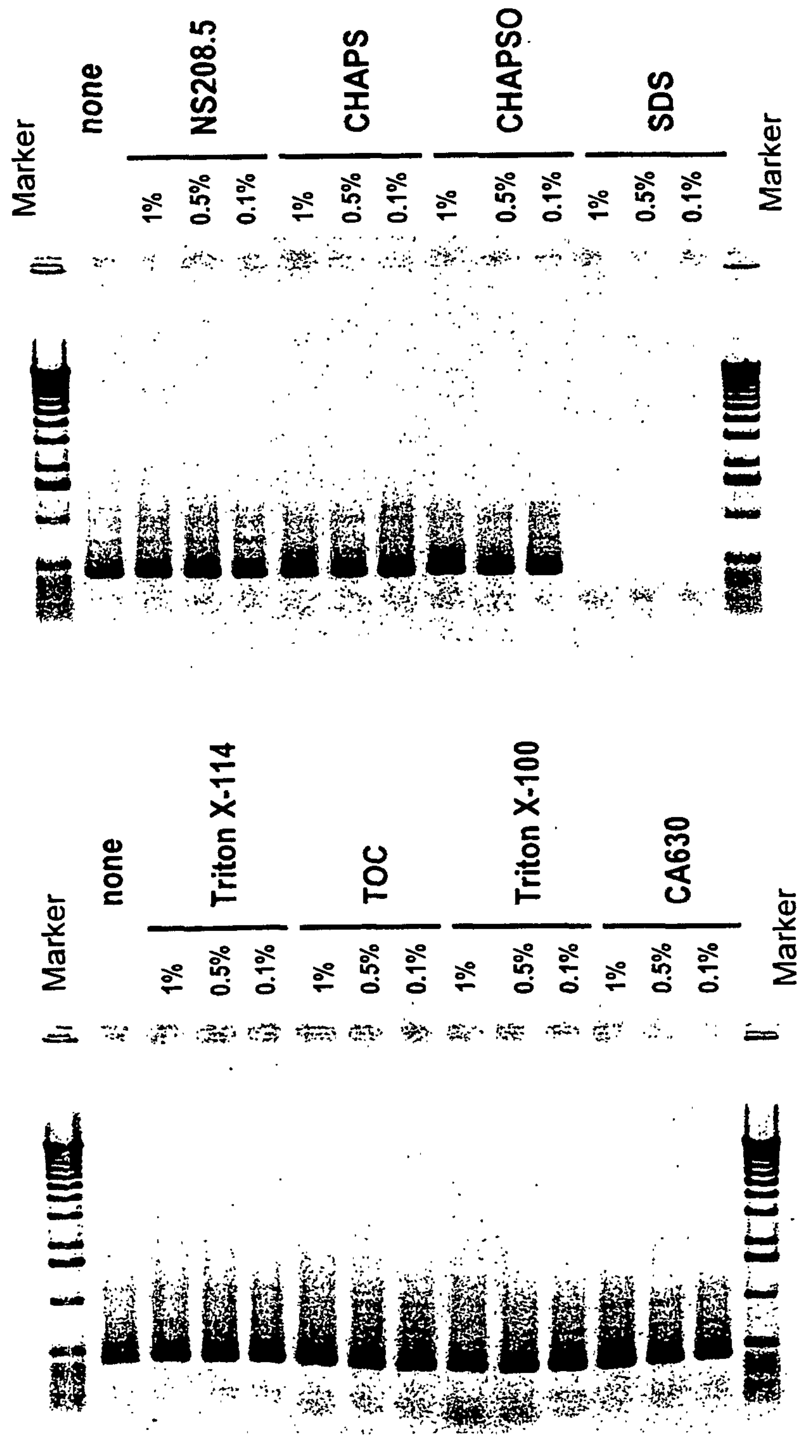
Marker
none
NS208.5
CHAPS
CHAPSO
SDS
1%
0.5%
0.1%
Marker
Triton X-114
TOC
Triton X-100
CA630

FIG. 4 7

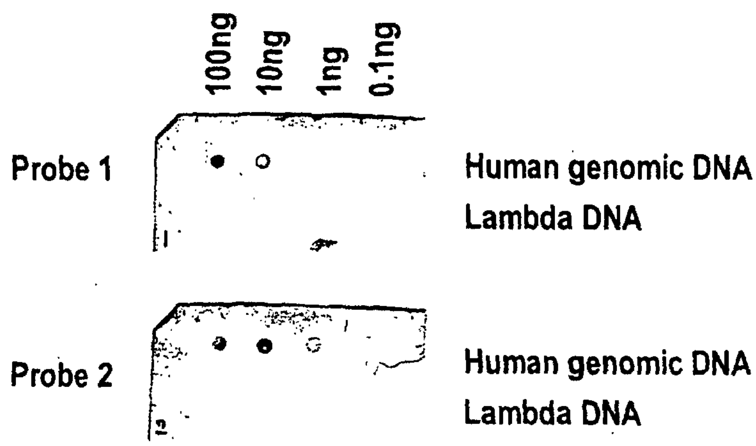
100ng
10ng
1ng
0.1ng
Probe 1
Human genomic DNA
Lambda DNA
Probe 2
Human genomic DNA
Lambda DNA

## INTERNATIONAL SEARCH REPORT

International application No.
PCT/JP02/06939

A. CLASSIFICATION OF SUBJECT MATTER
Int.Cl$^7$ C12N15/09, C12M1/00, C12M1/12

According to International Patent Classification (IPC) or to both national classification and IPC

B. FIELDS SEARCHED

Minimum documentation searched (classification system followed by classification symbols)
Int.Cl$^7$ C12N15/09, C12M1/00, C12M1/12

Documentation searched other than minimum documentation to the extent that such documents are included in the fields searched

Electronic data base consulted during the international search (name of data base and, where practicable, search terms used)
JICST FILE(JOIS), PubMed

C. DOCUMENTS CONSIDERED TO BE RELEVANT

| Category* | Citation of document, with indication, where appropriate, of the relevant passages | Relevant to claim No. |
|---|---|---|
| Y | US 5645723 A (TOMY SEIKO KABUSHIKI KAISHA), 18 February, 1997 (18.02.97), & JP 9-47278 A | 1,2,4,9-11, 21-46,49,50 |
| Y | WO 95/24418 A1 (MINNESOTA MINING & MFG.), 14 September, 1995 (14.09.95), & JP 9-510200 A | 1,2,4,9-11, 21-46,49,50 |
| Y | WO 96/18731 A1 (DYNAL AS.), 20 June, 1996 (20.06.96), & JP 11-501504 A | 1,2,4,9-11, 21-46,49,50 |
| Y | JP 8-103653 A (Terumo Corp.), 23 April, 1996 (23.04.96), (Family: none) | 1,2,4,9-11, 21-46,49,50 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 October, 2002 (07.10.02) | 29 October, 2002 (29.10.02) |

| Name and mailing address of the ISA/ | Authorized officer |
|---|---|
| Japanese Patent Office | |
| Facsimile No. | Telephone No. |

Form PCT/ISA/210 (second sheet) (July 1998)

INTERNATIONAL SEARCH REPORT

International application No.
PCT/JP02/06939

C (Continuation). DOCUMENTS CONSIDERED TO BE RELEVANT

| Category* | Citation of document, with indication, where appropriate, of the relevant passages | Relevant to claim No. |
|---|---|---|
| A | JP 2-295485 A (Kabushiki Kaisha Nitsusho), 06 December, 1990 (06.12.90), (Family: none) | 1-50 |
| A | WO 99/05321 A1 (RAPIGENE INC.), 04 February, 1999 (04.02.99), & JP 2001-511361 A | 1-50 |

Form PCT/ISA/210 (continuation of second sheet) (July 1998)

SEQUENCE LISTING

<110> ASAHI KASEI KABUSHIKI KAISHA

<120> A method of purification and detection of nucleic acids using nonwoven fabric

<130> PH-1593-PCT

<150> JP 2001-208514
<151> 2001-07-09

<150> JP 2001-364878
<151> 2001-11-29

<160> 12

<210> 1
<211> 21
<212> DNA
<213> Artificial sequence

<220>
<223> Synthetic DNA

<400> 1
caacgcagaa gtacgtaaag a 21

<210> 2

<211> 21

<212> DNA

<213> Artificial sequence

<220>

<223> Synthetic DNA

<400> 2

tctttaccgt caacaacgat g 21

<210> 3

<211> 20

<212> DNA

<213> Artificial Sequence

<220>

<223> Synthetic DNA

<400> 3

gcgacgtcca agaagccttg 20

<210> 4

<211> 20

<212> DNA

<213> Artificial Sequence

<220>

<223> Synthetic DNA

<400> 4

ggcagacccc tccttattgc 20

<210> 5

<211> 20

<212> DNA

<213> Artificial Sequence

<220>

<223> Synthetic DNA

<400> 5

gtaggcgtgg aacagatcaa 20

<210> 6

<211> 20

<212> DNA

<213> Artificial Sequence

<220>

<223> Synthetic DNA

<400> 6

cttgaagagt gcatgctgga 20

<210> 7

<211> 20

<212> DNA

<213> Artificial Sequence

<220>

<223> Synthetic DNA

<400> 7

ctccccacag atagaagagc 20

<210> 8

<211> 20

<212> DNA

<213> Artificial Sequence

<220>

<223> Synthetic DNA

<400> 8

gcagaatctg gcttgctttg 20

<210> 9

<211> 20

<212> DNA

<213> Artificial Sequence

<220>

<223> Synthetic DNA

<400> 9

ctggcatcgt gatggactcc 20

<210> 10

<211> 20

<212> DNA

<213> Artificial Sequence

<220>

<223> Synthetic DNA

<400> 10

catactcctg cttgctgatc 20

<210> 11

<211> 20

<212> DNA

<213> Artificial sequence

<220>

<223> Synthetic DNA

<400> 11

tccaccaccc tgttgctgta 20

<210> 12

<211> 20

<212> DNA

<213> Artificial sequence

<220>

<223> Synthetic DNA

<400> 12

accacagtcc atgccatcac 20